(19) Europäisches Patentamt
European Patent Office
Office européen des brevets

(11) **EP 4 640 222 A2**

(12) **EUROPEAN PATENT APPLICATION**


(43) Date of publication:
**29.10.2025 Bulletin 2025/44**

(21) Application number: **25187334.5**

(22) Date of filing: **27.12.2024**

(51) International Patent Classification (IPC):
***A61K 31/593*** *(2006.01)*

(52) Cooperative Patent Classification (CPC):
(C-Sets available)
**A61P 25/02; A23L 33/105; A23L 33/15; A23L 33/155; A61K 31/197; A61K 31/221; A61K 31/4415; A61K 31/51; A61K 31/525; A61K 31/592; A61K 31/593; A61K 31/7068; A61K 31/714; A61K 36/8988; A61P 29/00** (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **03.01.2024 IT 202400000033**
**03.01.2024 IT 202400000045**

(62) Document number(s) of the earlier application(s) in accordance with Art. 76 EPC:
**24841794.1 / 4 626 452**



(71) Applicant: **Futura S.r.l.**
**20155 Milano (IT)**

(72) Inventors:
- **UBERTI, Francesca**
  **28838 Stresa (Verbano-Cusio-Ossola) (IT)**
- **TISO, Domenico**
  **20155 Milano (IT)**

(74) Representative: **Delbarba, Andrea**
**Bugnion S.p.A.**
**Viale Lancetti, 17**
**20158 Milano (IT)**


Remarks:
This application was filed on 03-07-2025 as a divisional application to the application mentioned under INID code 62.

(54) **NOVEL ASSOCIATION OF GASTRODIA ELATA, CITICOLINE, L-ACETYLCARNITINE AND VITAMIN D**


(57) The present invention relates to a novel association or combination of active ingredients useful in the prevention and repair of neuronal damage, both centrally and peripherally. In greater detail, the present invention relates to an association or combination of an extract of *Gastrodia elata,* citicoline, L-acetylcarnitine and at least one vitamin of group D. Furthermore, the invention also relates to the pharmaceutical compositions and/or compositions for medical devices and/or nutraceutical and/or food compositions comprising that association and to their use in therapy, in particular in the protection of the functionality of the central and of the peripheral nervous system.


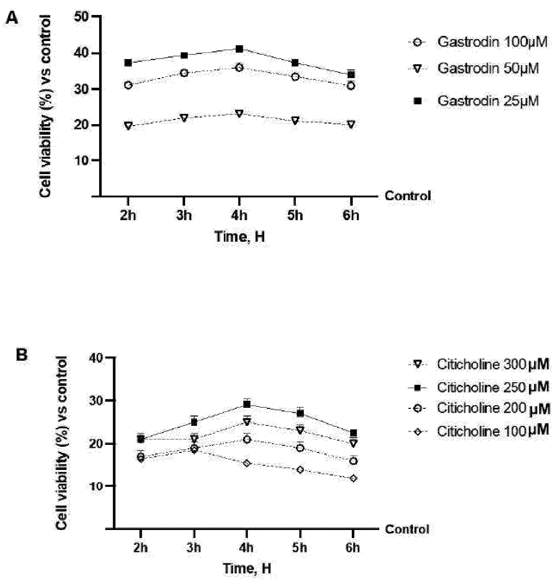


FIG. 1 (continued)

EP 4 640 222 A2

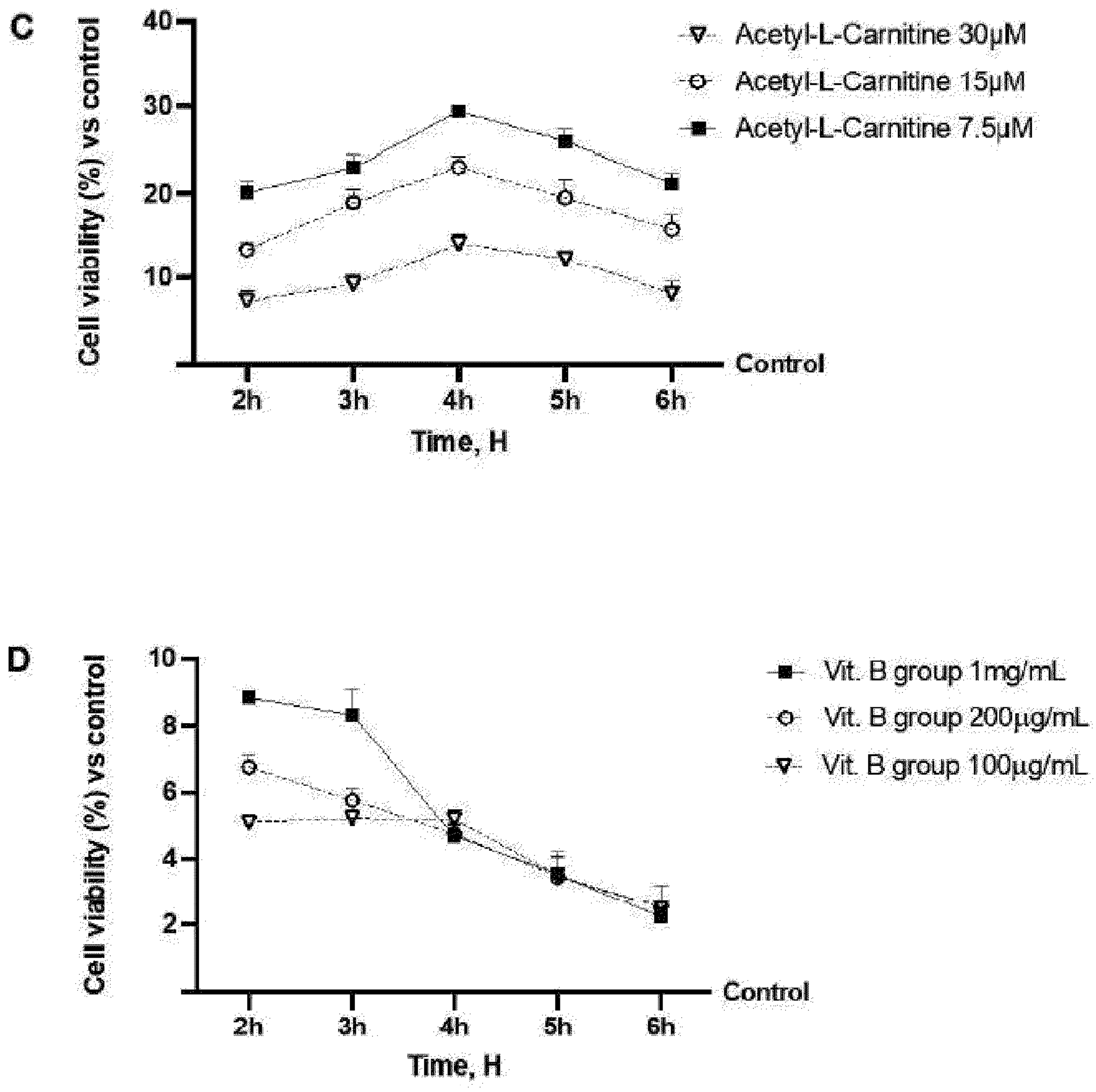


FIG. 1

(52) Cooperative Patent Classification (CPC): (Cont.)

C-Sets
**A61K 31/221, A61K 2300/00;**
**A61K 31/592, A61K 2300/00;**
**A61K 31/593, A61K 2300/00;**
**A61K 31/7068, A61K 2300/00;**
**A61K 31/714, A61K 2300/00;**
**A61K 36/8988, A61K 2300/00**

## Description

The present invention relates to a novel association or combination of active ingredients useful in the prevention and repair of neuronal damage, both centrally and peripherally. In greater detail, the present invention relates to an association or combination of i) an extract of *Gastrodia elata* and ii) at least one ingredient selected from the group comprising or, altematively, consisting of citicoline, L-acetylcarnitine and, preferably, at least one vitamin of group D, and/or mixtures thereof, and at least one vitamin of group B, and/or mixtures thereof. Furthermore, the invention also relates to the pharmaceutical compositions and/or compositions for medical devices (Regulation (EU) 2017/745) and/or nutraceutical compositions and/or food compositions comprising that association or combination and to their use in therapy, in particular in the protection of the functionality of the nervous system, preferably central nervous system (CNS) and peripheral nervous system (PNS) and in the prevention and/or treatment of central and peripheral neuropathies.

## BACKGROUND OF THE INVENTION

The nervous system is made up of the central nervous system (CNS) and peripheral nervous system (PNS).
The central nervous system (CNS) is made up of the encephalon (brain) and spinal cord, whilst the peripheral nervous system (PNS) is made up of the ganglia (neurons) and nerves (axons) which connect the information of the central nervous system to the rest of the body.
Pathologies causing damage to the central and peripheral nervous systems are widespread and represent the leading cause of disability, with over a million deaths each year. Their considerable increase is due, in part, to the aging of the population, the lack of curative treatments and the poor effectiveness of preventive measures.
Damage to the central nervous system of mammals may produce severe physical damage and result in many complications, which may also be lethal. Damage to the central nervous system may derive, for example, from a spinal cord injury or a stroke-induced injury or it may be consequence of pathologies affecting other organs.
As far as the peripheral nervous system is concerned, neuropathy may result from the deterioration and consequent malfunctioning of the nerves. This deterioration may be the consequence of injury, trauma or repeated stress. Peripheral neuropathy is very often the consequence of a metabolic-endocrine pathology, such as, for example, diabetes. Peripheral neuropathy is a very widespread disease among middle-aged to elderly individuals, and it has been estimated that about 60% of diabetic patients undergo damage at the level of the peripheral nerves. Although peripheral neuropathy is usually a disease without a fatal outcome, in contrast to central pathologies, it is nevertheless very painful and often disabling.
Associations of various substances that are useful for protecting or improving the health of the central and peripheral nervous systems are well known (from commercially available products and/or the scientific and patent literature). Such substances have been associated with one another to provide a treatment which acts on the nervous system through different mechanisms of action, often able to exert synergistic effects in terms of effectiveness for the maintenance of nerve function.
It can by no means be taken for granted that combining different active ingredients will lead to a reasonable expectation of obtaining a therapeutic success. In fact, there are various technical problems to be overcome in order that a composition may be effective. In particular, the ideal combination must:

- have good bioavailability of the active substances:
- be well tolerated;
- show very low toxicity;
- be suitable for extended and repeated use;
- be easy to administer;
- have a pleasing odor and flavor:
- be easy to prepare
- have affordable costs.

Pain is a frequent sign of many illnesses and typically denotes the presence of tissue damage; in contrast, "chronic pain" is the term used to describe pain that lasts for extended periods, either as a result of a disease process or after the typical healing time of an injury. Approximately 1 in 9 young adults worldwide experience chronic pain, corresponding to 11.6%. This increased pain has drastic and expensive effects on the population as regards work performance and quality of daily life. As a result, there is a need for new and more effective treatments, and the discovery of novel mechanisms and pathways of action may offer future developments for new compositions. While initial studies on pain mechanisms focused on the role of neurons, researchers’ attention has now shifted to the role of non-neuronal cells. In this context, peripheral neuropathy (PN) is a disorder caused by malfunctioning of peripheral nerves, including sensory and motor neurons, as well as their axons and myelin sheaths. Severe discomfort, muscular weakness, and sensory loss

are clinical symptoms of PN. The fundamental causes of prolonged pain are neuronal hyperactivity and/or hyperexcitability, which arise following nerve injury. The ideas that explain the mechanisms associated with central neuropathy include intracellular signaling, microglial activation, neurotrophic factors, elevated levels of pro-inflammatory and inflammatory mediators, and oxidative stress (OS). Indeed, an increasing number of studies suggest that OS has a role in neuropathic pain via peripheral and central sensitization. Excess reactive oxygen and nitrogen species (ROS, RNS) harm neurons by interacting with proteins, carbohydrates, lipids, and nucleic acids. In this context, SIRT3 has an important role in several biological processes, including mitochondrial function. Furthermore, OS causes inflammatory cells, including lymphocytes, monocytes, neutrophils, basophils, and eosinophils, to produce a range of substances (proinflammatory cytokines, vasoactive amines and peptides, eicosanoids).

It has been established that neuropathic pain is caused by the overexpression of proinflammatory mediators (TNF-$\alpha$, IL-1). In this context, current compositions and methods of treatment still have and show many limitations and side effects when administered for treating this pathological pain, which is a maladaptive mechanism. Due to this, the broad availability of targeted therapeutics for neuropathic pain, nutraceuticals (dietary supplements and herbal/natural products), are becoming increasingly popular due to their low cost, high nutritional and therapeutic qualities, and capacity to bind to many molecular targets.

To date, lipoic acid has emerged as a viable alternative in recent years; its mechanisms of action in experimental diabetic neuropathy include reduced OS and an improvement in neural blood flow, nerve conduction velocity, and a variety of other nerve function indicators. However, at present it is also known that the phytoprotection system has given rise to a large number of complaints of adverse responses to dietary supplements based on lipoic acid: among them are cases of autoimmune hyperinsulinemia. This uncommon illness, also known as Hirata syndrome, is a severe adverse response characterized by extreme hypoglycaemia. Otherwise, the adverse effects of lipoic acid are correlated with diabetic polyneuropathy, which also includes gastrointestinal symptoms with treatment at higher doses (>600mg/die). Therefore, whilst on the one hand alpha-lipoic acid is an ingredient widely used in compositions for protecting or improving the health of the peripheral nervous system, on the other hand there is scientific evidence reporting adverse reactions, even severe ones, following the use of supplements containing lipoic acid (https://www.epicentro.iss.it/fitosorveglianza/reazioni-avverse-integratori-acido-lipidi).

Therefore, scientific research is looking for alternatives to counter the negative effects of this substance, also at reduced doses. Specifically, scientific research is constantly looking for novel solutions which envisage the use of reduced doses, if not elimination, of lipoic acid, thanks to the use of other ingredients of natural origin, such as, for example, plant extracts, or extracts of synthetic origin.

It is understood, therefore, that there is a need to find new therapeutic approaches and new effective compositions for maintaining the well-being of the nervous system, for limiting the damage thereof and possibly promoting its regeneration in case of injury, said new approaches and new compositions being effective, well tolerated and free of side effects and/or adverse reactions.

The technical problem that the present invention addresses and solves is therefore to provide a valid and effective solution for maintaining the health of the nervous system and for treating neuropathic pain, in the absence of side effects and/or adverse reactions.

The Applicant, following intensive research activities, has developed and fine-tuned a new association or combination of active ingredients, and pharmaceutical compositions and/or compositions for medical devices (Regulation (EU) 2017/745) and/or nutraceutical compositions and/or food compositions (the composition(s) of the present invention) that are effective, free of side effects and with high tolerability. Said association and combinations having been developed for maintaining the well-being and health of the peripheral nervous system, for preventing and/or treating the diseases related thereto, for the protection of peripheral nerve function, for the prevention and/or treatment of peripheral neuropathies, for preventing and/or treating neuropathic pain and for stimulating the endogenous production of neurotrophic factors.

The new associations or combinations of active ingredients, and the pharmaceutical compositions and/or compositions for medical devices (Regulation (EU) 2017/745) and/or nutraceutical and/or food compositions (the composition(s) of the present invention) containing them, due to the presence of natural ingredients, do not have significant adverse effects, are free of side effects and have high tolerability, and can be administered to a broad category of individuals.

Furthermore, the novel associations or combinations of active ingredients, and pharmaceutical compositions and/or compositions for medical devices (Regulation (EU) 2017/745) and/or nutraceutical compositions and/or food compositions (the composition(s) of the present invention) containing them are easy to prepare and inexpensive, and can be prepared using techniques, methods and apparatus known to the person skilled in the art. Furthermore, due to the presence of natural ingredients, said compositions do not have significant adverse effects, are free of side effects and have high tolerability, and can be administered to a broad category of individuals. These purposes and still others, which will be clear from the detailed description below, are achieved by the novel associations or combinations of active ingredients, and the pharmaceutical compositions and/or compositions for medical devices (Regulation (EU) 2017/745) and/or

nutraceutical and/or food compositions (the composition(s) of the present invention) containing them, by virtue of the technical features claimed in the appended claims.

## DETAILED DESCRIPTION OF THE INVENTION

It is an object of the present invention an association or combination of i) an extract of *Gastrodia elata,* preferably, said extract comprises or consists of gastrodin, and ii) at least one ingredient selected from the group comprising or, alternatively, consisting of citicoline and L-acetylcamitine, and/or mixtures thereof (A1 for short).

It is an object of the present invention an association or combination of i) an extract of *Gastrodia elata*; preferably, said extract comprises or consists of gastrodin, and ii) at least one ingredient selected from the group comprising or, altematively, consisting of citicoline and L-acetylcamitine, and/or mixtures thereof; said association or combination possesses a noteworthy activity in preventing and repairing damage to the nervous system. Preferably, said combination or combination A1 is for use in therapy, in particular in a method for the protection of nervous system function and/or in a method of prevention and/or treatment of central and peripheral neuropathies.

It is an object of the present invention an association or combination of i) an extract of *Gastrodia elata,* preferably, said extract comprises or consists of gastrodin, and ii) at least one ingredient selected from the group comprising or, alternatively, consisting of citicoline, L-acetylcamitine and at least one B-group vitamin, and/or mixtures thereof (A2 for short).

It is an object of the present invention an association or combination of i) an extract of *Gastrodia elata,* preferably, said extract comprises or consists of gastrodin, and ii) at least one ingredient selected from the group comprising or, alternatively, consisting of citicoline, L-acetylcamitine and at least one B-group vitamin, and/or mixtures thereof, said association or combination possesses a noteworthy activity in preventing and repairing damage to the nervous system. Preferably, said association or combination A2 is for use in therapy, in particular in a method for the protection of nervous system function and/or in a method of prevention and/or treatment of central and peripheral neuropathies.

Preferably, said association or combination A2 comprises i) an extract of *Gastrodia elata,* preferably, said extract comprises or consists of gastrodin, and ii) citicoline, L-acetylcamitine and/or at least one B-group vitamin selected from the group comprising or, altematively, consisting of vitamin B1, vitamin B2, vitamin B5, vitamin B6, vitamin B9 and vitamin B12, and/or mixtures thereof. Preferably, said association or combination A2 is for use in therapy, in particular in a method for the protection of nervous system function and/or in a method for the prevention and/or treatment of central and peripheral neuropathies.

It is an object of the present invention an association or combination of i) an extract of *Gastrodia elata,* preferably, said extract comprises or consists of gastrodin, and ii) at least one ingredient selected from the group comprising or, altematively, consisting of citicoline, L-acetylcarnitine and at least one D-group vitamin, and/or mixtures thereof (A3 for short).

It is an object of the present invention an association or combination of i) an extract of *Gastrodia elata,* preferably, said extract comprises or consists of gastrodin, and ii) at least one ingredient selected from the group comprising or, altematively, consisting of citicoline, L-acetylcarnitine and at least one D-group vitamin, and/or mixtures thereof, said association or combination possesses a noteworthy activity in preventing and repairing damage to the nervous system. Preferably, said association or combination A3 is for use in therapy, in particular in a method for the protection of nervous system function and/or in a method of prevention and/or treatment of central and peripheral neuropathies.

It is an object of the present invention an association or combination of i) an extract of *Gastrodia elata,* preferably, said extract comprises or consists of gastrodin, and ii) at least one ingredient selected from the group comprising or, alternatively, consisting of citicoline, L-acetylcamitine, at least one D-group vitamin and at least one B-group vitamin, and/or mixtures thereof (A4 for short).

It is an object of the present invention an association or combination of i) an extract of *Gastrodia elata,* preferably, said extract comprises or consists of gastrodin, and ii) at least one ingredient selected from the group comprising or, altematively, consisting of citicoline, L-acetylcamitine, at least one D-group vitamin and at least one B-group vitamin, and/or mixtures thereof, said association or combination possesses a noteworthy activity in preventing and repairing damage to the nervous system. Preferably, said association or combination A4 is for use in therapy, in particular in a method for the protection of nervous system function and/or in a method of prevention and/or treatment of central and peripheral neuropathies.

Preferably, said association or combination A4 consisting of i) an extract of *Gastrodia elata* and ii) citicoline, L-acetylcarnitine and vitamin D, preferably vitamin D3, and/or B-group vitamins selected from the group comprising or, altematively, consisting of vitamin B1, vitamin B2, vitamin B5, vitamin B6, vitamin B9 and vitamin B12, and mixtures thereof. Preferably, said association or combination is for use in therapy, in particular in a method for the protection of nervous system function and/or in a method of prevention and/or treatment of central and peripheral neuropathies.

Preferably, 'combination' means each of the various possible ways in which two or more elements can be joined or found together. For example, ingredients i) and ii) are found joined together in the combination and are administered

together when the combination is administered.

Preferably, 'association' means the act of associating distinct elements that are administered at the same time or at short intervals (first one and then the other). For example, ingredients i) and ii) are administered at the same time, but in a distinct manner, or are administered a short distance apart, first one and then the other, but still in a distinct manner.

In the context of the present invention, the terms association, or combination or mixture may be used interchangeably with each other.

Gastrodia elata belongs to the *Orchidaceae* family. The part of the plant that shows activity is its rhizome (*Rhizoma Gastrodiae*) from which, for example, phenols, polysaccharides, organic acids and sterols, and gastrodin, which is one of the phenolic glycosides, can be isolated. Each of these categories of compounds may contain, within them, several specific compounds or specific chemical entities such as, for example, gastrodin.

Gastrodia extract, preferably extract of *Gastrodia elata,* is therefore understood to comprise an extract comprising or consisting of one or more of said categories of isolated compounds, each of which, may be present in said extract in an amount varying from zero to 100% by weight, relative to the weight of the extract. For example, said extract may comprise or consist of gastrodin, or may comprise or consist of a mixture of said specific compounds or specific chemical entities belonging to one or more of said categories of compounds. We shall note that it has become customary in the marketplace to call an "extract" of Gastrodia, preferably an extract of *Gastrodia elata,* by the term "gastrodin extract 10:1". In practice, even though the word gastrodin refers to a specific compound isolated from the rhizome of the plant (Gastrodin, from *Gastrodia elata* ≥98%; (2R,3S,4S,5R,6S)-2-hydroxymethyl-6-(4-hydroxymethyl-phenoxy)-tetrahydropyran-3,4,5-triol, 4-β-D-glucopyranosyloxybenzyl alcohol, 4-hydroxybenzyl alcohol 4-O-β-D-glucoside, CAS 62499-27-8), in the market, the word gastrodin is also used to refer to an extract, e.g., a 10:1 extract or a 5:1 extract, which may contain not only gastrodin in itself or as such (per se), but also other specific compounds. (For the sake of clarity, we make reference to Gastrodina in Italian and Gastrodin in English to indicate the same compound).

In the context of the present invention, gastrodia extract is meant to comprise all types of extracts containing gastrodin, e.g., 20:1 or 15:1 or 10:1 or 5:1, but also gastrodin as such, without any limitation.

The extract of *Gastrodia elata,* preferably the extract of *Gastrodia elata* 10:1, comprises or consists of gastrodin.

As mentioned above, the Gastrodia extracts, preferably the extracts of *Gastrodia elata,* more preferably extracts of *Gastrodia elata* 10:1, comprise or consist of gastrodin; said extracts contain active ingredients (specific compounds or specific chemical entities) that include not only molecular compounds of small dimensions such as, for instance, gastrodin, i.e. 4-hydroxybenzyl alcohol-4-O-β-D-glucopyranoside [CAS RN: 62499-27-8], of formula (I):

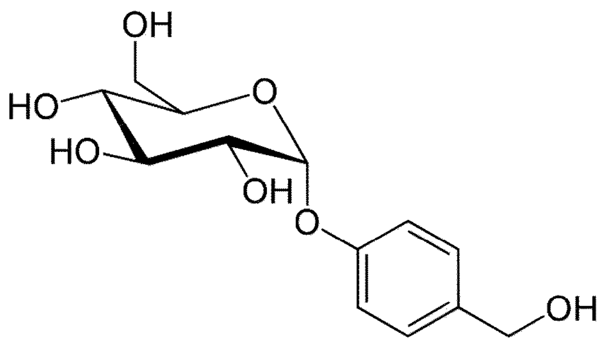

(I),

which is the active ingredient of *Gastrodia elata* that has been most extensively studied, Parishin, phenolic compounds, 4-hydroxybenzyl alcohol and β-sitosterol, but also active polysaccharide macromolecules.

In the context of the present invention, "an extract of *Gastrodia elata*" is intended to refer to, and include, any extract (and/or mixtures of extracts) of *Gastrodia elata,* obtained, from any source, with methods and apparatus well known to the person skilled in the art.

Preferably, the extract of *Gastrodia elata* may, for example, be a 10:1 extract of *Gastrodia elata,* i.e., standardized to contain, for example, a concentration 10 times higher than the concentration of active substances present in the tuber of *Gastrodia elata.*

The extract of *Gastrodia elata* may, for example, be a 5:1, or even a 3:1, extract of *Gastrodia elata.*

In the context of the present invention, 10:1 extract means the ratio [drug:extract] (1 gram of extract corresponds to the active ingredients contained in 10 grams of plant).

Said extract of *Gastrodia elata,* preferably a dry extract of *Gastrodia elata,* can be obtained by extraction with an extraction solvent, e.g., water or a water/alcohol solution (e.g., ethyl alcohol or ethanol), at a temperature comprised from, for example, 20°C to 70°C. Next, a powder is preferably obtained by drying or spraying with a spray-drying system to give a nebulized dry extract, in which, for example, the extract is nebulized in a stream of superheated steam. The short contact time between the extract and the steam causes instant drying without damaging the active ingredients. Highly concentrated extracts can be obtained.

Preferably, the extract of *Gastrodia elata,* for example a dry extract of *Gastrodia elata,* at a drug/extract ratio of 10:1, may have a phytochemical profile of the following type:

- a total polysaccharide content ranging from 40% to 90%, preferably from 50% to 80%, more preferably from 60% to 70%; for example, alpha-glucans and/or maltodextrins may be present in an amount from greater than zero to less than 10%, preferably from greater than 0.1% to less than 7.5%, more preferably from greater than 2% to less than 5%;
- a triterpene content ranging from 0.5% to 5%, preferably from 1% to 4%, more preferably from 1.5% to 3%;
- a total polyphenol content ranging from 0.05% to 3%, preferably from 0.1% to 2%, more preferably from 0.2% to 1%.

In addition, lignans in an amount greater than zero to less than 0.01%, e.g., less than 0.05%, and triterpenes in an amount greater than zero to less than 0.01%, e.g., less than 0.05%, may also be present in said extract. Preferably, said triterpenes and polyphenols may be of a low molecular weight and belong to the class of phenolic glycosides.

As a further example, the extract of *Gastrodia elata* may be an extract of *Gastrodia elata* prepared from rhizome or root, for example, by extraction with water and ethanol having a polysaccharide content (measured with the UV Phenol Sulfuric Acid method, at 490 nm) from 50% to 90%, preferably 60% to 80%; a particle size of 80 mesh (min. 95% passes through 80 mesh) and a bulk density CP <2020> ranging from 0.3 g/ml to 0.6 g/ml.

As a further example, an extract of *Gastrodia elata* may be *Gastrodia elata* rhizome P.E. 10:1 (dry extract in the presence of dextrin as an excipient) and having a polysaccharide content (measured with the UV Phenol Sulfuric Acid method, at 490 nm) of 10% to 40%, preferably 15% to 20%; a particle size of 100 mesh (100% passes through 100 mesh), a water solubility of 100% at a temperature of 25°C and a tap density of 0.35 to 0.55 g/cm3.

Citicoline, IUPAC name 2-[{[5-(4-amino-2-oxopyrimidin-1-yl)-3,4-dihydroxyoxolan-2-yl]methoxy-hydroxyphosphoryl}oxy-hydroxyphosphoryl]oxyethyl-trimethylazane (CAS RN: 987-78-0), also known as cytidine diphosphate choline or CDP-choline is an intermediate in the biosynthesis of phosphatidylcholine. It is a psychostimulant/nootropic of well-known use in supporting nervous system health.

In the context of the present invention, "citicoline" is intended to refer to, and include, citicoline as such and/or in the form of a pharmaceutically acceptable salt thereof and/or mixtures thereof.

L-acetylcarnitine, IUPAC name (R)-3-acetyloxy-4-trimethylammonium-butanoate (CAS RN: 3040-38-8), also known as LAC, acetyl-L-camitine, levacecamine or ALCAR, is an acetylated form of L-camitine or carnitine.

In the context of the present invention "L-acetylcarnitine" is intended to refer to, and generally includes, L-acetylcarnitine as such and/or in the form of a pharmaceutically acceptable salt thereof and/or mixtures thereof, and/or L-carnitine and/or carnitine.

Preferably, said at least one B-group vitamin, present in combination with i) an extract of *Gastrodia elata* and ii) at least one ingredient selected from the group comprising or, alternatively, consisting of citicoline, L-acetylcarnitine (e.g., association or combination of A2 and A4), is selected from the group comprising or, alternatively, consisting of vitamin B1 (thiamine), vitamin B2 (riboflavin) vitamin B3 (niacin), vitamin B5 (pantothenic acid), vitamin B6 (pyridoxine), vitamin B7 (inositol), vitamin B8 (biotin), vitamin B9 (folic acid), vitamin B12 (cobalamin) and mixtures thereof, preferably vitamin B1 (thiamine), vitamin B2 (riboflavin), vitamin B5 (pantothenic acid), vitamin B6 (pyridoxine), vitamin B12 and mixtures thereof.

Preferably, said at least one D-group vitamin, present in association with i) an extract of *Gastrodia elata* and ii) at least one ingredient selected from the group comprising or, alternatively, consisting of citicoline, L-acetylcarnitine (e.g., association or combination of A3 and A4), is selected from the group comprising or, altematively, consisting of: vitamin D1, a compound consisting of 1:1 parts of ergocalciferol and lumisterol, vitamin D2 ergocalciferol, vitamin D3 cholecalciferol, vitamin D4 dihydroergocalciferol, vitamin D5 sitocalciferol, and/or mixtures thereof.

Said at least one D-group vitamin is useful for the well-being of the skeletal system and teeth, and also exerts functions at a cerebral, muscular, metabolic and immune system level.

Preferably, said at least one D-group vitamin included in the association of the present invention (e.g., association or combination of A3 and A4) is vitamin D2 and/or vitamin D3 and/or a mixture thereof; even more preferably, the vitamin D included in the association of the present invention (e.g., association or combination of A3 and A4) is vitamin D3. Preferably, said at least one B-group vitamin, present in association with i) an extract of *Gastrodia elata* and ii) at least one ingredient selected from the group comprising or, alternatively, consisting of citicoline, L-acetylcamitine (e.g., association or combination A2 and A4), is selected from the group comprising or, altematively, consisting of vitamin B1 (thiamine), vitamin B2 (riboflavin), vitamin B3 (niacin), vitamin B5 (pantothenic acid), vitamin B6 (pyridoxine), vitamin B7 (inositol), vitamin B8 (biotin), vitamin B9 (folic acid), vitamin B12 (cobalamin) and mixtures thereof; preferably vitamin B1 (thiamine), vitamin B2 (riboflavin), vitamin B5 (pantothenic acid), vitamin B6 (pyridoxine), vitamin B12 and mixtures thereof.

It is an object of the present invention to provide pharmaceutical compositions and/or compositions for medical devices (Regulation (EU) 2017/745) and/or nutraceutical compositions and/or food compositions comprising an association or combination of the present invention, preferably the association or combination A1, A2, A3 or A4; optionally,

together with one or more pharmaceutically acceptable excipients and/or vehicles.

Preferably, said compositions are for use in therapy, in particular in a method for the protection of nervous system function and/or in a method for the prevention and/or treatment of central and peripheral neuropathies.

Preferably, the pharmaceutical and/or nutraceutical composition or supplement composition of the invention comprises: an extract of *Gastrodia elata,* citicoline, L-acetylcarnitine, a vitamin D, in particular vitamin D2 and/or D3, B-group vitamins, in particular vitamin B1, vitamin B2, vitamin B5, vitamin B6 and vitamin B12; together with at least one pharmaceutically acceptable excipient and/or vehicle.

The composition of the invention is a composition for oral use, preferably a solid-state composition, preferably formulated in dosage units. "Solid state" means that the composition may exist in the form of granules or powders. The granular or powder compositions are mixed with pharmacologically acceptable additives and excipients to provide a final product such as, for example, a supplement product, medical device or pharmaceutical composition. The final product may be in pharmaceutical dosage units such as granules in a sachet, stick, orodispersible stick, tablet, gel or capsule.

The tablets may have different shapes among those known in the field of pharmaceutical forms, such as a cylindrical or spheroidal shape. The tablets may be coated or filmed with one or more layers of coating or film capable of passing through the gastric barrier, according to known methods.

The gel capsules may consist of hard gelatin or soft gelatin or soft gel.

Preferably, the oral composition of the invention is a solid or semi-solid (gel) composition as described above. However, if desired or necessary, the composition may be formulated in liquid form, for example by dissolution or suspension in water.

The solid compositions of the invention may contain, as mentioned above, physiologically acceptable conventional excipients and/or vehicles, such as diluents, bulking agents, binders, disaggregating agents, flow aids, lubricants, etc. Non-limiting examples of suitable vehicles and excipients are described in Remington: The Science and Practice of Pharmacy, 21st Ed., Lippincott, Williams & Wilkins. Compositions of the invention may, for example, include cellulose derivatives, glucose, lactose, sucrose, gelatin, malt, rice, flour, gypsum, silica gel, sodium stearate, glycerol monostearate, talc, sodium chloride, skim milk powder, glycerol, propylene, glycol, water, ethanol, and the like. The composition may also contain pH buffering reagents and wetting or emulsifying agents.

In addition to the above-described components, if desired or necessary, the composition of the invention may comprise further active components, for example components capable of assisting the active ingredients in performing their action in the body of the treated individual, provided that said further components are physically and chemically compatible with the active ingredients of the composition. Examples of further components are, for example, vitamins of the E group, or hyaluronic acid and the salts thereof, such as sodium hyaluronate.

Preferably, the compositions of the present invention comprise or, alternatively, consist of i) an extract of *Gastrodia elata,* and ii) citicoline, L-acetylcarnitine, at least one B-group vitamin, in particular vitamin B1, vitamin B2, vitamin B5, vitamin B6, vitamin B12; together with at least one pharmaceutically acceptable excipient and/or vehicle.

Preferably, the compositions of the present invention comprise or, alternatively, consist of i) an extract of *Gastrodia elata,* and ii) citicoline, L-acetylcamitine, at least one D-group vitamin, in particular vitamin D3, at least one B-group vitamin, in particular vitamin B1, vitamin B2, vitamin B5, vitamin B6, vitamin B12; together with at least one pharmaceutically acceptable excipient and/or vehicle.

Preferably, the compositions of the present invention may be in the form of a capsule, a tablet, a sachet, a stick, an orodispersible stick (orodispersible stick orally soluble in the mouth), an oral gel or granules.

The composition of the invention, preferably formulated in dosage units, may be administered once or several times a day, for example once or twice or three times a day.

The pharmaceutical and/or nutraceutical composition of the invention preferably comprises:

- 10 to 1,000 mg, preferably 50 to 500 mg, even more preferably 100 to 300 mg of *Gastrodia elata* extract and
- 50 to 500 mg, preferably 100 to 300 mg, even more preferably 150 to 250 mg of citicoline or a pharmaceutically acceptable salt thereof and/or mixtures thereof; and/or
- 100 mg to 2,000 mg, preferably 200 mg to 600 mg, even more preferably 400 mg to 500 mg of L-acetylcamitine (L-camitine and/or carnitine) or a pharmaceutically acceptable salt thereof and/or mixtures thereof; and
- 1 $\mu$g to 100 mg of a mixture of vitamins of the B group (B1, B2, B3, B5, B6, B7, B8, B9, B12) in particular a mixture of vitamin B1, vitamin B2, vitamin B5, vitamin B6, vitamin B12; together with one or more pharmaceutically acceptable excipients and/or vehicles.

Preferably, the pharmaceutical and/or nutraceutical composition of the invention comprises:

- 10 to 1,000 mg, preferably 50 to 500 mg, even more preferably 100 to 300 mg of *Gastrodia elata* extract; and
- 50 to 500 mg, preferably 100 to 300 mg, even more preferably 150 to 250 mg of citicoline or a pharmaceutically acceptable salt thereof and/or mixtures thereof; and

- 100 to 2,000 mg, preferably 200 to 600 mg, even more preferably 400 to 500 mg of L-acetylcarnitine (L-camitine and/or camitine) or a pharmaceutically acceptable salt thereof and/or mixtures thereof; and
- 5 μg to 200 μg, preferably 10 to 100 μg of vitamin D, preferably D2 and/or D3; and
- 1 μg to 100 mg of a mixture of B-group vitamins (B1, B2, B3, B5, B6, B7, B8, B9, B12), in particular a mixture of vitamin B1, vitamin B2, vitamin B5, vitamin B6, vitamin B12; together with one or more pharmaceutically acceptable excipients and/or vehicles.

The B-group vitamins (B1, B2, B3, B5, B6, B7, B8, B9, B12), in particular a mixture containing vitamin B1, vitamin B2, vitamin B5, vitamin B6 and vitamin B12, and the D-group vitamins, preferably D2 and/or D3, may all be absent, or all be present in the compositions of the present invention; or else there may be only B-group vitamins or only D-group vitamins.

The compositions of the invention may be prepared by mixing the individual components, and any conventional excipients and/or vehicles.

The daily dosage depends on the patient's health status, weight, gender and age. In general, the compositions of the invention are well tolerated and can be administered once or several times a day, even over an extended period of time. The Applicant has found that the association of the invention and the pharmaceutical and/or nutraceutical compositions containing it are useful for maintaining the well-being and health of the nervous system and for preventing and/or treating diseases related thereto, as well as for preventing and/or treating neuropathic pain.

According to another aspect thereof, it is an object of the invention to provide an association and/or composition of the invention for use in therapy, in particular for maintaining the well-being and health of the peripheral nervous system and for preventing and/or treating pathologies related thereto, in protecting peripheral nerve function, in preventing and/or treating peripheral neuropathies, and in preventing and/or treating neuropathic pain and stimulating the endogenous production of neurotrophic factors.

According to another aspect thereof, it is an object of the invention to provide a method for preventing and repairing damage to the central and peripheral nervous system and for preventing and/or treating pathologies related thereto, for protecting the functionality of the peripheral nervous system and for preventing and/or treating central and peripheral neuropathies, as well as for preventing and/or treating neuropathic pain and for stimulating the endogenous production of neurotrophic factors, comprising administering, to an individual in need thereof, the association and/or composition of the invention, preferably in a dose effective for the purpose.

Preferably, 1 tablet, for example weighing 125 mg, is administered, preferably twice daily.

Preferably 1 sachet, for example weighing 250 mg, is administered, preferably once daily.

Preferably, by means of the composition of the present invention, a daily dose of *Gastrodia elata* weighing 200 mg is administered, for example, by one administration of a sachet, or two administrations of a tablet.

Preferably, by means of the composition of the present invention, a daily dose of citicoline weighing 250 mg is administered, for example, by one administration of a sachet, or two administrations of a tablet.

Preferably, by means of the composition of the present invention, a daily dose of L-acetylcamitine or L-camitine or carnitine weighing 1,000 mg is administered, for example, by one administration of a sachet, or two administrations of a tablet.

Furthermore, the association or combination of the invention and the pharmaceutical and/or nutraceutical compositions containing it, due to the presence of natural ingredients do not have significant adverse effects, are free of side effects and have high tolerability, and can be administered to a wide category of individuals.

Finally, the combinations and/or compositions of the invention are easy to prepare and inexpensive.

Some examples of preferred embodiments of the compositions of the invention are given in the Experimental Section below solely by way of illustration and are in no way limiting.

As will be shown in the Experimental Section that follows and by the figures illustrating the results of the experimental assays conducted, the association/composition of the invention has been shown to be effective in treating disorders of the nervous system, in maintaining the state of health thereof, and in regenerating the central and peripheral nervous system.

Furthermore, the association (or combination) of the present invention has been shown to be more effective and safer than a composition of a supplement product on the market containing alpha-lipoic acid, L-acetylcarnitine, citicoline and B-group vitamins (CF), with which it was compared in the experimental studies reported herein.

**Brief Description of the Figures**

**Figure 1.** Analysis of different substances in a dose-response and time-dependent study evaluating cell viability by means of the MTT assay. Effects on cell viability of: In (**A**) Gastrodin; in (**B**) Citicoline; in (**C**) Acetyl-L-Camitine; in (D) Vit. B group. Data are expressed as the mean ± SD (%) of 5 independent experiments normalized to the control (0%)

line.

**Figure 2.** Analysis of cell viability (**A**) and ROS production (**B**) of the single components and their combinations in a time-dependent study. Data are expressed as the mean ± SD (%) of 5 independent experiments normalized to the control.

**Figure 3.** Permeability study on Caco-2 cells. In (**A**) TEER (transepithelial electrical resistance) value using EVOM3; from (**B-D**) analysis of TJ measured by means of the enzyme-linked immunosorbent assay (ELISA) (Occludin, Claudin1, and ZO-1 respectively); from (**B-D**), means ± SD are expressed comparing the data to the control (0% line), and all molecules result in * $p < 0.05$ vs control; α $p < 0.05$ vs Citicoline, Acetyl-L-Carnitine and Vit. B group; β vs Lipoic Acid; γ vs Gastrodin; the bars $p<0.05$ vs CF.

**Figure 4.** Analysis of 3D EngNT under physiological conditions. In (**A**) cell viability is measured by means of the MTT assay; (**B**) TNFα quantification by means of an ELISA kit; the ELISA kit measures (**C**) NGF activity; and (**D**) ERK/MAPK activity measured with the ELISA kit. The data are the mean ± SD of five independent experiments performed in triplicate and normalized to control values (0% line). * $p < 0.05$ vs control; α $p < 0.05$ vs Citicoline, Acetyl-L-Carnitine and Vit. B group; β vs Lipoic Acid; γ vs Gastrodin; the bars $p<0.05$ vs CF.

**Figure 5.** Analysis of the effects of single agents and combinations under PNI conditions. In (**A**) cell viability by MTT; in (**B**) analysis of ROS production measured by cytochrome C reduction; in (**C**) TNFα quantification by ELISA; and in (**D**) IL-2 analysis by ELISA. The data are the means ± SD (%) of five independent experiments performed in triplicate and normalized to control values (0% line). GGF = glial growth factor 2; * $p < 0.05$ vs control; α $p < 0.05$ vs Citicoline, Acetyl-L-Carnitine and Vit. B group; β vs Lipoic Acid; γ vs Gastrodin; δ $p<0.05$ vs GGF 200ng/mL; the bars $p<0.05$ vs CF.

**Figure 6.** Analysis of single agents and combinations on NaV channels and GABA activity under PNI conditions. In (**A**) Nav 1.7 protein activity; in (**B**) Nav 1.8 protein activity; in (**C**) GABA-A activity. All these tests were performed with a specific ELISA kit. The data are the means ± SD (%) of five independent experiments performed in triplicate and normalized to control values (0% line). GGF = glial growth factor 2; * $p < 0.05$ vs control; α $p < 0.05$ vs Citicoline, Acetyl-L-Carnitine and Vit. B group; β vs Lipoic Acid; γ vs Gastrodin; δ $p<0.05$ vs GGF 200ng/mL; the bars $p<0.05$ vs CF.

**Figure 7.** Analysis of single agents and combinations on SNP molecular marker under PNI conditions. In (**A**) p75 activity measured by ELISA; in (**B**) myelin protein zero (MPZ) measured by ELISA; in (**C**) neuregulin 1 (NRG1) measured by ELISA; in (**D**) estrogen receptor beta (ERb) measured by ELISA; in (**E**) NGF activity measured with the ELISA kit. The data are the means ± SD (%) of five independent experiments performed in triplicate and normalized to control values (0% line). GGF = glial growth factor 2; * $p < 0.05$ vs control; α $p < 0.05$ vs Citicoline, Acetyl-L-Carnitine and Vit. B group; β vs Lipoic Acid; γ vs Gastrodin; δ $p<0.05$ vs GGF 200ng/mL; the bars $p<0.05$ vs CF.

**EXPERIMENTAL PART 1**

Example 1A

Composition in tablet form (e.g., 1 tablet twice a day) containing:

- 100 mg or 200 mg of *Gastrodia elata* extract 50% polysaccharides
- 125 mg of citicoline
- 500 mg L-acetylcarnitine and/or carnitine
- 12.5 mg vitamin B1 + 12.5 mg vitamin B2 + 9 mg vitamin B5 + 5 mg vitamin B6 + 16.5 μg vitamin B12 and, optionally,
- excipients and/or additives of food or pharmaceutical grade.

Example 1B

Composition in tablet form (e.g., 1 tablet twice a day) containing:

- 100 mg or 200 mg of *Gastrodia elata* extract 10:1
- 125 mg of citicoline
- 500 mg L-acetylcarnitine and/or carnitine
- 12.5 mg vitamin B1 + 12.5 mg vitamin B2 + 9 mg vitamin B5 + 5 mg vitamin B6 + 16.5 μg vitamin B12 and, optionally,
- excipients and/or additives of food or pharmaceutical grade.

Example 2A

Composition in sachet form (e.g., 1 sachet once daily) containing:

- 200 mg or 400 mg of *Gastrodia elata* extract 50% polysaccharides

- 250 mg of citicoline
- 1,000 mg L-acetylcamitine and/or carnitine
- 25 mg vitamin B1 + 25 mg vitamin B2 + 18 mg vitamin B5 + 10 mg vitamin B6 + 33 μg vitamin B12 and, optionally,
- excipients and/or additives of food or pharmaceutical grade.

Example 2B

Composition in sachet form (e.g., 1 sachet once daily) containing:

- 200 mg or 400 mg of *Gastrodia elata* extract 10:1
- 250 mg of citicoline
- 1,000 mg L-acetylcarnitine and/or carnitine
- 25 mg vitamin B1 + 25 mg vitamin B2 + 18 mg vitamin B5 + 10 mg vitamin B6 + 33 μg vitamin B12 and, optionally,
- excipients and/or additives of food or pharmaceutical grade.

The activity of the associations or combinations, and of the compositions containing them, was evaluated on the basis of several experimental assays.

1. Introduction

The present study aims to replace lipoic acid with an extract of Gastrodia, preferably a *Gastrodia elata* extract 10:1 called gastrodin, combined with citicoline, carnitine, and B-group vitamins to improve the antioxidant and anti-inflammatory properties thereof in order to reduce pain signaling in an *in vitro* model.

Compositions CI and CF differ from each other given the presence of gastrodin in CI and lipoic acid in CF.

2. Results

*2.1 Dose-Response Study of the Single Components on* Caco-2

Before exploring the potential beneficial effects of the new formulation against neuropathic pain, the effects of the single agents on the viability of the Caco-2 cell line were evaluated using a dose-response study to verify the toxicological analysis, considering a period of between 2h and 6h as the relevant exposure time. As shown in Figure 1A, all substances improved Caco-2 cell viability, as reported in the literature, compared to the control ($p<0.05$). These effects are more relevant considering Gastrodin 25μM, since it was able to improve mitochondrial metabolism by about 40% compared to the control ($p<0.05$) and shows a better profile in terms of cell viability compared to Gastrodin 50μM and Gastrodin 100μM ($p<0.05$). In addition, regarding Citicoline and Acetyl-L-Carnitine, although they have a similar time course profile, Citicoline 250mM and Acetyl-L-Carnitine 7.5μM demonstrated a better trend compared to the other two concentrations selected ($p<0.05$). Finally, only B-group vitamins (Vit. B group) 1mg/mL exerted more beneficial effects compared to 100μg/mL and 200μg/mL ($p<0.05$), which may explain the agent-induced self-protective response. For this reason, the concentration able to maintain or produce a minimal increase in cell viability ($p<0.05$ vs. control) was selected for each agent to be used in the final formulation. In particular, Gastrodin 25μM, Citicoline 250mM, Acetyl-L-Carnitine 7.5μM and Vit. B group 1 mg/mL were selected.

Indeed, when all the agents were added together (Figure 2A), significant changes were observed ($p<0.05$), indicating the maintenance of a self-protection response also by the complete formulation of the present invention (abbreviated CI) without lipoic acid, but with Gastrodin or *Gastrodia* extract 10:1, and hence the possibility of a synergistic effect using all substances in the same formulation. Additionally, CI showed a better performance compared to the other substances tested ($p<0.05$), particularly compared to CF (containing Lipoic Acid, Citicoline, Acetyl-L-Carnitine and Vit. B group) ($p<0.05$). This may be due to Gastrodin's greater effectiveness compared to Lipoic Acid ($p<0.05$), as it enhances mitochondrial metabolism and produces a more favorable response. The same results were also found when analyzing ROS production (Figure 2B), since CI exerted a significant effect compared to the other substances tested ($p<0.05$).

*2.2. Permeability and Absorption Mechanisms Analyzed in an In Vitro Intestinal Barrier Model*

Based on the results obtained in the study's first phase, additional investigations were performed to acquire key information on intestinal absorption and transport mechanisms, by constructing an *in vitro* intestinal barrier model. In this context, the transepithelial electrical resistance (TEER), intestinal absorption and the activity of TJs, such as occludin, claudin1, and ZO-1, were evaluated. As reported in Figure 3A, the TEER analysis shows that all tested agents actively support optimal absorption and maintain correct intestinal homeostasis ($p<0.05$). Specifically, the passage through the

intestinal epithelium demonstrates that Gastrodin 25μM maintained epithelial integrity, while increasing the ion flux of the paracellular exchanges across the intestinal epithelium better than the other single agents ($p < 0.05$). The enhancing effect of Gastrodin 25μM is particularly appreciable when it is combined with Citicoline 250mM, Acetyl-L-Carnitine 7.5μM and Vit. B group 1mg/mL, since CI showed more beneficial effects than CF ($p<0.05$). These data were confirmed with the TJ analysis; Figure 3B-D Gastrodin 25μM exerted the most significant effects compared to the other single agents for all the TJs tested (p<0.05). Moreover, in this case, when Gastrodin was combined in the formulation, CI exerted the most significant effect compared to CF as regards claudin-1 (about 51%, $p < 0.05$), occludin (about 47%, $p < 0.05$) and ZO-1 (about 57%, $p < 0.05$) and compared to the control value (shown as the 0-line, $p < 0.05$). Furthermore, these data are supported by Gastrodin 25μM alone, which demonstrated ameliorative effects on the TJs compared to Lipoic Acid 50μM ($p<0.05$). Finally, the data from the absorption analysis (Table 1) supports the results reported above, since Gastrodin 25μM on its own possessed a slightly higher bioavailability than Lipoic Acid 50μM ($p < 0.05$), demonstrating the ability to cross the intestinal barrier in a particularly efficient way and allowing CI to have a higher absorption profile compared to CF ($p<0.05$). All these results revealed that the combination of Gastrodin 25μM, Citicoline 250mM, Acetyl-L-Carnitine 7.5μM and Vit. B group 1mg/mL has a better absorption profile than CF, confirming an improvement in bioavailability and suggesting a synergistic effect of the combination.

Absorption Rate obtained from the application of the following formula: J = Jmax [C]/(Kt + [C]).

Table 1

| | 2h | 3h | 4h | 5h | 6h |
|---|---|---|---|---|---|
| Gastrodin | 74.04% | 83.14% | 88.1% | 77.76% | 69.9% |
| Citicoline | 37.28% | 41.36% | 45.5% | 37.23% | 33.09% |
| Acetyl-L-Camitine | 69.49% | 77.35% | 80.65% | 69.90% | 54.16% |
| Vit. B Group | 58.32% | 67.01% | 71.97% | 59.15% | 49.63% |
| CI | 82.31% | 91.42% | 96.38% | 93.07% | 88.93% |
| Lipoic acid | 47.98% | 49.27% | 53.77% | 51.71% | 41.36% |
| CF | 74.46% | 82.72% | 86.88% | 84.79% | 78.59% |

*2.3. Effects of Single Components and Combinations on 3D EngNT Co-Cultures*

Considering the data obtained from the absorption analysis, further experiments were conducted on 3D engineered neural tissue (3D EngNT) to investigate the ability of Gastrodin 25μM, Citicoline 250mM, Acetyl-L-Carnitine 7.5μM and Vit. B group 1mg/mL, alone and in combination, to reach and act on the peripheral nerve after intestinal passage under physiological conditions. All data were compared with CF and its main ingredient. As shown in Figure 4, all the single agents tested were able to reach the target site after intestinal passage without negatively affecting mitochondrial metabolism or the inflammation response ($p < 0.05$). In particular, Gastrodin 25μM was able to amplify the cell viability compared to the other single agents ($p < 0.05$, Figure 4A), demonstrating an ability to maintain mitochondrial well-being and, at the same time, showing an ability to reduce the amount of inflammatory markers ($p < 0.05$). Gastrodin 25μM also exerted a significant effect on cell viability and TNFα production (approximately 44% and 33%, respectively, $p < 0.05$) compared to Lipoic Acid 50μM. Moreover, CI induced a significant effect compared to CF in both the parameters tested (about 15% in the case of cell viability and 24% in the case of TNFα production, $p < 0.05$), demonstrating the higher effectiveness of this combination. NGF activity was analyzed to confirm their effects under physiological conditions, since it is a pleiotropic factor involved in growth, differentiation, and repair of neuronal damage. As shown in Figure 4C, in this case Gastrodin 25μM exerted the most beneficial effect compared to the single agents (p < 0.05), demonstrating this substance's effectiveness compared to the raw materials already available on the market as well (p<0.05). Moreover, this advantage was confirmed by its combination with the other substances: CI induced a significant impact compared to CF (about 42% $p < 0.05$). Finally, the activity of ERK/MAPK was also analyzed considering that, following nerve injury, ERK phosphorylation increases. Indeed, it is known that nerve injury-induced phosphorylation of ERK occurs early and is long-lasting, so regulation of ERK/MAPK may be considered a promising therapeutic target for the treatment of neuropathic pain. As expected, the effect of Gastrodin 25μM was greater than that of Lipoic Acid 50μM (about 16%, p<0.05); as a final confirmation of the effects observed so far, in this case as well the greatest effect was obtained following treatment with CI (p < 0.05).

*2.4. Effects of Single Components and Combinations on an In Vitro Model of Peripheral Nerve Injury (PNI)*

To replicate a strong demyelination before stimulation with the two formulations and the single drugs, the 3D EngNT was pre-treated with 200 ng/mL glial growth factor 2 (GGF) starting on day 14 of maturation. This was necessary since the study needed to replicate the peripheral nerve tissue damage *in vitro.* Therefore, further studies were conducted to investigate the effects on cell viability and ROS production (Figures 5A-B). As expected, nerve tissue treated with only 200 ng/mL GGF produced a considerable quantity of ROS and significantly less biological activity ($p$<0.05). In contrast, both the individual components and formulations were able not only to re-establish physiological conditions, but also to induce a significant increase in cell viability and a decrease in ROS production ($p$<0.05). Indeed, CI increased cell viability by about 19% and decreased ROS production by almost 4 times compared to CF ($p$<0.05). Additionally, as regards the inflammatory panel, even though all the agents were able to reduce the production of TNF$\alpha$ and IL2 compared to GGF 200ng/mL alone ($p$<0.05), once again CI demonstrated more significant effects also compared to CF, with a 3-fold decrease in TNF$\alpha$ production and 2.7-fold decrease in IL2 expression ($p$<0.05). This is due to the presence of Gastrodin 25$\mu$M in the formulation, which, thanks to its powerful antioxidant effect, can reverse the damaging effects synergistically with the other components of the formulation.

Additionally, considering that Nav1.7 and Nav1.8 are sodium channels that are highly expressed in pain pathophysiology, further analyses were performed on their protein activity. In particular, sodium channels are naturally involved in transmitting information from pain receptors in the PNS to the central nervous system (CNS). As shown in Figure 6 (Panels A-B), all the tested agents were able to induce a decrease in both Nav1.7 and Nav1.8 proteins compared to GGF 200ng/mL (p<0.05). Unsurprisingly, in this case as well, the main effects were obtained with the novel formulation CI, which induced an almost 2.5-fold decrease in NaV1.7 protein activity compared to CF and a nearly 10-fold reduction in NaV1.8 protein activity compared to CF (p<0.05). Specifically, these data demonstrate that CI can reduce pain by reducing the activity of both the NaV 1.7 and NaV 1.8 channels, which serve to modulate pain. Several studies have looked at the function of GABA receptors near sodium channels, which facilitate spike propagation in myelinated axons (Figure 6 C). Notably, the analysis of the activity of GABA, whose functionality was statistically increased by the treatment with all the agents on their own compared to GGF 200ng/mL (p<0.05), further supported the beneficial effects of Gastrodin 25$\mu$M; indeed, aided by its presence, CI brought about a higher decrease in GABA-A activity than CF (by approximately one time more, p<0.05).

Lastly, the *in vitro* modulation of specific molecular pathways involved in neuropathic pain was investigated. As shown in Figure 7A, our results illustrated how Gastrodin is efficient in restoring myelination following nerve injury caused by 200 ng/mL GGF (p<0.05), as demonstrated by the analysis of p75 and, consequently, the inhibition of MPZ activity. Specifically, as can be seen in Figure 7A, CI was able to completely reverse the damage induced by GGF 200ng/mL (by about 77% more, p<0.05), while also causing an increase in p75 activity compared to CF (about 59%, p<0.05). Moreover, the activity of MPZ, another marker involved in maintaining the myelin sheath, was improved following the administration of Gastrodin 25$\mu$M, Citicoline 250mM, Acetyl-L-Carnitine 7.5$\mu$M and Vit. B group 1mg/mL individually, but particularly when combined in CI (p<0.05). Under these circumstances, Figure 7B illustrates the beneficial activity of Gastrodin 25$\mu$M vs GGF 200ng/ml (p<0.05), and subsequently, the impact of CI vs both the latter and CF (about 1.5 times and 70% higher, respectively, p<00.5).

Other evidence of the amelioration of the nerve injury and restoration of the myelination process was given by the analyses of NRG1 and ERb activity (Figures 7 C-D). In detail, regarding NRG1 activity, Lipoic Acid 50$\mu$M was not able to improve it compared to GGF 200ng/mL (p<00.5), while its combination in the CF formulation achieved this result; in contrast, both Gastrodin 25$\mu$M and, particularly, CI reversed GGF 200ng/mL-induced damage, improving NRG1 activity (by about 18% and 31% respectively, p<0.05). Furthermore, CI achieved a significant activity compared to CF (about 58% higher, p<0.05), demonstrating the higher efficacy of this novel formulation. Moreover, the analysis of ERb activity revealed a similar trend: the PNI condition impacted its activity, but the administration of all the agents tested showed a reversal of and recovery from this condition (p<0.05). In particular, the more evident effects were obtained with Gastrodin 25$\mu$M and, particularly, with CI, and the latter exerted a significant influence compared to CF (about 33% higher, $p$<0.05). The analysis of NGF activity gives final evidence about the restoration of the correct mechanism. In this case as well, Gastrodin 25$\mu$M on its own exerted a higher effect than Lipoic Acid 50$\mu$M (p<0.05), and, when included in the novel combination, it tends towards a synergistic effect which improves the self-restoration response. Indeed, the most significant impact is exerted by CI, with a substantial increase in NGF activity compared to CF (approximately 30%, $p$<0.05).

3. Discussion

Numerous non-pharmacological treatments, including non-invasive methods like exercise therapy, integrated cognitive behavioral therapy, and nutritional supplements, have been suggested to treat neuropathic pain. Indeed, in recent years, there has been a lot of interest in so-called "nutraceuticals" and other non-pharmacological supplements, which may be able to complement current pharmaceutical-based treatment for chronic neuropathic pain. In this context,

numerous nutraceuticals have emerged recently to treat neuropathic pain in a variety of illnesses, including peripheral neuropathy, diabetic neuropathy, and neuropathic pain associated with chemotherapy [33]. The present investigation aimed to find an effective new food supplement that could aid patients with PNI in managing their discomfort. Specifically, the effects of *Gastrodiae Rhizoma* dry extract 10:1 (called OXADIA or Gastrodin or Gastrodia extract 10:1) were investigated compared to Lipoic Acid and in combination with Acetyl-L-Carnitine, Citicoline and vitamins of the B group (B1, B2, B5, B6 and B12) to evaluate whether their combination (called CI) could be more effective than a CF containing Lipoic Acid. Gastrodin demonstrated optimal results as regards cell viability and less involvement of oxidation inducing mechanisms compared with Lipoic Acid alone, a component of the original formulation or CF. This allowed us to confirm the lower irritant action at the intestinal level caused by the nutraceutical component under investigation, while at the same time reinforcing any complications reported for the oral use of Lipoic Acid [10]. This was also confirmed by the increased activity of intestinal TJs, which are crucial parameters in evaluating an epithelial monolayer set up *in vitro.* Indeed, analyses of Zo-1, which mediates adhesion, claudin-4, which maintains the structure, and occludin, which contributes to stabilization, confirm proper gut function. In addition, TEER analysis confirms a better active role of Gastrodin compared with Lipoic Acid, suggesting better uptake. All this information was confirmed by a good absorption rate obtained by correlating it with the results of the fluorescent probe used. Indeed, it demonstrated a better percentage of absorption than Lipoic Acid alone; similar results can also be found in the two compositions, with CI exerting a better effect than CF. After intestinal passage, all the nutraceutical substances under examination were characterized on the specific target of the study, a 3D nervous system. Specifically, the antioxidant and anti-inflammatory action described in the literature [35] were evaluated. Indeed, the production of TNF$\alpha$, an inflammatory marker, was suppressed after stimulation with Gastrodin, indicating lower neuronal damage. Furthermore, mechanisms that are fundamental in maintaining viability, functionality, and cell survival were analyzed; in particular, NGF activity was analyzed. NGF was the first neurotrophin identified as having a key role in the survival and function of sensory and sympathetic neurons in the peripheral nervous system (PNS) and of cholinergic neurons of the basal forebrain in the CNS [36]. Therefore, its increased activity is directly related to better cell viability. ERK/MAPK evaluation was used fundamentally because the ERK/MAPK pathway, traditionally associated with important roles in cell proliferation and differentiation, has been found to have strong relevance at the peripheral level in maintaining neuronal plasticity. In addition, ERK/MAPK activation has recently been shown to contribute to nociceptive responses that follow inflammation and/or nerve injury [30]. Indeed, the ERK/MAPK biological system was also evaluated and Gastrodin was found to exert the most beneficial effect compared to other individual samples ($p<0.05$). Moreover, in this case the superiority of CI compared to CF was assessed. Based on these data obtained under physiological nervous conditions, our research proceeded with characterization at the level of a 3D nervous model under conditions of damage (induced with 200 ng/ml GGF), resulting in demyelination. In this study phase, the findings largely confirmed the antioxidant (reduction of ROS levels) and anti-inflammatory (lowering of TNF$\alpha$ levels and IL-2 levels) effects promoted by Gastrodin, which were amplified after treatment with CI thanks to the antioxidant and anti-inflammatory effects of the other components, compared to CF and Lipoic Acid ($p<0.05$). Specifically, CI countered the inflammatory mechanism and oxidative stress. Furthermore, normal peripheral nerve growth, migration, and differentiation of Schwann cells are regulated by NRG1 activating ERb receptors; the condition of PNI modifies NRG1/ERb signaling by disturbing the balance between NRG1 isoforms and reducing the expression of multiple molecules involved in cell survival [37]. While the disruption of NRG-1/ERb B2 signaling and impaired neurotrophic support are undoubtedly linked to the degeneration of sensory neurons in PNI, most likely in the biological activity of Schwann cells, treatment with CI restored the altered neurotropism, preventing damage to motor fibers and slowing down nerve conduction, thus demonstrating its neurotropic properties.

In addition, our research also allowed us not only to outline the role of the proposed new formulation from an antioxidant and anti-inflammatory point of view, but also to characterize the effect on the mechanisms directly involved in initiating neuropathic pain. In the literature, the role of sodium channels Nav1.7 and Nav1.8, widely expressed in the pathophysiological condition of pain, has been defined as crucial [32]. The analyses of their activity confirmed the effects of the analyzed substance, as they were all able to decrease the expression and the activity of Nav1.7 and Nav1.8, especially after treatment with CI; the same results were also observed for GABA-A, an important inhibitory neurotransmitter in the mammalian CNS [38]. Furthermore, the data revealed that CI appears to be able to repair damage to the myelin sheath that protects the axon and simultaneously to act on NGF release and bind the p75 neurotrophin receptor, reproducing the mechanism of analgesia observed in humans and mimicking, *in vitro,* the neuropathic pain condition, specifically inflammation and oxidative stress leading to an imbalance of ion transport. Indeed, as previously shown [39], a rise in NGF levels in various inflammatory situations may be a key feature of the human chronic pain syndrome. As a result, lowering the inflammatory response is critical for mitigating the detrimental effects of chronic pain. Conversely, pain is associated with a functional imbalance between MPZ, ERb and NRG1, whose relative balance is crucial for maintaining Schwann cell homeostasis during PNI. NRG1 normally activates ERB receptors in peripheral nerves to regulate Schwann cell growth, migration, differentiation, and dedifferentiation; however, PNI alters NRG1/ERb signaling by disrupting the balance between NRG1 isoforms, decreasing the expression of several molecules involved in cell survival, and thus activating the MAPK pathway [40]. While sensory neurodegeneration in PNI is associated with impaired neurotrophic support and disruption of NRG-1/ERb signaling, presumably in the biological activity of Schwann cells, CI treatment

restored the altered neurotropism, preventing nerve conduction slowing and motor neuron damage. All these outcomes indicate the potential usability of a novel formulation composed of Gastrodin (OXADIA) 25μM, Citicoline 250mM, Acetyl-L-Carnitine 7.5μM and Vit. B group 1mg/mL, capable of crossing biological membranes, notably the intestinal barrier, and reaching the target location, showing optimal effects in an *in vitro* model of PNI.

4. Materials and Methods

*4.1 Preparation of Agents*

*Gastrodiae elata* dry extract 10:1 from rhizoma (called OXADIA™), Citicoline, Acetyl-L-Carnitine and Vitamins of group B (Vit. B group, composed of 22.5% vitamins B1 and B2, 16.2% vitamin B5, 9% vitamin B6 and 29.7% vitamin B12) were tested alone and in combination to verify their effectiveness in crossing the intestinal barrier and reaching the peripheral nervous system (PNS). Specifically, Gastrodin was tested in a range from 25μM to 100μM [41], Citicoline in a range from 100mM to 300mM [42], Acetyl-L-Carnitine in a range from 7.5μM to 30μM [43] and Vit. B group from 100μg/mL to 1mg/mL [44, 45]. All substances tested were prepared directly in Dulbecco's Modified Eagle's Medium (DMEM, Merck Life Science, Rome, Italy) without phenol red and supplemented with 0.5% fetal bovine serum (FBS; Merck Life Science, Rome, Italy), 2 mM L-glutamine (Merck Life Science, Rome, Italy), and 1% penicillin-streptomycin (Merck Life Science, Rome, Italy) for all analyses. Based on their beneficial effects, the best concentration of each substance was selected and combined in a new formulation composed of Gastrodin 25μM, Citicoline 250mM, Acetyl-L-Carnitine 7.5μM and Vit. B group 1mg/mL (called CI). Moreover, CI was analyzed throughout the project by comparing its effects with the individual active ingredients, Lipoic Acid alone and a CF (composed of Lipoic Acid, Citicoline, Acetyl-L-Carnitine and Vit. B group). The Lipoic Acid was used at a concentration of 50μM as reported in the literature [46], and the CF was tested at the same dosage as CI, containing the same percentage of Citicoline, Acetyl-L-Carnitine and Vit. B group. Furthermore, all substances tested were prepared as already mentioned and donated by AGAVE S.r.l., which obtained them from Vivatis Pharma GBHE.

*4.2 Cell Culture*

The human epithelial intestinal Caco-2 cell line was used as a model to predict the features of human intestinal absorption following oral intake [47, 48]. The cells were supplied by the American Type Culture Collection (ATCC) and cultured in Dulbecco's Modified Eagle's Medium Advance (DMEM-Adv, Thermo Fisher Scientific, Rodano, MI, Italy) supplemented with 5% FBS, 2 mM L-glutamine, and 1% penicillin-streptomycin, maintained in an incubator at 37°C and 5% $CO_2$ and 95% of humidity. Experiments were performed using Caco-2 cells at passage numbers of between 26 and 32 to maintain the physiological balance among paracellular permeability and transport properties [49]. Cells were plated differently: 1 x $10^4$ cells on 96-well plates to study cell viability with the MTT-based In Vitro Toxicology Assay Kit (Merck Life Science, Rome, Italy) and ROS production, which were tested after synchronizing the cells for 8 hours with DMEM without red phenol and supplemented with 0.5% FBS, 2 mM L-glutamine, and 1% penicillin-streptomycin, maintaining the cells in an incubator at 37 °C with 5% $CO_2$. In addition, 2 x $10^4$ cells were plated on 6.5 mm Transwell® (Coming® Costar®, Merck Life Science, Rome, Italy) with a 0.4 μm pore polycarbonate membrane insert (Corning® Costar®, Merck Life Science, Rome, Italy) in a 24-well plate to perform the absorption analyses [50].

The rat-derived Schwann cell line RSC-96, purchased from ATCC, was cultured in DMEM-Adv (Thermo Fisher Scientific, Rodano, MI, Italy) supplemented with 5% FBS, 2 mM L-glutamine, and 1% penicillin-streptomycin [51] maintained in an incubator at 37°C with 5% $CO_2$ and 95% humidity. Experiments were conducted using RSC96 cells between 10 and 15 passages, subculturing 2-3 times a week.

The rat neuronal cell line PC12, supplied by ATCC, was cultured in Roswell Park Memorial Institute-1640 Advanced medium (RPMI-Adv, Thermo Fisher Scientific, Rodano, MI, Italy) supplemented with 2 mM glutamine, 5% horse serum (HS; Merck Life Science, Rome, Italy), and 5% FBS. The cultures were maintained at sub-confluency in an incubator at 37°C, with 5% $CO_2$ and 95% humidity; the cells used for the experiments had between 3 and 13 passages [52]. PC12 cells are among the most suitable and frequently employed neuronal cell lines for *in vitro* screening for neuroprotective compounds [53]. 4 x $10^6$ RSC96 cells and 1 x $10^5$ PC12 cells were plated in a co-culture system to reproduce *in vitro* the 3D EngNT, creating the peripheral nerve environment *in vitro* model [51].

*4.3 Experimental Protocol*

This study was carried out in three different phases; the starting point of the study protocol was intended to study the absorption and transport processes of the novel formulation and individual components through the intestinal barrier using a 3D *in vitro* model with Caco-2 cells [49]. The analyses performed regarded the mitochondrial metabolism and cellular well-being, oxidative stress, permeability and measurement of transepithelial electrical resistance (TEER),

absorption, and tight junction (TJ) activity. In the second phase, the product metabolized by the intestinal cells was subsequentially in contact with a 3D model for studying the peripheral nerve (3D EngNT), evaluating cell metabolism, inflammation, and some molecular pathways. Finally, in the third phase, peripheral nerve damage and demyelination were simulated *in vitro,* treating 3D EngNT with GGF 200ng/ml, to explore cell survival and mitochondrial activity, oxidative stress and ROS quantification, the inflammatory processes involved in neuropathy, nociceptive pathways for pain control, and the main pathways involved in the processes of myelin sheath protection and neurite formation [51].

*4.4 Cell Viability*

The MTT-based In Vitro Toxicology Assay Kit (Merk Life Science, Rome, Italy) was used to perform an assay in a 96 well-plate to determine cell-viability, following a classic protocol reported in the literature [49]. The absorbance of all solubilized samples (treated and untreated) was measured by means of a spectrophotometer (Infinite 200 Pro MPlex, Tecan, Männedorf, Switzerland) at 570 nm with correction at 690 nm and the results were expressed by comparing the data to the control sample (untreated samples defined as the 0% line) and reported as the means of five independent experiments performed in triplicate.

*4.5 ROS Production*

The ROS production was quantified by analyzing the reduction of cytochrome C using a standard protocol [49]. The absorbance was measured at 550 nm by means of spectrophotometer (Infinite 200 Pro MPlex, Tecan, Männedorf, Switzerland). The $O_2$ ratio was expressed as the mean $\pm$ SD (%) of nanomoles of reduced cytochrome C per microgram of protein compared to the control (untreated samples) of five independent experiments performed in triplicate.

*4.6 In vitro Intestinal Barrier Model*

An *in vitro* intestinal barrier model was created, following a standard protocol reported in the literature, using the Transwell® system to assess whether the substances used can cross the intestinal barrier [49, 54] following the protocol approved by the European Medicines Agency (EMA) and Food and Drug Administration (FDA) [55, 56] and which is used to predict absorption, metabolism, and bioavailability of several substances after oral intake in humans. Briefly, Caco-2 cells, plated on Transwell® inserts were maintained in a complete medium changed every two days, in both the basolateral and apical parts, for 21 days before the stimulation [49]. The TEER values were assessed using EVOM3 and STX2 chopstick electrodes (World Precision Instruments, Sarasota, FL, USA) throughout the entire maturation time, to evaluate mature intestinal epithelium formation and a proper paracellular mechanism. Absorption analysis started on the 21$^{st}$ day when TEER values were $\geq 400\ \Omega cm^2$ [57]. On the apical side, before the stimulation, the culture medium was verified to be at pH 6.5, which is similar to the pH in the lumen of the small intestine. In contrast, the pH of 7.4 on the basolateral side represented blood [49, 58]. The cells were stimulated with all substances from 2 to 6 hours before the subsequent analyses. At each time point, the uptake was measured using a fluorescent tracer at a concentration of 0.04% (Santa-Cruz, CA, USA [59]). The amount of fluorescein transferred was measured at 37 °C after incubating Caco-2 cells for 40 min at the above concentration. Fluorescence was detected with a fluorescence spectrophotometer (multilabel plate reader, VICTOR X4, Perkin Elmer, Waltham, MA, USA) at excitation/emission wavelengths of 490/514 nm. The results are expressed as the proportion (%) of the original amount permeating the cells. The permeation rate [nmol min (mg protein)], J, was calculated as follows:

$$J = Jmax\,[C]/(Kt + [C])$$

where:

- Jmax: maximum permeation rate;
- [C]: initial concentration of fluorescein;
- Kt: Michaelis-Menten constant.

The results are expressed as the mean $\pm$ SD (%), and negative controls without cells were tested to exclude influence by the Transwell® membrane.

*4.7 TJ analysis*

The Caco-2 lysates were used to analyze occludin activity with the Human Occludin (OCLN) ELISA Kit

(MyBiosource, San Diego, CA, USA), claudin-1 activity with an ELISA Kit (Cusabio Technology LCC, Huston, Houston, TX, USA) and Zona Occludens 1 (ZO-1) activity by means of the human tight junction protein 1 (TJP1) ELISA kit (MyBiosource, San Diego, CA, USA), following the manufacturer's instructions [49]. The absorbance was measured with a spectrophotometer (Infinite 200 Pro MPlex, Tecan, Männedorf, Switzerland) at 450 nm. The data were analyzed by comparing them to the standard curve (from 0 to 1,500 pg/mL for occludin, from 0 to 1,000 pg/mL for claudin-1 and ZO-1). They were expressed as a percentage (%) versus the control (0 line) of five independent experiments performed in triplicate.

4.8 3D *Set-up of EngNT Co-Cultures*

The interaction between RSC96 and PC12 cell lines is a key feature for mimicking *in vitro* the peripheral nerve environment, regenerating neurites, and supporting Schwann cells [51, 60, 61]. Based on the literature, a 3D nerve tissue model was prepared [51]. Briefly, 1 mL of a solution containing 80% (vol/vol) Type I rat tail collagen (2 mg/mL in 0.6% acetic acid, Thermo Fischer, Milan, Italy), 10% (vol/vol) Minimum Essential Medium (MEM, Merck Life Science, Milan, Italy), 5.8% (vol/vol) neutralizing solution (Biosystems, Monza, Italy), and 4.2% Schwann cell suspension (4 x $10^6$ RSC96 cells per 1 mL gel) was added to a rectangular scaffold with dimensions of 16.4 mm x 6.5 mm x 5 mm. When the gel had set, it was immersed in 10 mL DMEM and incubated at 37 °C with 5% $CO_2$ for 24 hours to permit cellular self-alignment; at the end, the gel was stabilized using plastic compression (120 g weight for 1 minute). Each stabilized aligned cellular gel was cut into equal segments according to the samples to be treated. Each gel segment was transferred to a 24-well plate, and 1 x $10^5$ PC12 was seeded on top of each segment to establish the co-cultures; this step is crucial in order to permit neurite extension across the horizontal plane which follows the aligned Schwann gels. The 24-well plate containing the gels was maintained in an incubator at 37°C with 5% $CO_2$ and 95% humidity to allow attachment of neuronal cells to the collagen gel; next, 1 mL of culture medium (DMEM, Merck Life Science, Rome, Italy) supplemented with 10% (vol/vol) FBS, 100 U/mL of penicillin, and 100 μg/mL of streptomycin, was added to each well.

*4.9 TNFα ELISA Assay*

TNFα quantification was obtained using the TNFα ELISA kit (Merck Life Science, Rome, Italy) according to the manufacturer's instructions [62]. The absorbance of the samples was measured at 450 nm using a plate reader (Infinite 200 ProMPlex, Tecan, Männedorf, Switzerland). The data were obtained and compared to the standard curve (ranging from 0 to 6000 pg/mL), and the results were expressed as a mean ± SD (%) versus the control (0 line) of five independent experiments performed in triplicate.

*4.10 Human Beta-NGF Assay*

The Rat beta-NGF ELISA kit (Abcam, Cambridge, UK) was used in cell lysates following the manufacturer's instructions [63]. Briefly, 100 μL of diluted samples were incubated overnight at 4 °C, washed four times with 1× Wash Buffer, and then 100 μL of detection antibody was added to each well and incubated for 1 h at room temperature with gentle shaking. Then, the wells were washed four times, and 100 μL of streptavidin-HRP and the plate were incubated for 45 min. After the incubation, the wells were washed again, and 100 μL of TMB substrate were added. Finally, the plate was incubated for 30 min at room temperature in the dark with gentle shaking, and the reaction was stopped with 50 μL of Stop Solution. The absorbance was measured by means of a spectrometer (Infinite 200 Pro MPlex, Tecan, Männedorf, Switzerland) at 450 nm and expressed as pg/mL compared to a standard curve (ranging from 15 to 15,000 pg/mL). The results were reported as the mean ± SD (%) versus the control of five independent experiments performed in triplicate.

*4.11 ERK/MAPK activity*

The PNS lysates were used to assess ERK/MAPK activity using the InstantOneTM ELISA kit (Thermo Fisher, Milan, Italy) following the manufacturer's instructions [59]. To each well of the InstantOne ELISA microplate strips, 50 μL of each lysate were added. The antibody cocktail and microplate strips were then incubated for 1 hour at room temperature on a microplate shaker. After 20 minutes of application of the detection reagent, the reaction was stopped by adding a stop solution. The Infinite 200 Pro MPlex (Tecan, Männedorf, Switzerland) spectrometer, which operates at a wavelength of 450 nm, was used to measure the strips. Average absorbance (%) compared to the control was used to represent the results.

*4.12 Interleukin-2 ELISA Assay*

Interleukin-2 was quantified using the Rat Interleukin-2 (IL-2) ELISA Kit (FineTest, Wuhan, China) on cell lysates according to the manufacturer's instructions [64]. The absorbance was read at 450 nm using a spectrophotometer (Infinite

200 ProMPlex, Tecan, Männedorf, Switzerland). A standard curve was plotted relating the intensity of the color (OD) to the concentration of standards (ranging from 31.25 to 2,000 pg/mL), and the results were expressed as the mean ± SD (%) versus the control (0 lines) of five independent experiments performed in triplicate.

*4.13 NAV1.7 ELISA Assay*

NAV1.7 was quantified using the SCN9A / Nav1.7 ELISA Kit (LifeSpan BioSciences, Lynnwood, Washington, USA) on co-culture lysates according to the manufacturer's instructions. Briefly, 100μL of each sample were incubated for 2h at 37°C on a pre-coated plate; after that, the samples were removed and 100μL of Detection Reagent A were added for 1h at 37°C. After the plate had been washed 3 times, 100μL of Detection Reagent B were added for 1h at 37°C. The plate was washed again 5 times and 90μL of TMB Substrate were added. After 20 minutes at 37°C, 50 μL of Stop Solution was added to each well, and the absorbance was read at 450 nm using a spectrophotometer (Infinite 200 ProMPlex, Tecan, Männedorf, Switzerland). A standard curve was plotted relating the intensity of the color (OD) to the concentration of standards (ranging from 0.312 to 20 ng/mL), and the results were expressed as the mean ± SD (%) versus the control (0 lines) of five independent experiments performed in triplicate.

*4.14 NAV1.8 ELISA Assay*

NAV1.8 was quantified using the Mouse Sodium Channel Protein Type 10 Subunit Alpha (SCN10A) ELISA Kit (MyBiosource, San Diego, CA, USA) on co-culture lysates according to the manufacturer's instructions [65]. The absorbance was read at 450 nm using a spectrophotometer (Infinite 200 ProMPlex, Tecan, Männedorf, Switzerland). A standard curve relating the intensity of the color (OD) to the concentration of standards was plotted, and the results were expressed as the mean ± SD (%) versus the control (0 lines) of five independent experiments performed in triplicate.

*4.15 y-Aminobutyric Acid (GABA) ELISA Assay*

The Rat GABA (y-aminobutyric acid) ELISA Kit (FineTest, Wuhan, China) was used to perform an assay on cell lysates following the manufacturer's instructions [66]. The absorbance was measured at 450 nm through a plate reader (Infinite 200 ProMPlex, Tecan, Männedorf, Switzerland). The data were obtained and compared to the standard curve (6 to 400 pg/mL). The results were expressed as the mean ± SD (%) versus the control (0 lines) of five independent experiments in triplicate.

*4.16 p75 activity by NGFR ELISA Assay*

The Rat NGFR ELISA kit (MyBiosource, San Diego, CA, USA) was used on cell lysates according to the manufacturer's instructions [67]. The plate was read immediately at 450 nm using a spectrophotometer (Infinite 200 ProMPlex, Tecan, Männedorf, Switzerland). The data were obtained and compared to the standard curve (0.312 to 20 ng/mL). The results were expressed as the mean ± SD (%) versus the control (0 lines) of five independent experiments performed in triplicate.

*4.17 MPZ ELISA Assay*

MPZ production was determined using a Rat ELISA kit (My-BioSource, San Diego, CA, USA) on cell lysates as per the manufacturer's instructions [67]. The plate was read at 450 nm using a spectrophotometer (Infinite 200 ProMPlex, Tecan, Männedorf, Switzerland). The concentration was expressed as ng/mL compared to a standard curve (ranging from 0.06 to 18 ng/mL), and the results were reported as the mean ± SD versus the control (0 lines) of five independent experiments performed in triplicate.

*4.18 NRG1 ELISA Assay*

The NRG1 Rat ELISA Kit (FineTest, Wuhan, China) was used on cell culture supernatants as per the manufacturer's instructions [67]. The enzymatic reaction was measured by means of a spectrophotometer (Infinite 200 Pro MPlex, Tecan, Männedorf, Switzerland) at 450 nm. The results were obtained by comparing the data to the standard curve (ranging from 0.156 to 10 ng/mL). They were expressed as a percentage (%) versus the control (0 lines) of five independent experiments performed in triplicate.

*4.19 Estrogen Receptor β ELISA Assay*

The Rat Estrogen Receptor β (ERβ) ELISA Kit (Cloud-Clone, Houston, TX, USA) was used on cell lysates according to the manufacturer's instructions [68]. The absorbance was measured by means of a spectrophotometer (Infinite 200 ProMPlex, Tecan, Männedorf, Switzerland) at 450 nm, and expressed as pg/mL compared to a standard curve (ranging from 0.312 to 20 ng/mL). The results were reported as the mean ± SD (%) versus the control of five independent experiments performed in triplicate.

*4.20 Statistical Analysis*

One-way analysis of variance (ANOVA) and Bonferroni post hoc tests were used to process the acquired data using Prism GraphPad statistical software 9.4.1. The two-tailed Student's t-test was adopted to compare the two groups. A two-way ANOVA was conducted to evaluate multiple group comparisons, followed by a two-sided Dunnett post hoc test. The mean ± SD of at least five independent variables determined in triplicate was used to express all results.

5. Conclusions

In conclusion, this study demonstrated how the antioxidant and anti-inflammatory properties of Lipoic Acid can be replaced using Gastrodin as an alternative. Specifically, CI containing Gastrodin (OXADIA™) 25μM, Citicoline 250mM, Acetyl-L-Carnitine 7.5μM and Vit. Group B 1 mg/ml has proven to be an adequate alternative to common analgesic drugs to relieve the mechanism underlying the pain caused by PNI. Consequently, our findings support for the first time the concept that this novel formulation can efficiently reduce neuropathy by modifying the fundamental pain mechanism by stimulating the recovery mechanisms of PNS cells. Further, CI prevented motor fiber damage and the slowing down of nerve conduction by restoring the altered neurotropism. In conclusion, the current study gives new insights into the effects of nutraceuticals on neuropathic pain disorders and can be regarded as a novel and safe neuropathy treatment.

Abbreviations

3D EngNT: 3D engineered neural tissue
ADE: adverse drug event
ADR: adverse drug response
ANOVA: One-way analysis of variance
ATCC: American Type Culture Collection
BDNF: brain derived neurotrophic factor
CNS: central nervous system
DMEM-Adv: Dulbecco's Modified Eagle's Medium Advance
DMEM: Dulbecco's Modified Eagle's Medium
ELISA: Enzyme-Linked Immunosorbent Assay
EMA: European Medicines Agency
FBS: fetal bovine serum
FDA: Food and Drug Administration
GABA: γ-aminobutyric acid
GGF: glial growth factor 2
GLTs: glucose transporters
HBA: p-hydroxy benzyl alcohol
MEM: Minimum Essential Medium
NGF: nerve growth factor
OS: oxidative stress
PN: peripheral neuropathy
PNI: peripheral nervous injury
PNS: peripheral nervous system
RNS: reactive nitrogen species
ROS: reactive oxygen species
RPMI-Adv: Roswell Park Memorial Institute-1640 Advanced
TEER: transepithelial electrical resistance
TJ: tight junction

Vit. B group: Vitamins of group B
ZO-1: Zona Occludens 1

**Figure 8** shows the results of an evaluation, with a fluorescent tracer, of the passage through the intestinal barrier of two different extracts of *Gastrodia elata* (*Gastrodia elata* 10:1 and *Gastrodia elata* 50% polysaccharides), at concentrations of 100 mg and 200 mg, compared with Lipoic acid 300 mg, 600 mg and 800 mg. The reported data are expressed as the mean +/- SD of 5 independent experiments conducted in triplicate. For both extracts, the results obtained between 1 hour and 6 hours are statistically significant compared with the control ($p<0.05$). Figure 8 also shows the results of the permeability analysis.

PERMEABILITY ANALYSIS

Table 2

| | 100 mg | 200 mg | 300 mg |
|---|---|---|---|
| **Gastrodia 221008 10:1** | **40%** | **33%** | **23%** |
| **Gastrodia 202304032 50% polysaccharides** | **38%** | **31%** | **21%** |
| **Lipoic acid 600 mg** | **13%** | | |

**Figures 9A and 9B** show the results of a viability analysis (at the top) and an analysis of reactive oxygen species production (at the bottom). The results of the test are expressed as the mean ± SD (%) of 5 independent experiments conducted in triplicate.
**Figures 10A and 10B** show the results of an analysis of TEER over time (1 hour - 6 hours) and TJ activity. The reported data are expressed as the mean +/- SD of 5 independent experiments conducted in triplicate. For both tested extracts, the results obtained between 1 hour and 6 hours are statistically significant compared with the control ($p<0.05$). CF: composition of a commercial supplement product CF containing alpha-lipoic acid, L-acetylcamitine, citicoline and B-group vitamins.

Table 3

| PERMEABILITY | 2 hours | 4 hours | 6 hours |
|---|---|---|---|
| **MIX 1 Gastrodia 50% polysaccharides 100 mg** | **38%** | **45%** | **27%** |
| **MIX 2 Gastrodia 221008 10:1 100 mg** | **42%** | **48%** | **33%** |
| **CF** | **21%** | **30%** | **15%** |

Table 4

| PERMEABILITY | 2 hours | 4 hours | 6 hours |
|---|---|---|---|
| **MIX 1 Gastrodia 50% polysaccharides 200 mg** | **30%** | **38%** | **17%** |
| **MIX 2 Gastrodia 221008 10:1 200 mg** | **33%** | **40%** | **20%** |
| **CF** | **21%** | **30%** | **15%** |

**Figure 11** shows the results of an evaluation of intestinal absorption and permeability. The reported data are expressed as the mean +/- SD of 5 independent experiments conducted in triplicate. For all tested forms, the results obtained between 1 hour and 6 hours are statistically significant compared with the control ($p<0.05$).
**Figures 12A and 12B** show the results of an evaluation of cell survival and mitochondrial activity. The reported data are expressed as the mean +/- SD of 5 independent experiments conducted in triplicate. * $p<0.05$ vs control; bars $p<0.05$ vs main component; $\gamma$ $p<0.05$ vs CF.
**Figures 13A and 13B** show the results of an analysis of oxidative stress following treatment with the compositions metabolized at the intestinal level, mimicking neuropathic damage. The reported data are expressed as the mean +/- SD of 5 independent experiments conducted in triplicate. * $p<0.05$ vs control; bars $p<0.05$ vs main component; y $p<0.05$ vs CF; $\phi$ $p<0.05$ vs MIX1.

**Figures 14A and 14B** show the evaluation of inducible nitric oxide synthase (iNos) activity (at the top) and of the mitochondrial membrane potential (at the bottom). The reported data are expressed as the mean+/- SD of 5 independent experiments conducted in triplicate. * $p<0.05$ vs control; bars $p<0.05$ vs main component; y $p<0.05$ vs CF; $\phi$ $p<0.05$ vs MIX1.

**Figures 15A and 15B** show the results of an analysis of inflammatory processes (TNF$\alpha$ and NFkB production). The reported data are expressed as the mean+/- SD of 5 independent experiments conducted in triplicate. * $p<0.05$ vs control; bars $p<0.05$ vs main component; y $p<0.05$ vs CF; $\phi$ $p<0.05$ vs MIX1.

**Figures 16A and 16B** show the results of an analysis of inflammatory processes (production of IL1$\beta$, IL-2 and IL-6). The reported data are expressed as the mean+/- SD of 5 independent experiments conducted in triplicate. * $p<0.05$ vs control; bars $p<0.05$ vs main component; y $p<0.05$ vs CF.

**Figures 17A and 17B** show the results of an analysis of pain mechanisms (NaV 1.7 and NaV 1.8 activity). The reported data are expressed as the mean+/- SD of 5 independent experiments conducted in triplicate. * $p<0.05$ vs control; bars $p<0.05$ vs main component; y $p<0.05$ vs CF.

**Figure 18** shows the results of an analysis of pain mechanisms (GABA activity). The reported data are expressed as the mean+/- SD of 5 independent experiments conducted in triplicate. * $p<0.05$ vs control; bars $p<0.05$ vs main component; y $p<0.05$ vs CF; $\phi$ $p<0.05$ vs MIX1.

**Figures 19A and 19B** show the results of an analysis of p75 and MPZ activity. The reported data are expressed as the mean +/- SD of 5 independent experiments conducted in triplicate. * $p<0.05$ vs control; bars $p<0.05$ vs main component; y $p<0.05$ vs CF.

**Figures 20A and 20B** show the results of an analysis of ERB and NRG1 activity. The reported data are expressed as the mean+/- SD of 5 independent experiments conducted in triplicate. * $p<0.05$ vs control; bars $p<0.05$ vs main component; y $p<0.05$ vs CF.

**Figure 21** shows the results of an analysis of NGF activity. * $p<0.05$ vs control; bars $p<0.05$ vs main component; y $p<0.05$ vs CF.

## EXPERIMENTAL PART 2

Example 1A (MIX 1)

Composition in tablet form (e.g., 1 tablet twice a day) containing:

- 100 mg or 200 mg of *Gastrodia elata* extract 50% polysaccharides
- 125 mg of citicoline
- 500 mg of L-acetylcarnitine and/or carnitine
- 12.5 mg of vitamin B1 + 12.5 mg of vitamin B2 + 9 mg of vitamin B5 + 5 mg of vitamin B6 + 16.5 $\mu$g of vitamin B12 and, optionally,
- excipients and/or additives of food or pharmaceutical grade.

Example 1B (MIX 2)

Composition in tablet form (e.g., 1 tablet twice a day) containing:

- 100 mg or 200 mg of *Gastrodia elata* extract 10:1
- 125 mg of citicoline
- 500 mg of L-acetylcarnitine and/or carnitine
- 12.5 mg of vitamin B1 + 12.5 mg of vitamin B2 + 9 mg of vitamin B5 + 5 mg of vitamin B6 + 16.5 $\mu$g of vitamin B12 and, optionally,
- excipients and/or additives of food or pharmaceutical grade.

Example 2A (MIX 1)

Composition in tablet form (e.g., 1 tablet twice a day) containing:

- 100 mg or 200 mg of *Gastrodia elata* extract 50% polysaccharides
- 125 mg of citicoline
- 500 mg of L-acetylcarnitine and/or carnitine
- 25 $\mu$g of vitamin D2 and/or D3
- 12.5 mg of vitamin B1 + 12.5 mg of vitamin B2 + 9 mg of vitamin B5 + 5 mg of vitamin B6 + 16.5 $\mu$g of vitamin B12 and,

optionally,
- excipients and/or additives of food or pharmaceutical grade.

Example 2B (MIX 2)

Composition in tablet form (e.g., 1 tablet twice a day) containing:

- 100 mg or 200 mg of *Gastrodia elata* extract 10:1
- 125 mg of citicoline
- 500 mg of L-acetylcarnitine and/or carnitine
- 25 μg of vitamin D2 and/or D3
- 12.5 mg of vitamin B1 + 12.5 mg of vitamin B2 + 9 mg of vitamin B5 + 5 mg of vitamin B6 + 16.5 μg of vitamin B12 and, optionally,
- excipients and/or additives of food or pharmaceutical grade.

Example 3A (MIX 1)

Composition in sachet form (e.g., 1 sachet once daily) containing:

- 200 mg or 400 mg of *Gastrodia elata* extract 50% polysaccharides
- 250 mg of citicoline
- 1,000 mg of L-acetylcamitine and/or carnitine
- 25 mg of vitamin B1 + 25 mg of vitamin B2 + 18 mg of vitamin B5 + 10 mg of vitamin B6 + 33 μg of vitamin B12 and, optionally,
- excipients and/or additives of food or pharmaceutical grade.

Example 3B (MIX 2)

Composition in sachet form (e.g., 1 sachet once daily) containing:

- 200 mg or 400 mg of *Gastrodia elata* extract 10:1
- 250 mg of citicoline
- 1,000 mg of L-acetylcamitine and/or carnitine
- 25 mg of vitamin B1 + 25 mg of vitamin B2 + 18 mg of vitamin B5 + 10 mg of vitamin B6 + 33 μg of vitamin B12 and, optionally,
- excipients and/or additives of food or pharmaceutical grade.

Example 4A (MIX 1)

Composition in sachet form (e.g., 1 sachet once daily) containing:

- 200 mg or 400 mg of *Gastrodia elata* extract 50% polysaccharides
- 250 mg of citicoline
- 1,000 mg of L-acetylcamitine and/or carnitine
- 50 μg of vitamin D2 and/or D3
- 25 mg of vitamin B1 + 25 mg of vitamin B2 + 18 mg of vitamin B5 + 10 mg of vitamin B6 + 33 μg of vitamin B12 and, optionally,
- excipients and/or additives of food or pharmaceutical grade.

Example 4B (MIX 2)

Composition in sachet form (e.g., 1 sachet once daily) containing:

- 200 mg or 400 mg of *Gastrodia elata* extract 10:1
- 250 mg of citicoline
- 1,000 mg of L-acetylcamitine and/or carnitine
- 50 μg of vitamin D2 and/or D3
- 25 mg of vitamin B1 + 25 mg of vitamin B2 + 18 mg of vitamin B5 + 10 mg of vitamin B6 + 33 μg of vitamin B12 and,

optionally,
- excipients and/or additives of food or pharmaceutical grade.

The activity of the associations or combinations, and of the compositions containing them, was evaluated on the basis of several experimental assays.

**Experimental protocol**

PHASE 1: ABSORPTION SCREENING

The intestinal cell line Caco-2 was used in a screening trial to determine which form of *Gastrodia elata* extract, and what concentration thereof, is most suitable for inclusion in the association of the invention, in terms of absorption at the intestinal level.
Different types of *Gastrodia elata* extracts were tested at a concentration of 300-200-100 mg, over a time period of between 1 hour and 6 hours (range of time in which absorption takes place within the small intestine).
The cells were seeded on a Transwell® insert and were maintained in a complete medium changed every other day for 21 days before the stimulations, in order to ensure the maturation and formation of intestinal microvilli. Maturation was complete when a TEER value $\geq 400\Omega/cm^2$ was reached.
The data obtained were compared with Lipoic acid 300 mg, 600 mg and 800 mg.
All types of *Gastrodia elata* extracts tested were able to have a better absorption compared to Lipoic acid ($p<0.05$) at all tested concentrations, with a similar trend over time and an absorption peak around 3-4 hours (data not shown).
The *Gastrodia elata* 10:1 and *Gastrodia elata* 50% polysaccharides (100 mg and 200 mg) extracts are the forms that show the best absorption profile, also in terms of the permeability analysis, and were thus selected and maintained in the subsequent tests.
Figure 8 shows the results of the above-described tests for the *Gastrodia elata* 10:1 and *Gastrodia elata* 50% polysaccharides (100 mg and 200 mg) extracts. Figure 8 also shows the results in terms of the permeability analysis.

PHASE 2: INTESTINAL ACTIVITY

A 3D *in vitro* model (approved by the FDA and EMA) was used to test the absorption and transport mechanisms of the novel association and the single components through the intestinal barrier. The following tests were conducted:

- Analysis of mitochondrial metabolism and cell well-being;
- Analysis of oxidative stress;
- Permeability and measurement of transepithelial electrical resistance (TEER);
- Prediction of absorption and plasma concentration;
- Tight junction activity.

Different experiments were thus carried out on the Caco-2 cell line to rule out any cytotoxic effect and confirm the absence of oxidative stress.
The following associations or combinations of the invention were tested:

MIX1 (Example 1A), in the two versions containing 100 mg or 200 mg of *Gastrodia elata* extract 50% polysaccharides.
MIX2 (Example 1B), in the two versions containing 100 mg or 200 mg of *Gastrodia elata* extract 10:1.

As shown in Figures 9A and 9B, both MIX1 and MIX2 are capable of increasing cell viability by reducing oxidative stress compared to lipoic acid ($p<0.05$).
Moreover, the associations or combinations of the invention revealed to be better than the composition present on the market containing lipoic acid, citicoline, L-acetylcarnitine and B-group vitamins (CF). Similar results were obtained with gastrodin (data not shown).
An analysis of TEER over time (1 hour-6 hours) and of TJ activity was performed to determine barrier integrity and functionality and rule out intestinal irritability. The results are shown in Figures 10A and 10B.
As shown, the analysis of TEER confirms the active role of MIX1 and MIX2, supporting the absorption thereof. Furthermore, analyses of Zo-1 (for adhesion); claudin (which maintains the structure) and occludin (which contributes to stabilization) confirm proper gut function.
Moreover, the associations or combinations of the invention revealed to be better than the composition present on the market (CF). Similar results were obtained with gastrodin (data not shown).
In order to obtain further information about intestinal absorption, an evaluation of intestinal absorption and

permeability was carried out to predict bioavailability.

As shown in Figures 11A and 11B, both MIX1 and MIX2 are capable of increasing permeability and plasma bioavailability ($p<0.05$).

Moreover, the associations or combinations of the invention revealed to be better than the composition present on the market (CF). Similar results were obtained with gastrodin (data not shown).

PHASE 3: ANALYSIS AT THE LEVEL OF THE PERIPHERAL NERVOUS SYSTEM (SNP)

The product metabolized by the intestinal cells was placed directly in contact with a 3D model for the study of the peripheral nerve (3D EngNT). This technique was developed by simulating peripheral nerve damage and demyelination with 200 ng/ml of GGF and analyzing:

- Cell survival and mitochondrial activity;
- Analysis of oxidative stress and ROS quantification;
- Analysis of the inflammatory processes involved in neuropathies;
- Analysis of nociceptive pathways for pain control;
- Evaluation of the main pathways involved in the processes of myelin sheath protection and neurite formation.

As shown in Figures 12A and 12B, both MIX1 and MIX2 are capable of increasing cell viability, increasing ERK activity and reducing the activation of annexin V ($p<0.05$).

Moreover, the associations or combinations of the invention revealed to be better than the composition present on the market (CF). Similar results were obtained with gastrodin (data not shown).

As shown in Figures 13A and 13B, both MIX1 and MIX2 are capable of reducing oxidative stress and the production of free radicals ($p<0.05$).

Moreover, the associations or combinations of the invention revealed to be better than the composition present on the market (CF). Similar results were obtained with gastrodin (data not shown).

An evaluation was also made of the activity of inducible nitric oxide synthase (iNos) as a mechanism induced by macrophages to produce free radicals, and a reduction in the activity supporting the previous antioxidant activity can be observed (Figures 14A and 14B, at the top). The membrane potential is also improved, demonstrating the activation of the cell survival system (Figures 14A and 14B, at the top).

In relation to these parameters as well, moreover, the associations or combinations of the invention revealed to be better than the composition present on the market (CF). Similar results were obtained with gastrodin (data not shown).

An analysis of the inflammatory processes following treatment with the formulations metabolized at the intestinal level was also conducted by mimicking neuropathic damage. The transcription factor NFkB and the activity of TNF$\alpha$, as the main markers triggering inflammatory mechanisms, were evaluated.

As shown in Figures 15A and 15B, both MIX1 and MIX2 are capable of reducing the activation of the main inflammatory markers involved in peripheral pain ($p<0.05$).

Moreover, the associations or combinations of the invention revealed to be better than the composition present on the market (CF). Similar results were obtained with gastrodin (data not shown).

The main cytokines involved in inflammatory processes, such as IL1b, IL-2 and IL-6, were also evaluated.

As shown in Figures 16A and 16B, both MIX1 and MIX2 are capable of reducing the production of the main inflammatory interleukins involved in peripheral pain ($p<0.05$).

Once again, the compositions of the invention revealed to be better than the commercial composition comprising lipoic acid (CF).

An analysis of pain mechanisms following treatment with the formulations metabolized at the intestinal level was also conducted by mimicking neuropathic damage.

The activity of Nav1.7 and Nav1.8 (sodium channels), which are highly expressed particularly in pain pathophysiology, was analyzed.

As shown in Figures 17A and 17B, both MIX1 and MIX2 are capable of reducing pain by reducing the activity of the NaV 1.7 and NaV 1.8 channels, which serve to modulate pain ($p<0.05$).

Once again, the compositions of the invention revealed to be better than the commercial composition comprising lipoic acid (CF).

A chemical signal that naturally inhibits pain is GABA. When there is a nerve injury, the neurons sensitive to this neurotransmitter no longer respond correctly and pain continues to be transmitted. GABA activity was thus also evaluated.

As shown in Figure 18, both MIX1 and MIX2 are capable of reducing peripheral pain by increasing GABA activity ($p<0.05$).

Once again, the compositions of the invention revealed to be better than the commercial composition comprising lipoic acid (CF).

Finally, in order to investigate the intramolecular mechanisms involved following treatment with the compositions metabolized at the intestinal level by mimicking neuropathic damage, the role of Schwann cells was studied by analyzing the modulation of neuropathic pain in vitro.

The regulation mechanisms at the basis of the observed effects were analyzed in Schwann cells by mimicking nerve injury through treatment with 200 ng/mL of GGF.

As shown in Figures 19A and 19B, both MIX1 and MIX2 are capable of restoring the activity of p75 and MPZ, as markers involved in the control of the myelin sheath ($p<0.05$).

ERb receptors are activated by NRG1 to regulate the growth, migration, differentiation, and de-differentiation of Schwann cells. As shown in Figures 20A and 20B, both MIX1 and MIX2 are capable of restoring Erb and NRG1 activity ($p<0.05$). Therefore, the tested compositions MIX 1 and MIX 2 have demonstrated to be capable of reversing the damage by modulating the markers involved in maintaining the normal activity of the myelin sheath, improving the activity of p75 ($p < 0.05$) and MPZ ($p < 0.05$) and, at the same time, increasing NRG1 and ERb activity, supporting the hypothesis of an improvement in both these markers such as to repair nerve damage and restore the myelination process.

Moreover, the associations or combinations of the invention revealed to be better than the composition present on the market (CF). Similar results were obtained with gastrodin (data not shown).

As shown in Figure 21, both MIX1 and MIX2 are also capable of increasing the production of NGF, the pleiotropic factor involved in growth, differentiation and repair of neuron damage.

Once again, the associations or combinations of the invention revealed to be better than the composition present on the market (CF, which is a composition of a supplement product on the market containing alpha-lipoic acid, L-acetylcamitine, citicoline and B-group vitamins). Similar results were obtained with gastrodin (data not shown).

**Conclusions**

In conclusion, based on the data obtained it is possible to highlight that:

- the novel associations/compositions are safe, maintaining the integrity of the intestinal epithelium and maintaining intact the permeability related to the opening/closing of the tight junctions, above all increasing their absorption;
- the novel associations/compositions inhibit the production of free radicals, reduce inflammation and maintain cell integrity,
- the novel associations/compositions correctly modulate the biological activity of Schwann cells;
- the novel associations/compositions are capable of slowing malfunction and repairing nerve damage at the peripheral level, suggesting better effectiveness with positive effects on the whole regulation system;
- the novel associations/compositions inhibit the main molecular pathways involved in demyelination, tending towards a correct homeostasis of neuronal cells;
- thus demonstrating their safety and biological effectiveness.

**Claims**

1. An association comprising an extract of *Gastrodia elata,* citicoline, L-acetylcamitine, at least one vitamin of group D.

2. The association according to claim 1, wherein said association consists of an extract of *Gastrodia elata,* citicoline, L-acetylcamitine, at least one vitamin of group D.

3. The association according to claim 1 or 2, wherein said at least one vitamin of group D is selected from the group consisting of: vitamin D1, a compound consisting of 1:1 parts of ergocalciferol and lumisterol, vitamin D2 ergocalciferol, vitamin D3 cholecalciferol, vitamin D4 dihydroergocalciferol, vitamin D5 calciferol, and/or mixtures thereof.

4. The association according to claim 3, wherein said at least one vitamin of group D is selected from the group consisting of vitamin D2 and vitamin D3.

5. The association according to claim 4, wherein said at least one vitamin of group D is vitamin D3..

6. A composition comprising the association according to any one of claims 1-5 and at least a pharmaceutical acceptable excipient and/or vehicle.

7. The composition according to claim 6, formulated for oral use.

8. The composition according to claim 7, formulated as sachet, stick, orodispersible stick, tablet, gel or capsule.

9. The association according to any one of claims 1-5 or the composition according to any one of claims 6-8, for use as medicament.

10. The association according to any one of claims 1-5 or the composition according to any one of claims 6-8, for use in in preventing or treating a damage or a pathology to the central nervous.

11. The association according to any one of claims 1-5 or the composition according to any one of claims 6-8, for use in in preventing or treating a damage or a pathology to the peripheral nervous system.

12. The association or the composition for use according to claim 11, wherein the damage or pathology to the peripheral nervous system is a peripheral neuropathy.

13. The association or the composition for use according to claim 11 or 12, wherein the damage or pathology to the peripheral nervous system is neuropathic pain.

14. A nutraceutical or supplement composition comprising the association according to any one of claims 1-7.

15. A non-medical use of the nutraceutical or supplement composition according to claim 14, for maintaining the well-being and health of the central and of the peripheral nervous system.

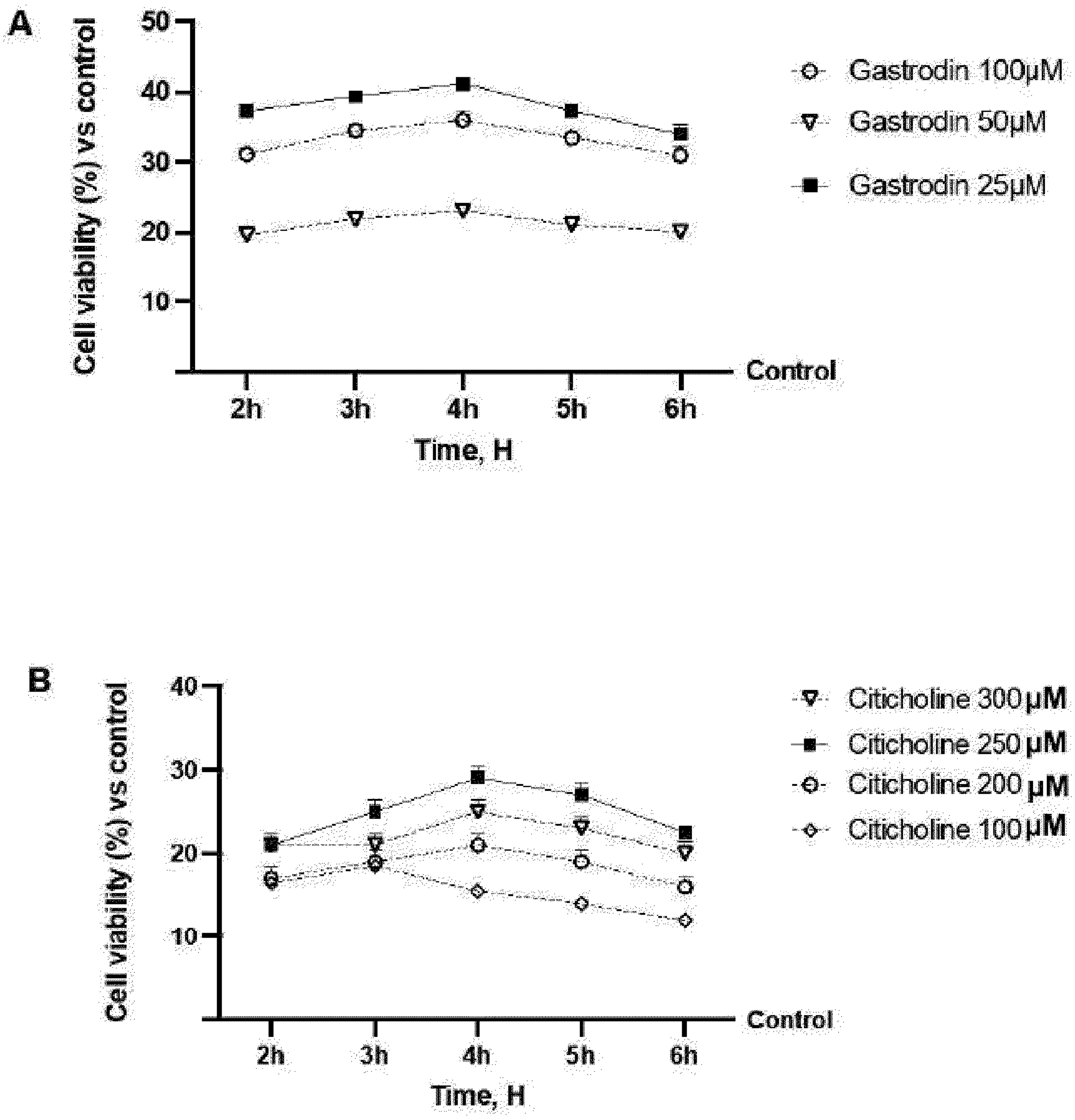


FIG. 1 (continued)

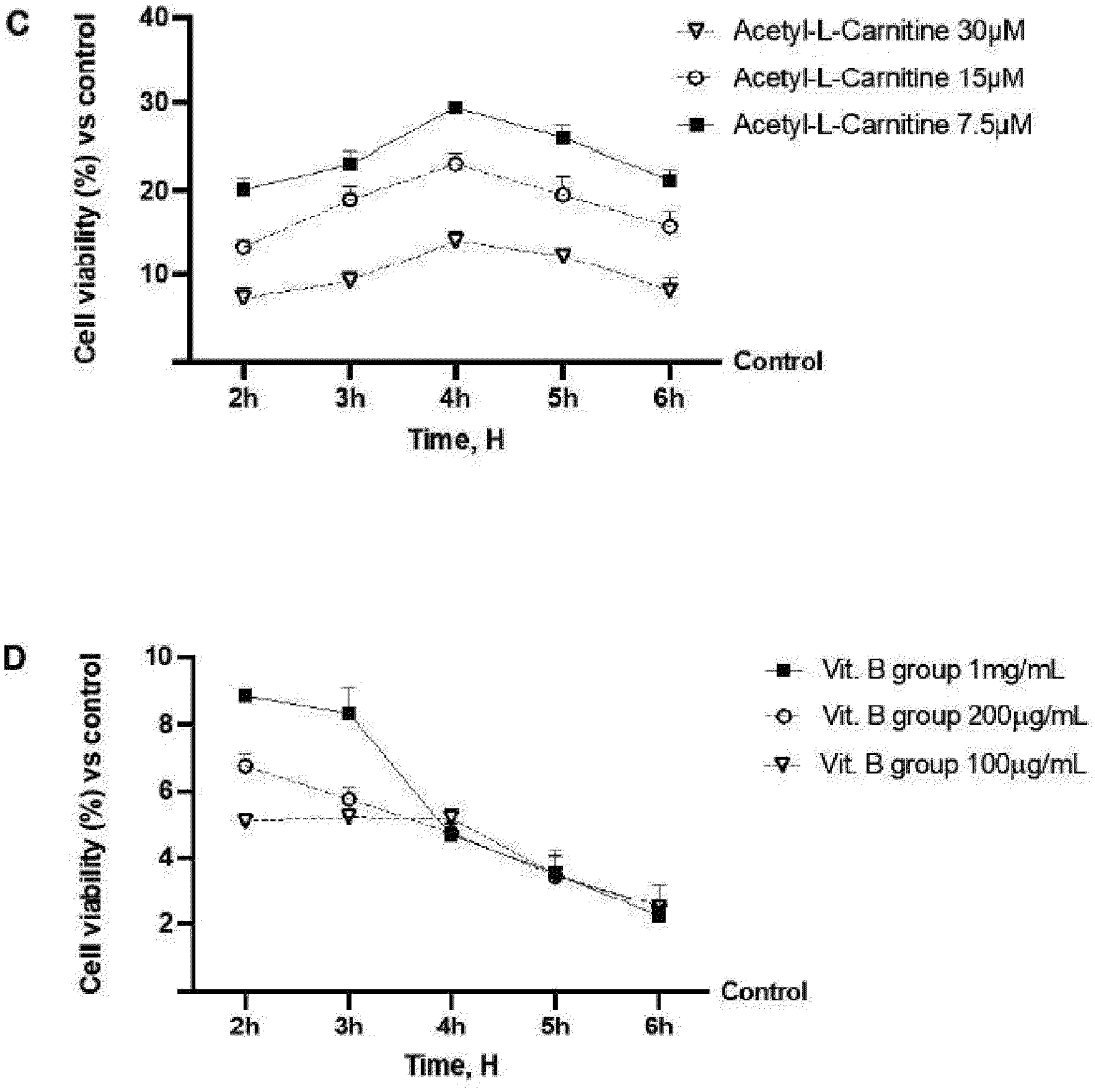
C
Cell viability (%) vs control
40
30
20
10
Acetyl-L-Carnitine 30μM
Acetyl-L-Carnitine 15μM
Acetyl-L-Carnitine 7.5μM
Control
2h
3h
4h
5h
6h
Time, H
D
Cell viability (%) vs control
10
8
6
4
2
Vit. B group 1mg/mL
Vit. B group 200μg/mL
Vit. B group 100μg/mL
Control
2h
3h
4h
5h
6h
Time, H

FIG. 1

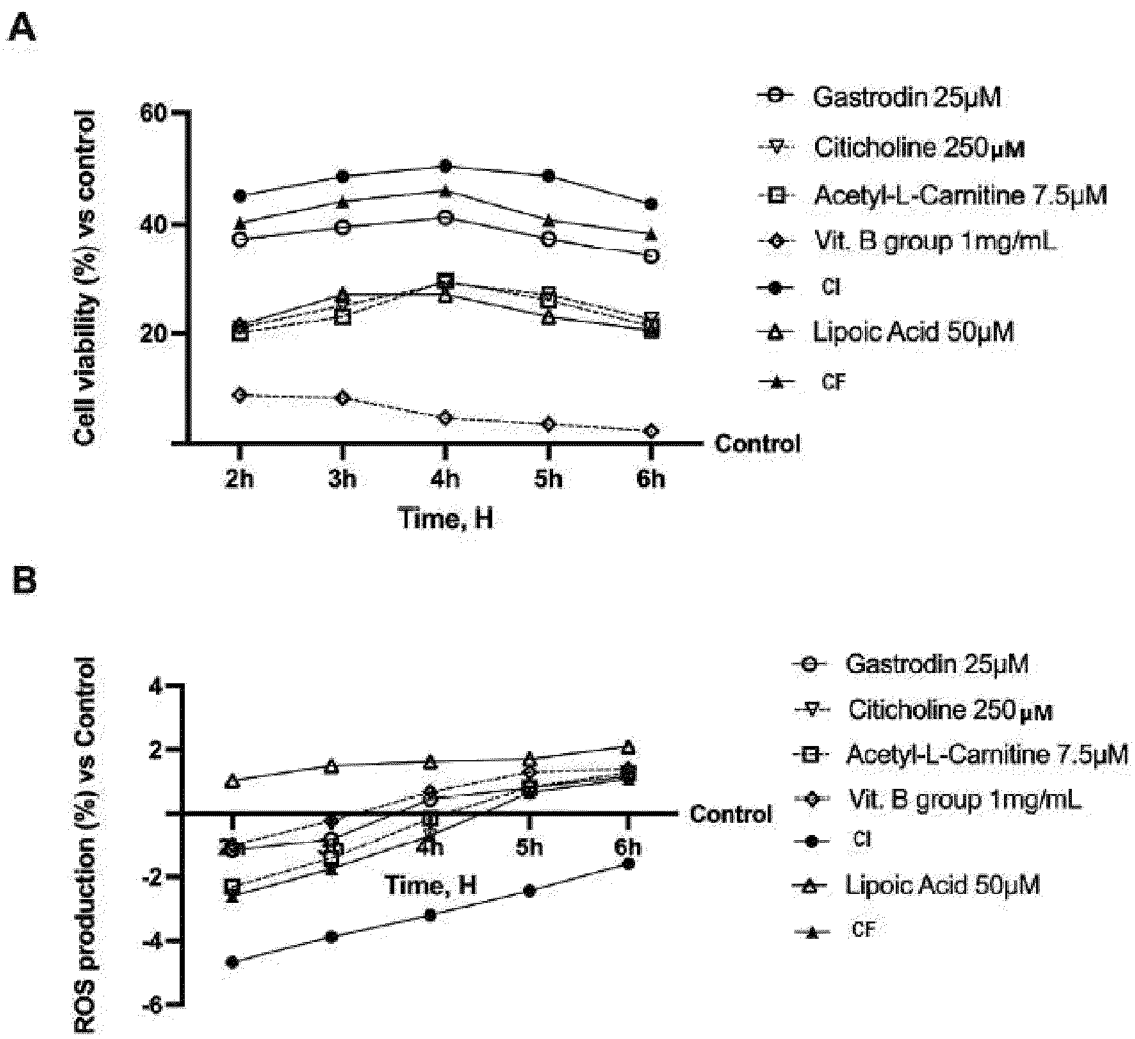
A
Cell viability (%) vs control
60
40
20
2h
3h
4h
5h
6h
Time, H
Control
Gastrodin 25μM
Citicholine 250μM
Acetyl-L-Carnitine 7.5μM
Vit. B group 1mg/mL
CI
Lipoic Acid 50μM
CF
B
ROS production (%) vs Control
4
2
-2
-4
-6
2h
3h
4h
5h
6h
Time, H
Control
Gastrodin 25μM
Citicholine 250μM
Acetyl-L-Carnitine 7.5μM
Vit. B group 1mg/mL
CI
Lipoic Acid 50μM
CF

FIG. 2

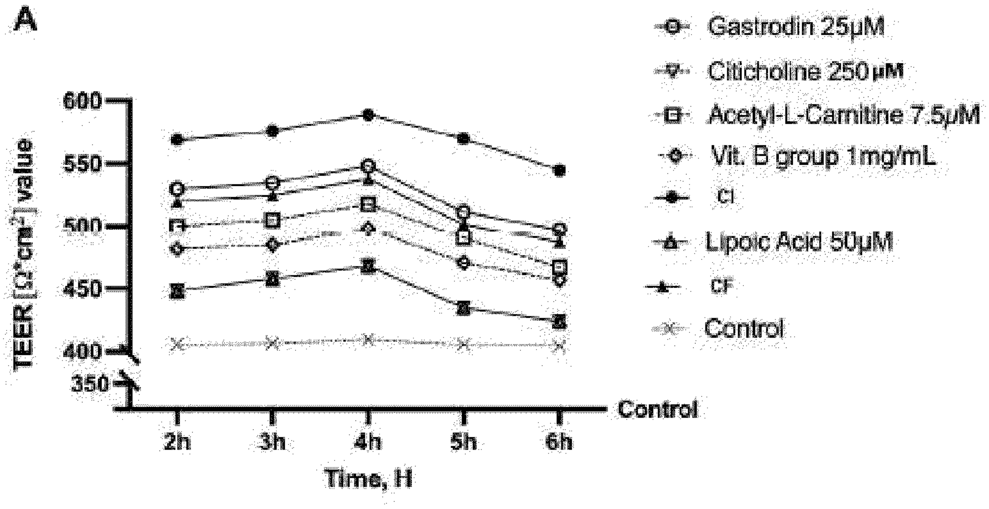


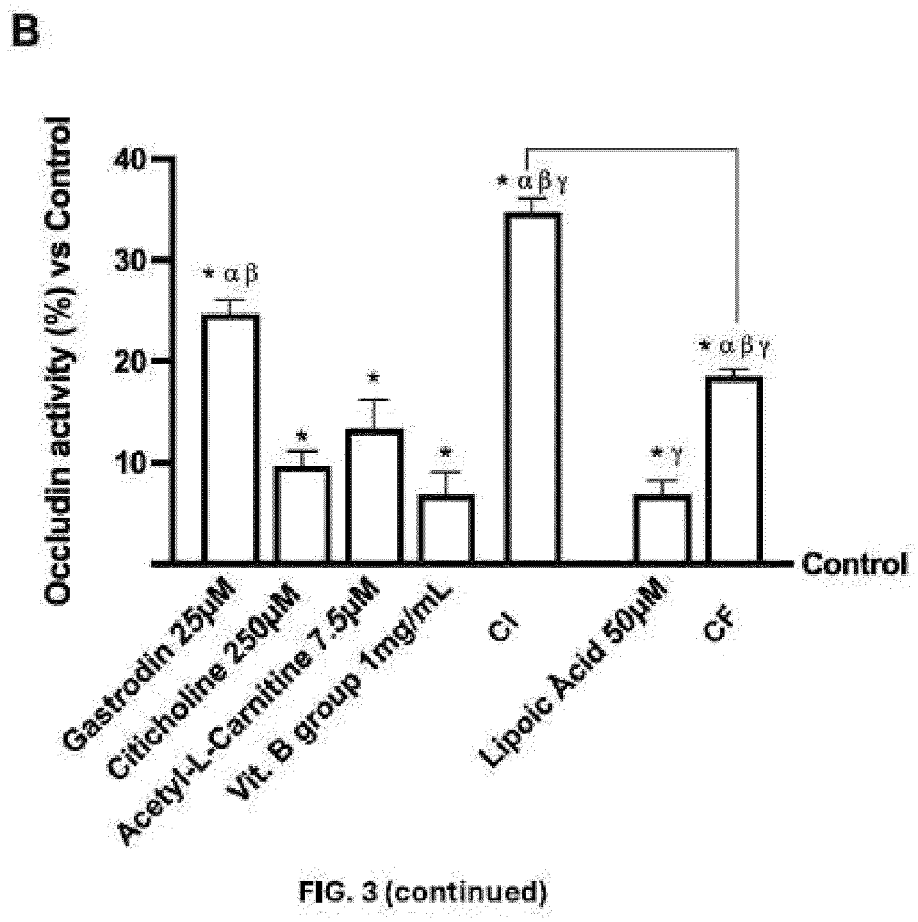


FIG. 3 (continued)

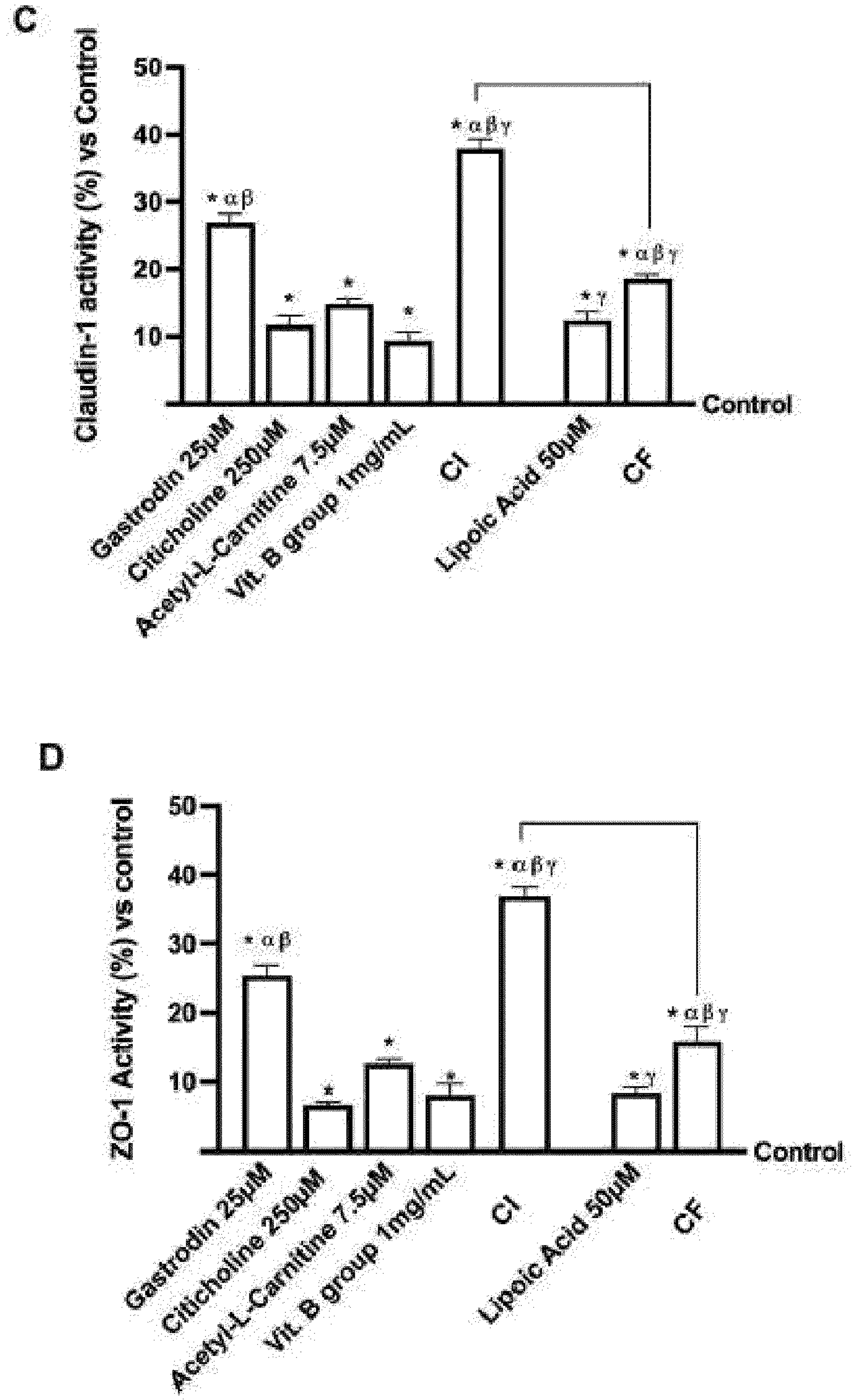
C
Claudin-1 activity (%) vs Control
50
40
30
20
10
Control
* α β
*
*
*
* α β γ
* γ
* α β γ
Gastrodin 25μM
Citicholine 250μM
Acetyl-L-Carnitine 7.5μM
Vit. B group 1mg/mL
CI
Lipoic Acid 50μM
CF
D
ZO-1 Activity (%) vs control
50
40
30
20
10
Control
* α β
*
*
*
* α β γ
* γ
* α β γ
Gastrodin 25μM
Citicholine 250μM
Acetyl-L-Carnitine 7.5μM
Vit. B group 1mg/mL
CI
Lipoic Acid 50μM
CF

FIG. 3

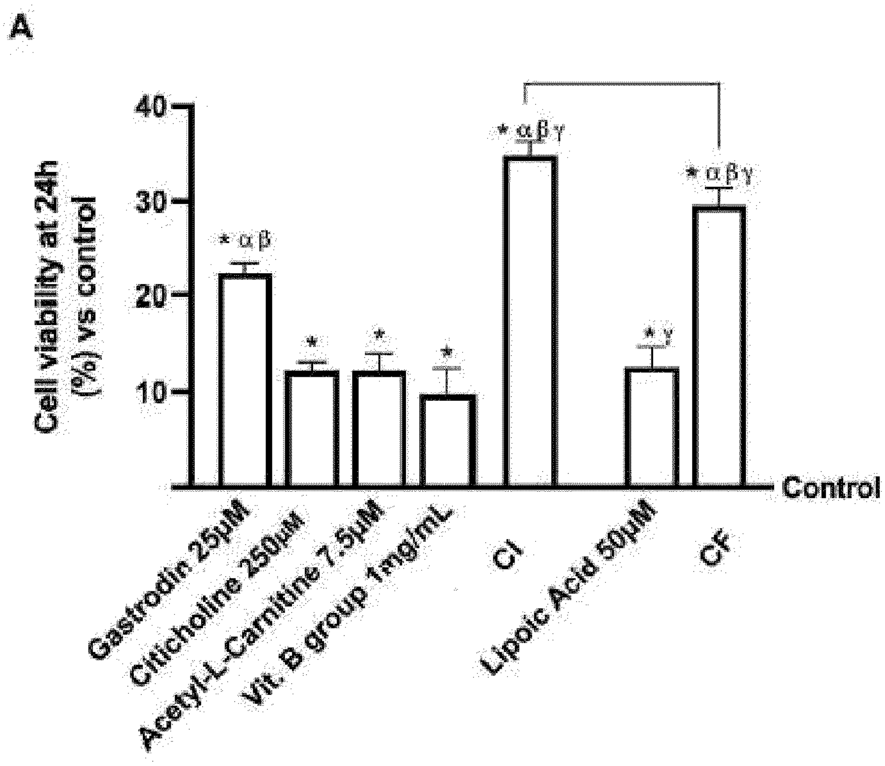


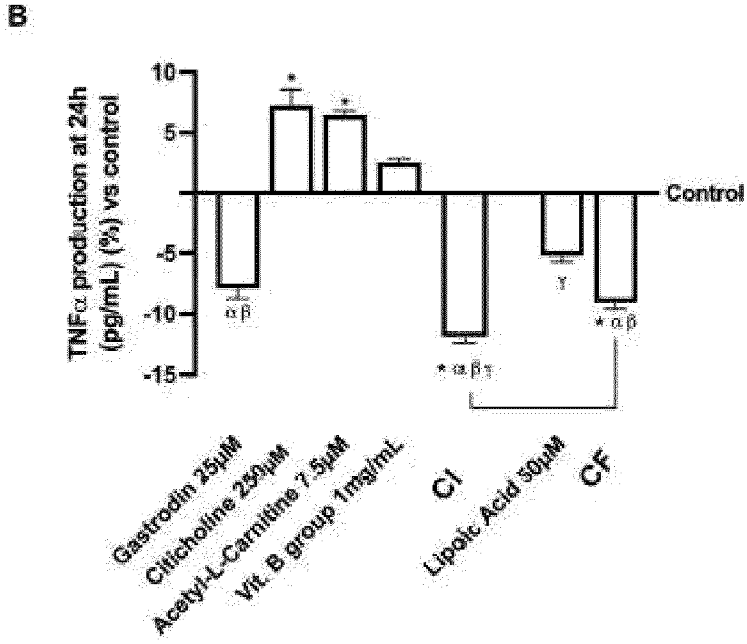


FIG. 4 (continued)

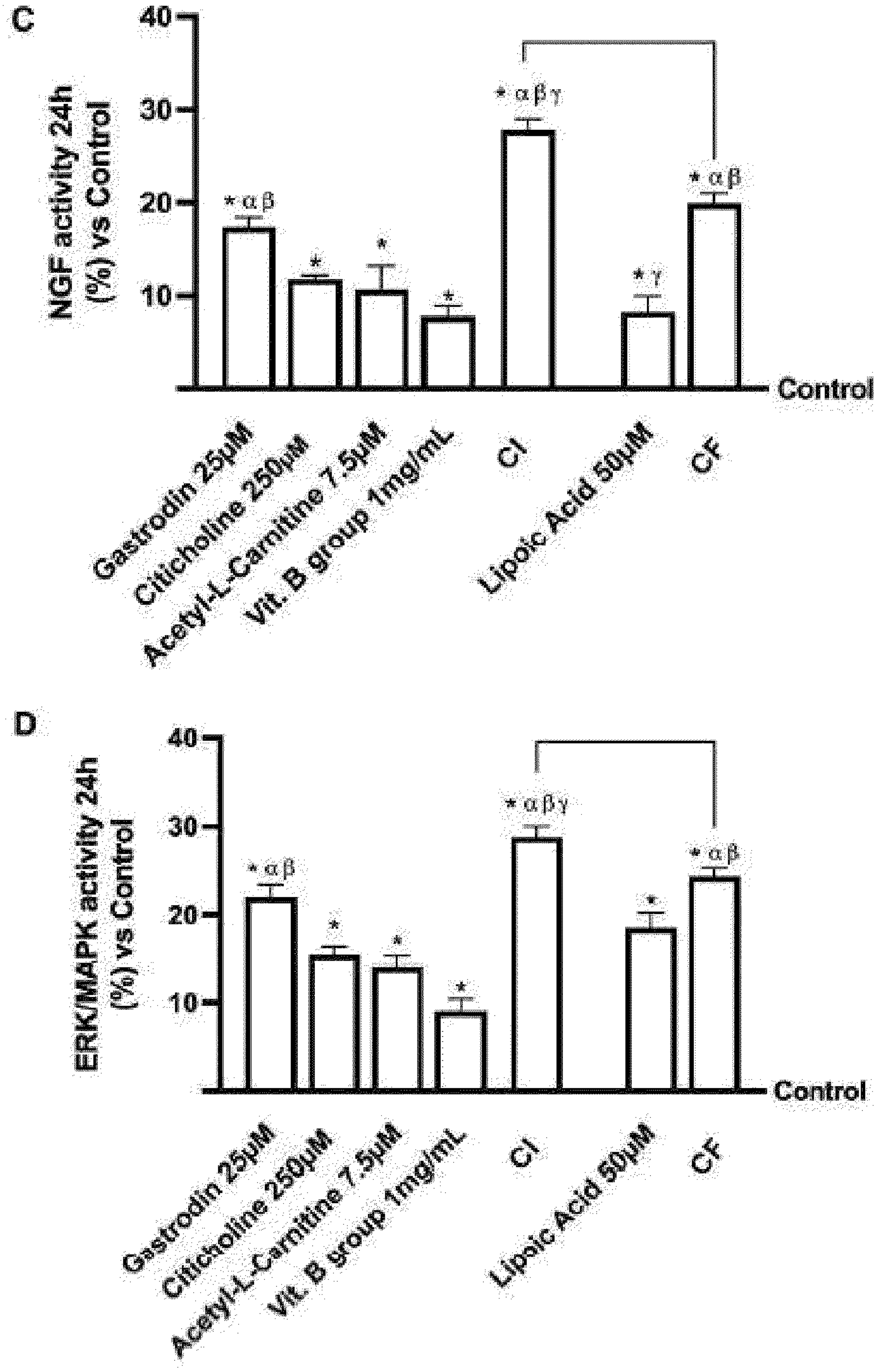
C
NGF activity 24h (%) vs Control
40
30
20
10
Control
* α β
*
*
*
* α β γ
* γ
* α β
Gastrodin 25µM
Citicholine 250µM
Acetyl-L-Carnitine 7.5µM
Vit. B group 1mg/mL
CI
Lipoic Acid 50µM
CF
D
ERK/MAPK activity 24h (%) vs Control
40
30
20
10
Control
* α β
*
*
*
* α β γ
*
* α β
Gastrodin 25µM
Citicholine 250µM
Acetyl-L-Carnitine 7.5µM
Vit. B group 1mg/mL
CI
Lipoic Acid 50µM
CF

FIG. 4

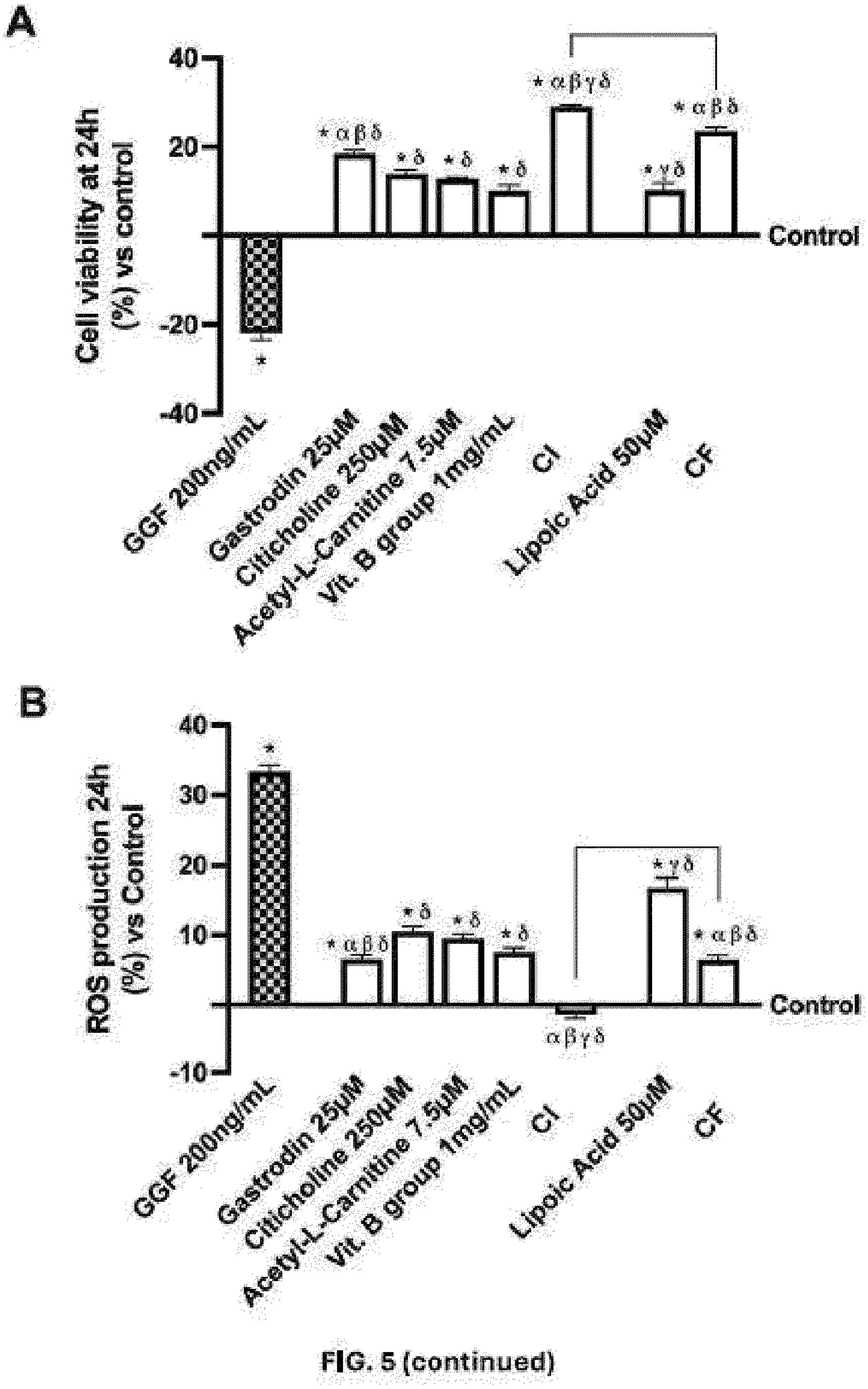


FIG. 5 (continued)

C

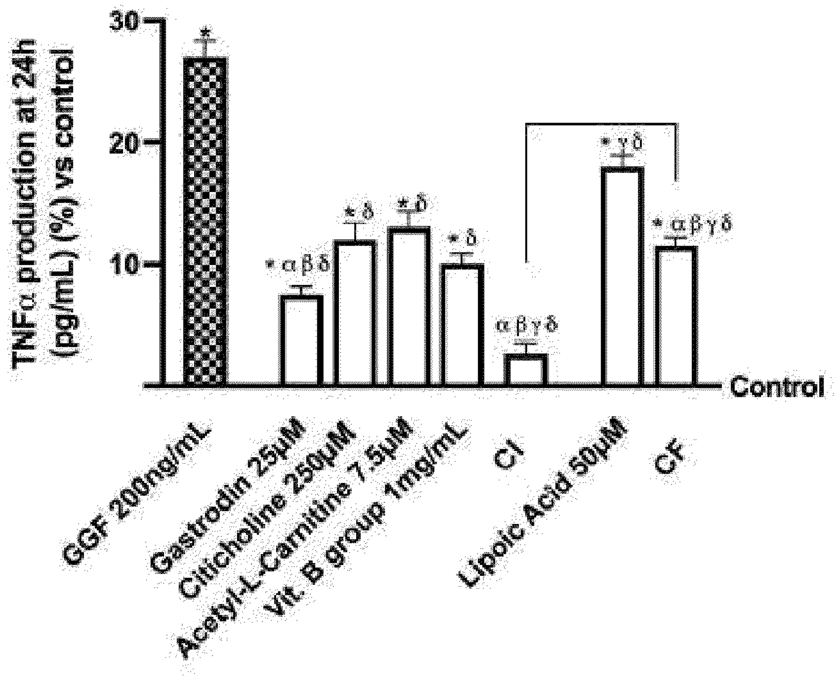
30
20
10
TNFα production at 24h (pg/mL) (%) vs control
*
* α β δ
* δ
* δ
* δ
α β γ δ
* γ δ
* α β γ δ
Control
GGF 200ng/mL
Gastrodin 25μM
Citicholine 250μM
Acetyl-L-Carnitine 7.5μM
Vit. B group 1mg/mL
CI
Lipoic Acid 50μM
CF

D

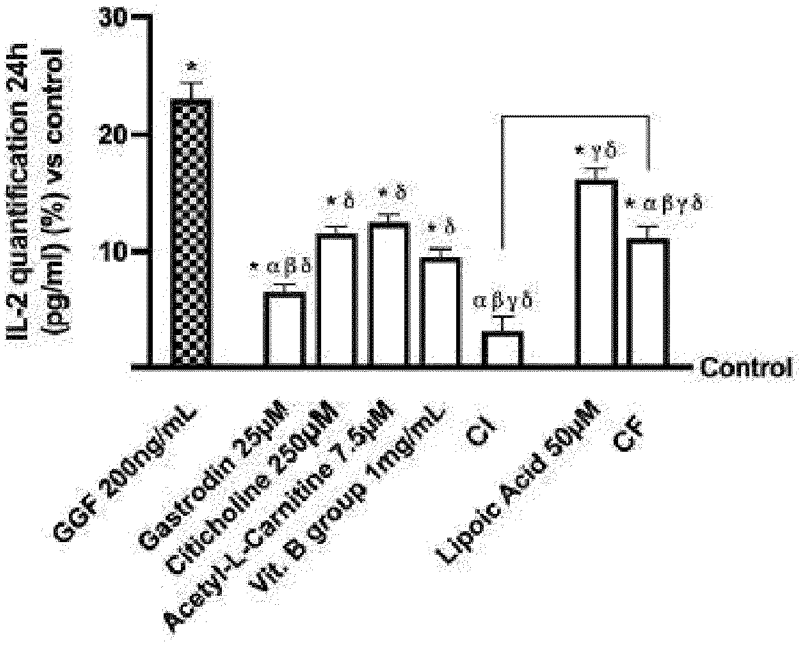
30
20
10
IL-2 quantification 24h (pg/ml) (%) vs control
*
* α β δ
* δ
* δ
* δ
α β γ δ
* γ δ
* α β γ δ
Control
GGF 200ng/mL
Gastrodin 25μM
Citicholine 250μM
Acetyl-L-Carnitine 7.5μM
Vit. B group 1mg/mL
CI
Lipoic Acid 50μM
CF

FIG. 5

A

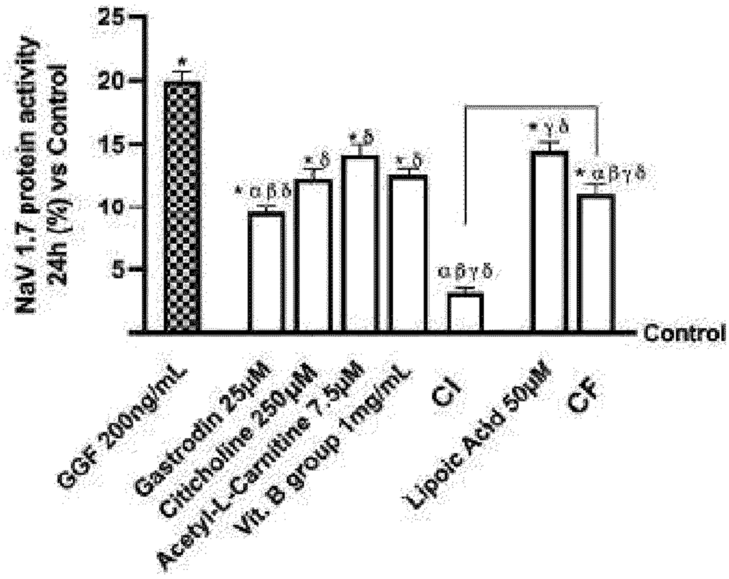


B

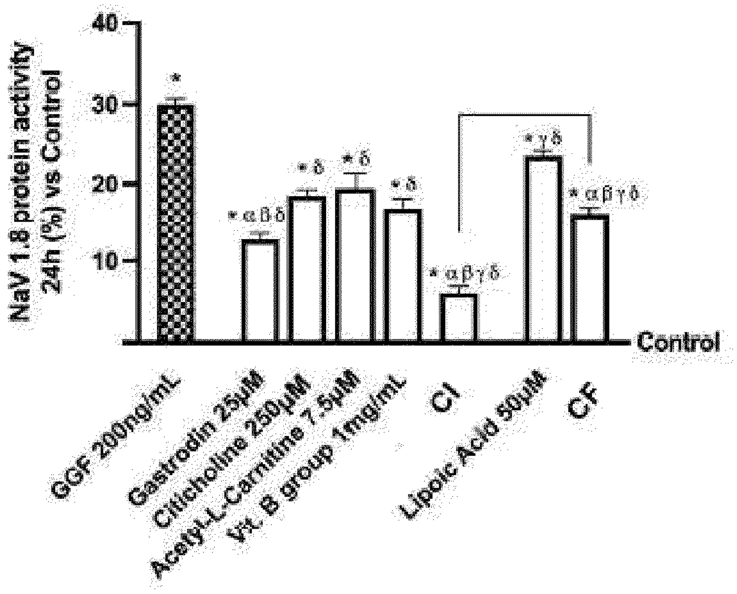


FIG. 6 (continued)

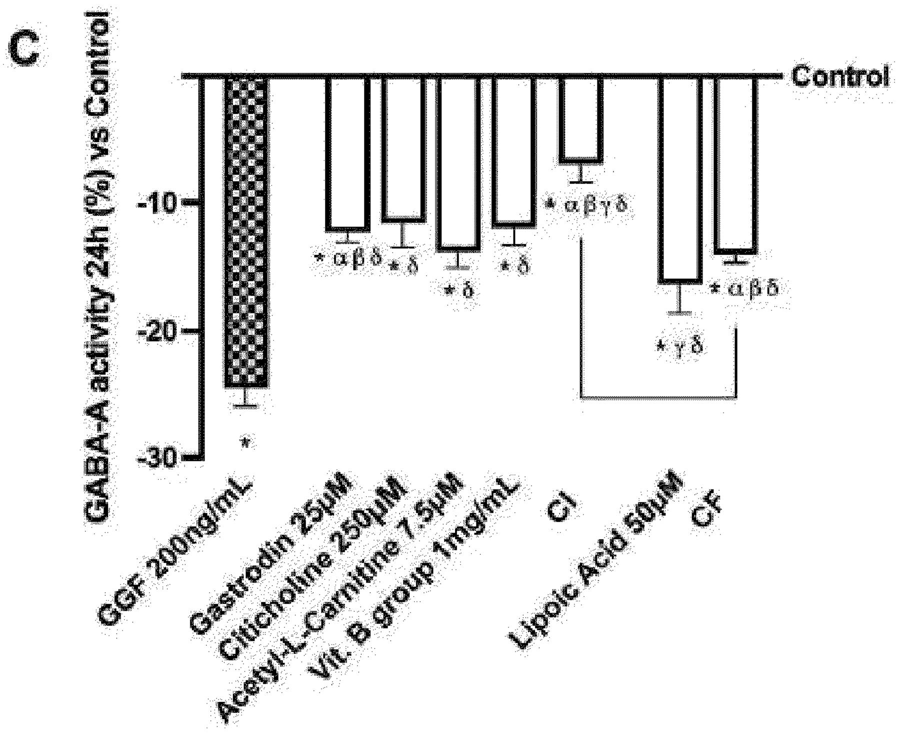
C
GABA-A activity 24h (%) vs Control
Control
-10
-20
-30
*
* α β δ
* δ
* δ
* δ
* α β γ δ
* γ δ
* α β δ
GGF 200ng/mL
Gastrodin 25μM
Citicholine 250μM
Acetyl-L-Carnitine 7.5μM
Vit. B group 1mg/mL
CI
Lipoic Acid 50μM
CF

FIG. 6

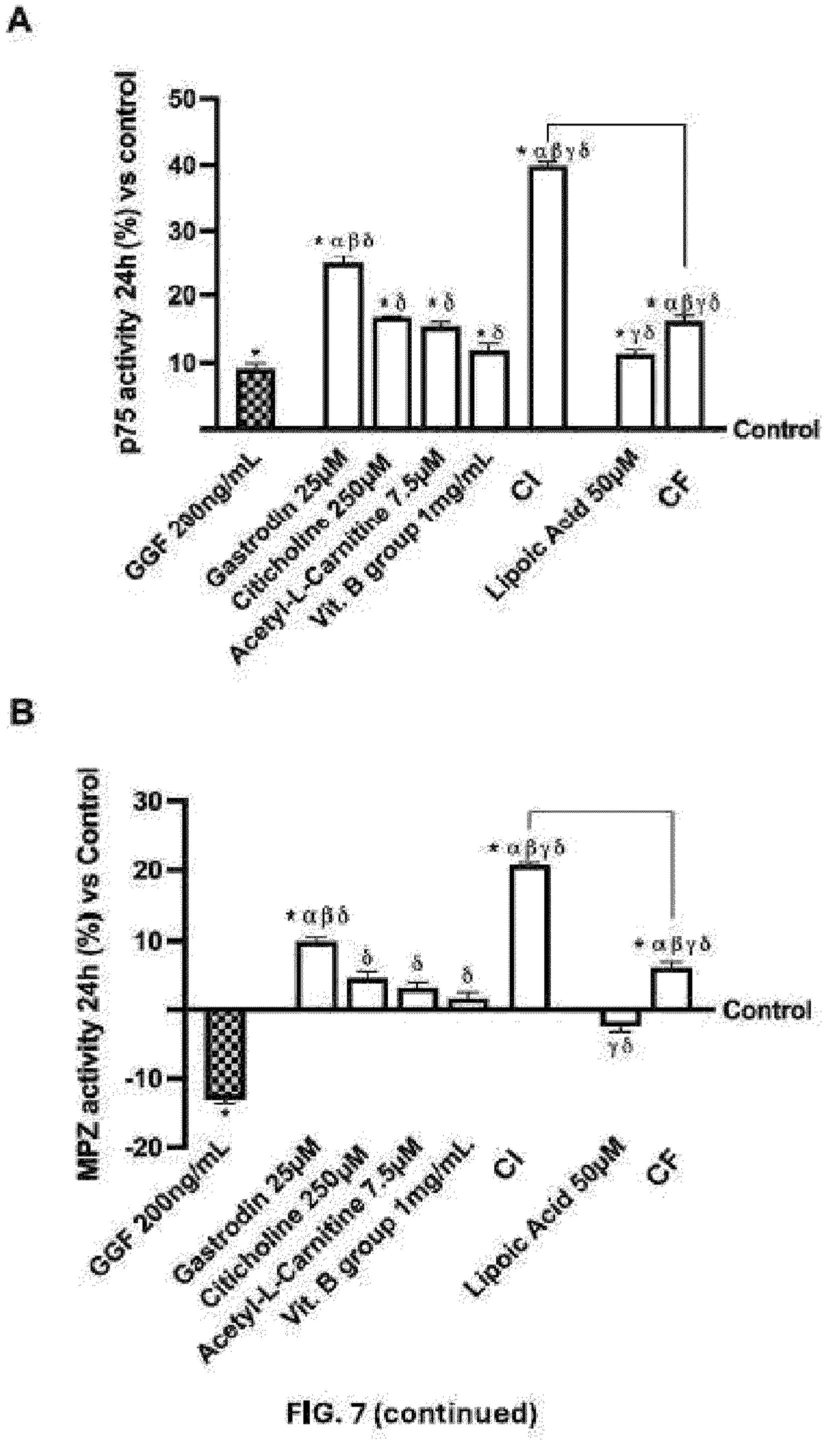


FIG. 7 (continued)

C

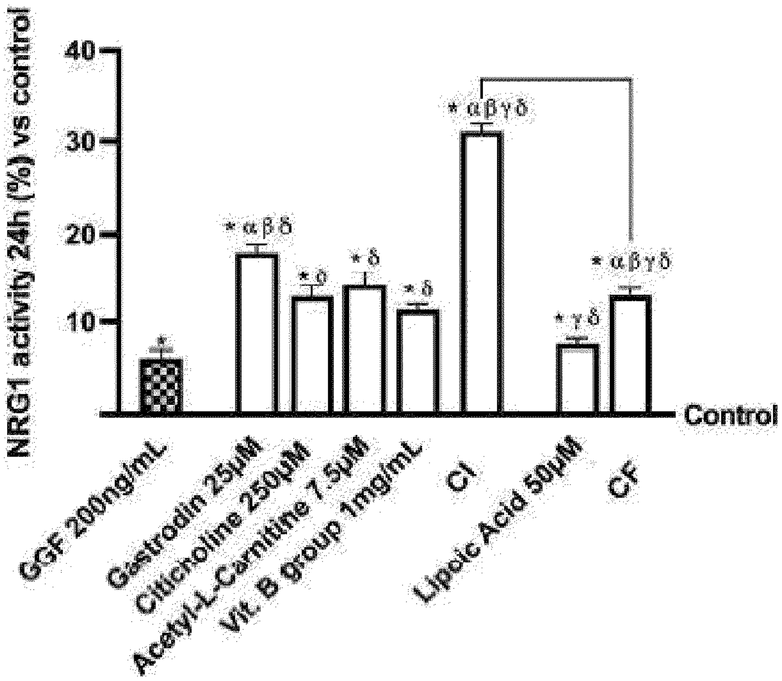


D

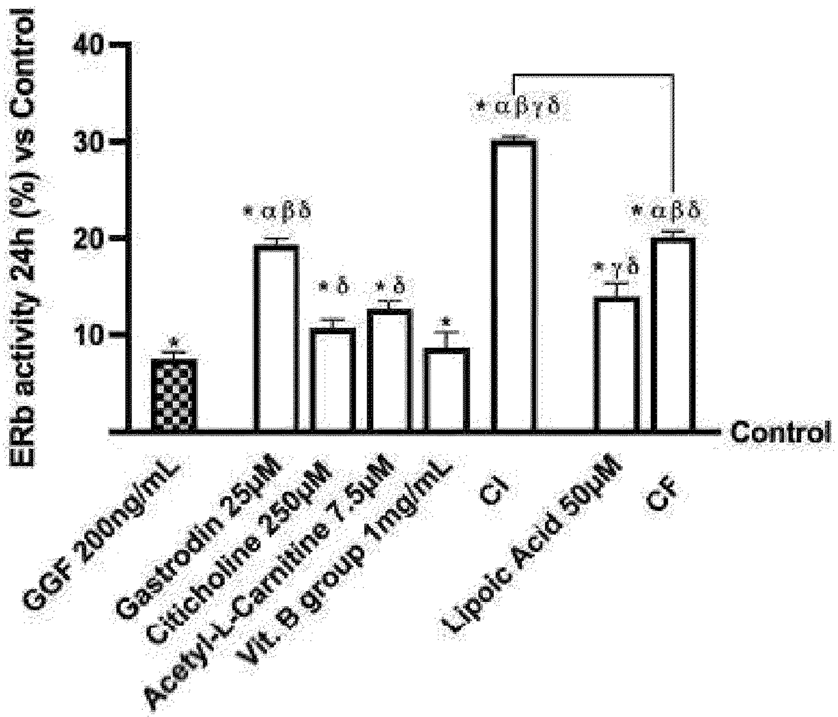


FIG. 7 (continued)

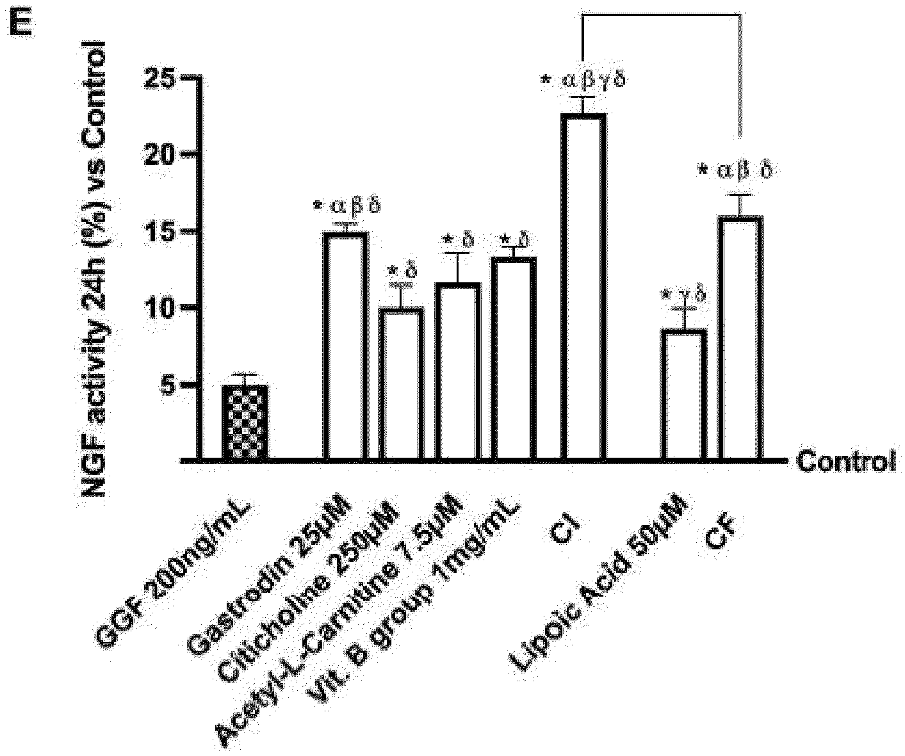
E
NGF activity 24h (%) vs Control
25
20
15
10
5
Control
* α β δ
* δ
* δ
* δ
* α β γ δ
* γ δ
* α β δ
GGF 200ng/mL
Gastrodin 25μM
Citicholine 250μM
Acetyl-L-Carnitine 7.5μM
Vit. B group 1mg/mL
CI
Lipoic Acid 50μM
CF

FIG. 7

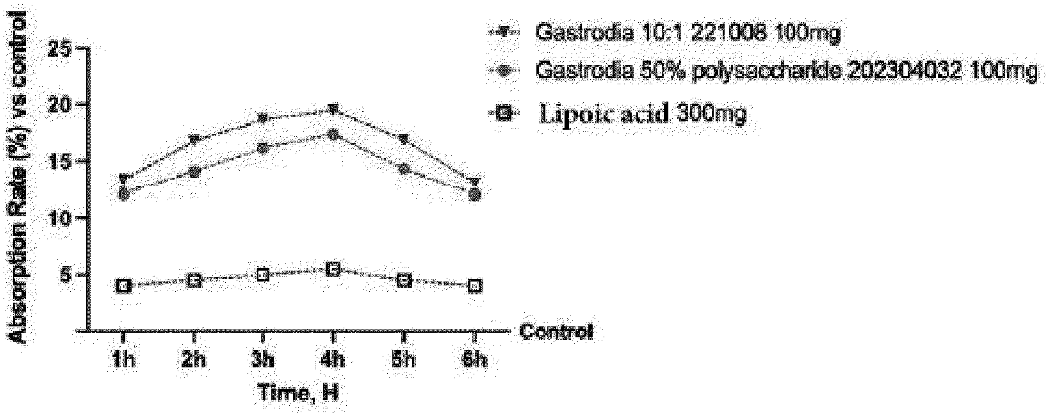


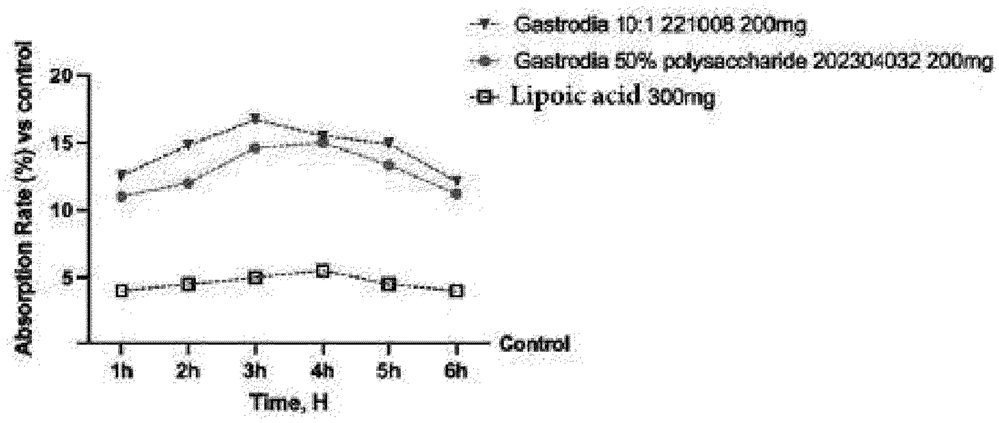


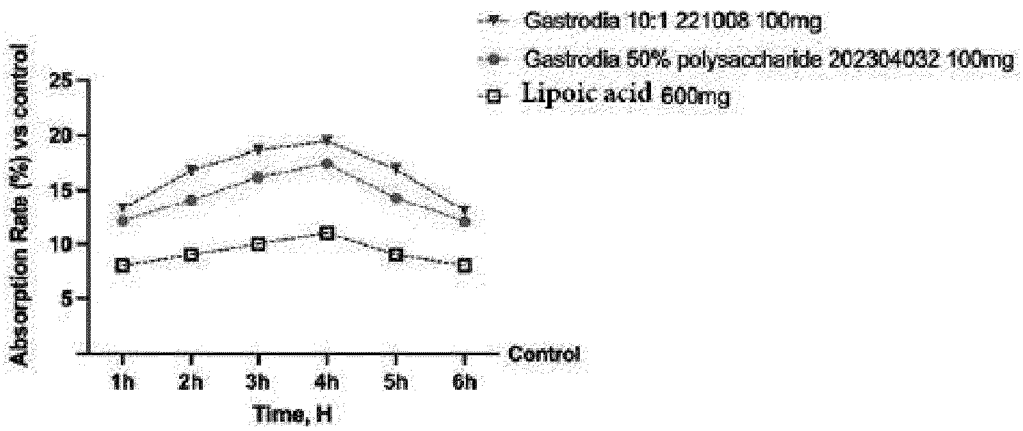


FIG. 8 (continued)

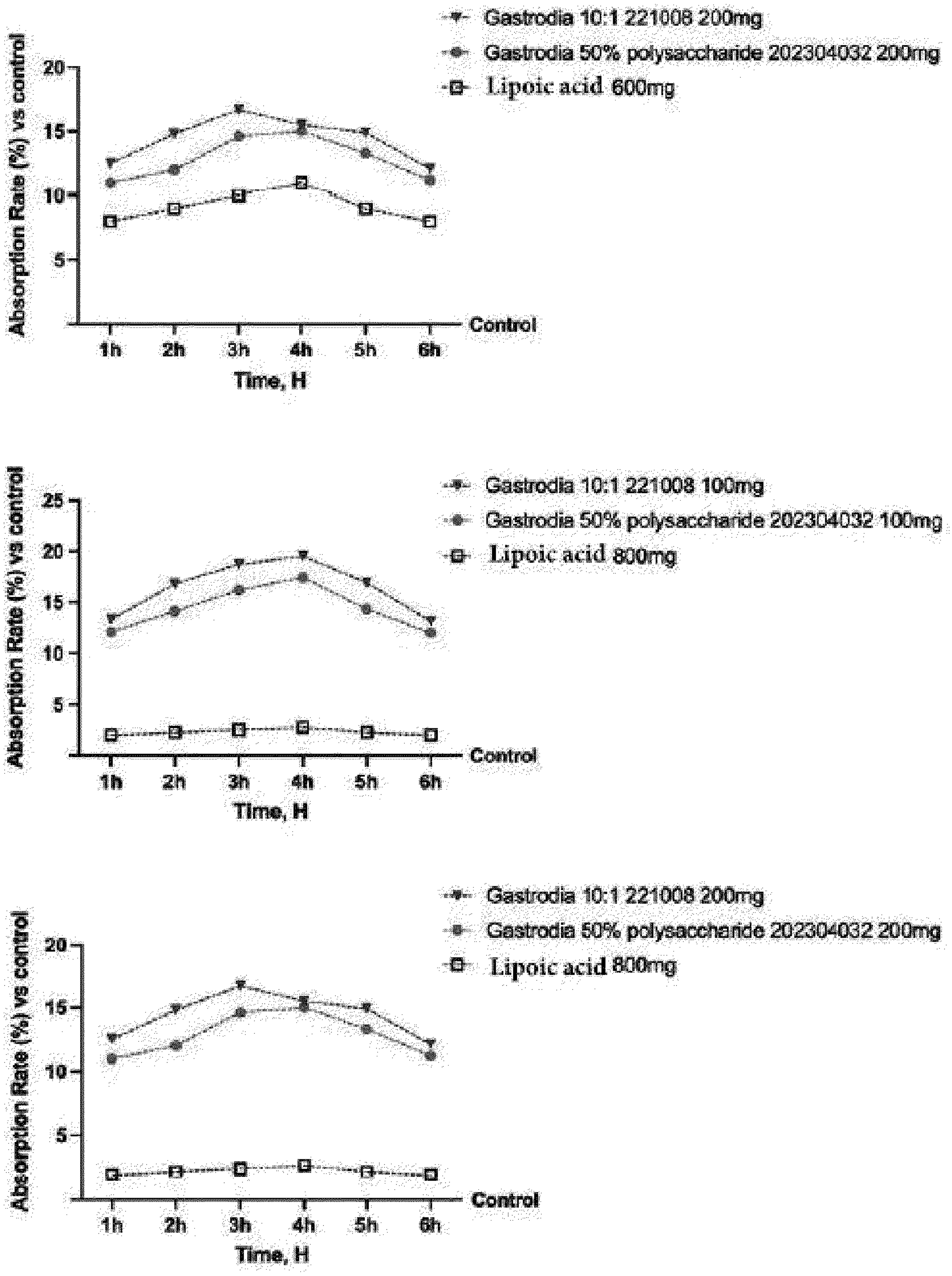

Gastrodia 10:1 221008 200mg
Gastrodia 50% polysaccharide 202304032 200mg
Lipoic acid 600mg
Absorption Rate (%) vs control
20
15
10
5
Control
1h
2h
3h
4h
5h
6h
Time, H
Gastrodia 10:1 221008 100mg
Gastrodia 50% polysaccharide 202304032 100mg
Lipoic acid 800mg
Absorption Rate (%) vs control
25
20
15
10
5
Control
1h
2h
3h
4h
5h
6h
Time, H
Gastrodia 10:1 221008 200mg
Gastrodia 50% polysaccharide 202304032 200mg
Lipoic acid 800mg
Absorption Rate (%) vs control
20
15
10
5
Control
1h
2h
3h
4h
5h
6h
Time, H


FIG. 8

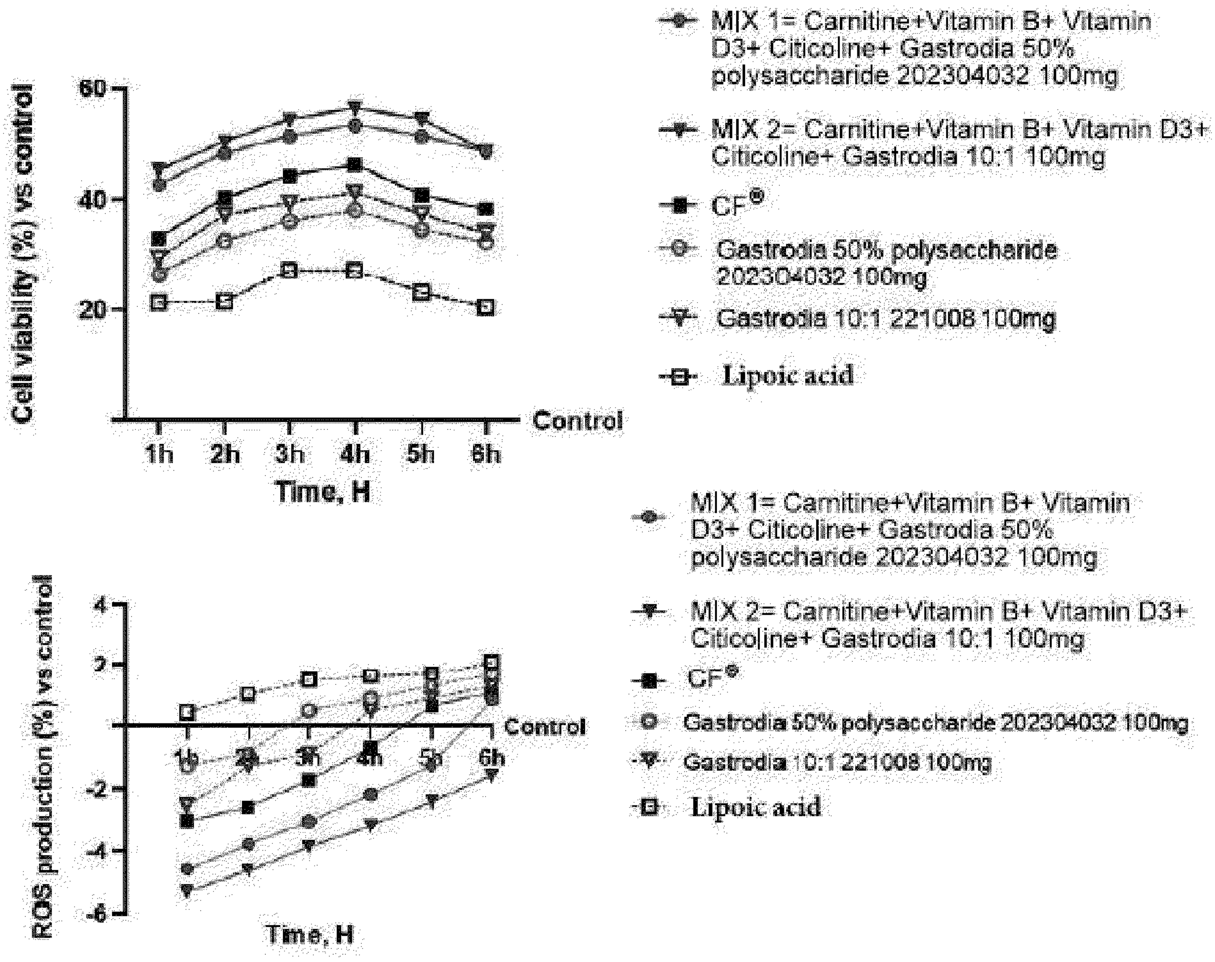
Cell viability (%) vs control
60
40
20
Control
1h
2h
3h
4h
5h
6h
Time, H
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 100mg
CF®
Gastrodia 50% polysaccharide 202304032 100mg
Gastrodia 10:1 221008 100mg
Lipoic acid
ROS production (%) vs control
4
2
-2
-4
-6
Control
1h
2h
3h
4h
5h
6h
Time, H
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 100mg
CF®
Gastrodia 50% polysaccharide 202304032 100mg
Gastrodia 10:1 221008 100mg
Lipoic acid

FIG. 9A

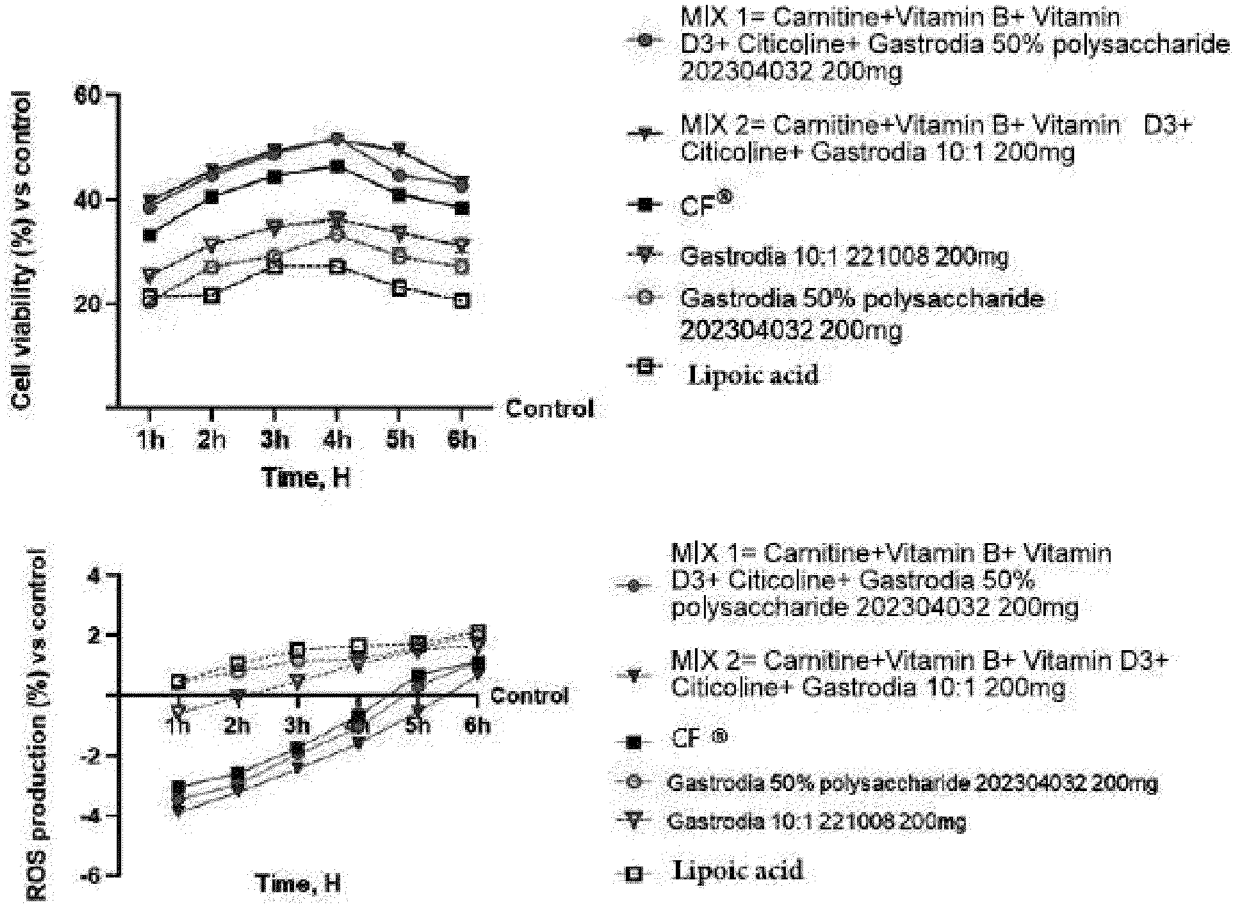
Cell viability (%) vs control
60
40
20
Control
1h 2h 3h 4h 5h 6h
Time, H
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 200mg
CF®
Gastrodia 10:1 221008 200mg
Gastrodia 50% polysaccharide 202304032 200mg
Lipoic acid
ROS production (%) vs control
4
2
-2
-4
-6
Control
1h 2h 3h 4h 5h 6h
Time, H
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 200mg
CF ®
Gastrodia 50% polysaccharide 202304032 200mg
Gastrodia 10:1 221008 200mg
Lipoic acid

FIG. 9B

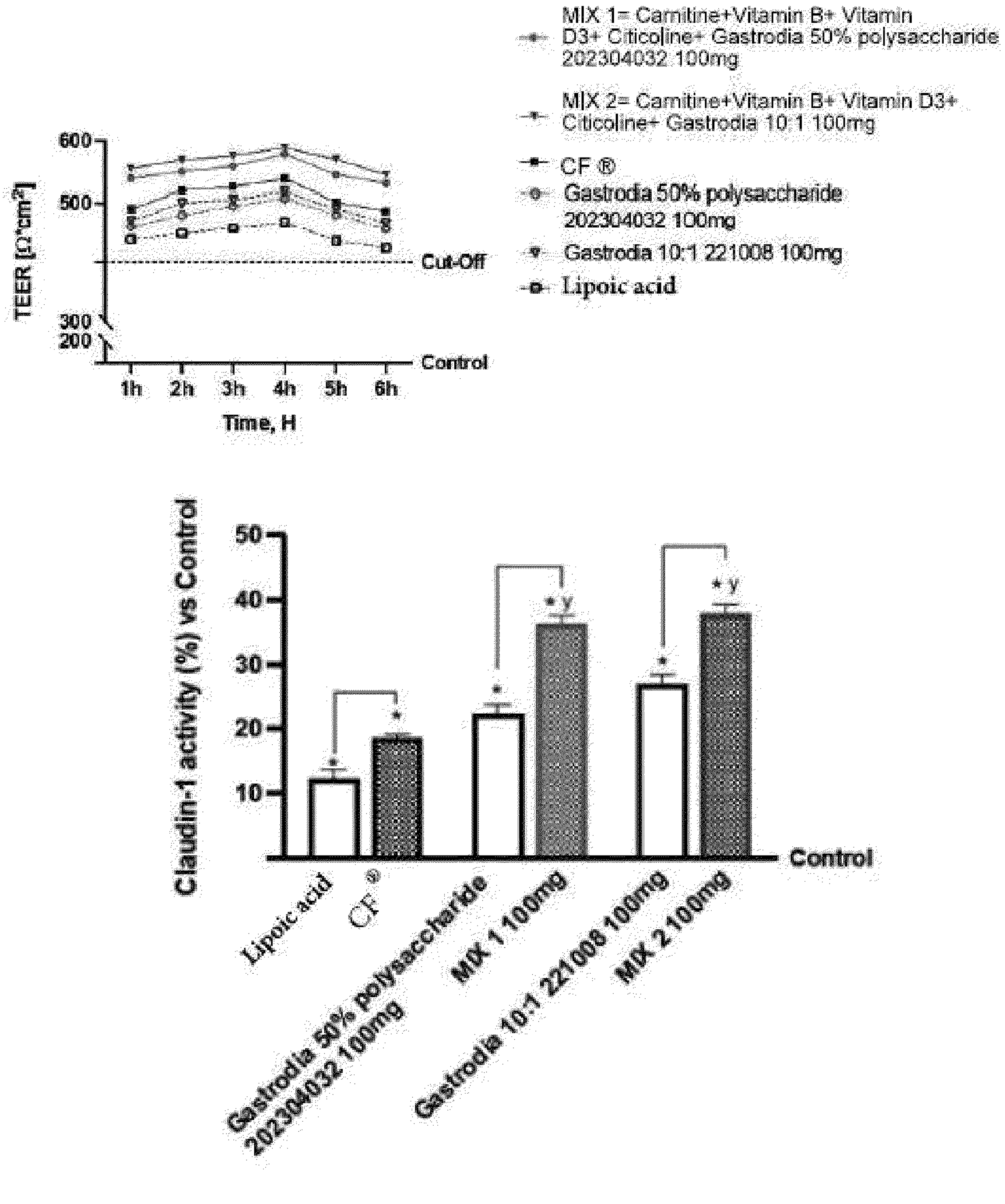


FIG. 10 A (continued)

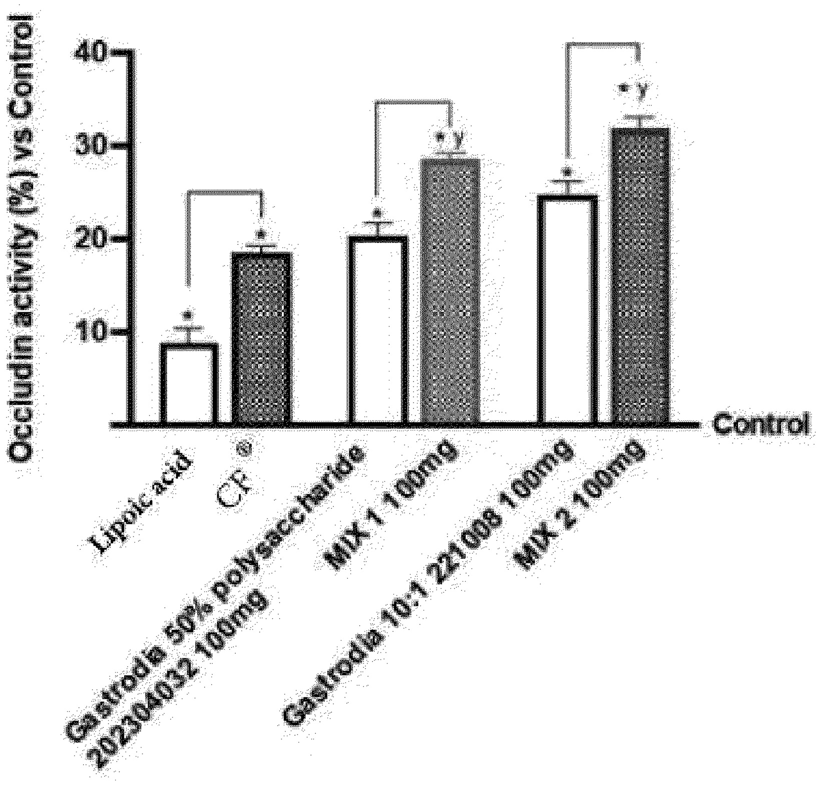
Occludin activity (%) vs Control
40
30
20
10
Control
* y
*
Lipoic acid
CF®
Gastrodia 50% polysaccharide 202304032 100mg
MIX 1 100mg
Gastrodia 10:1 221008 100mg
MIX 2 100mg

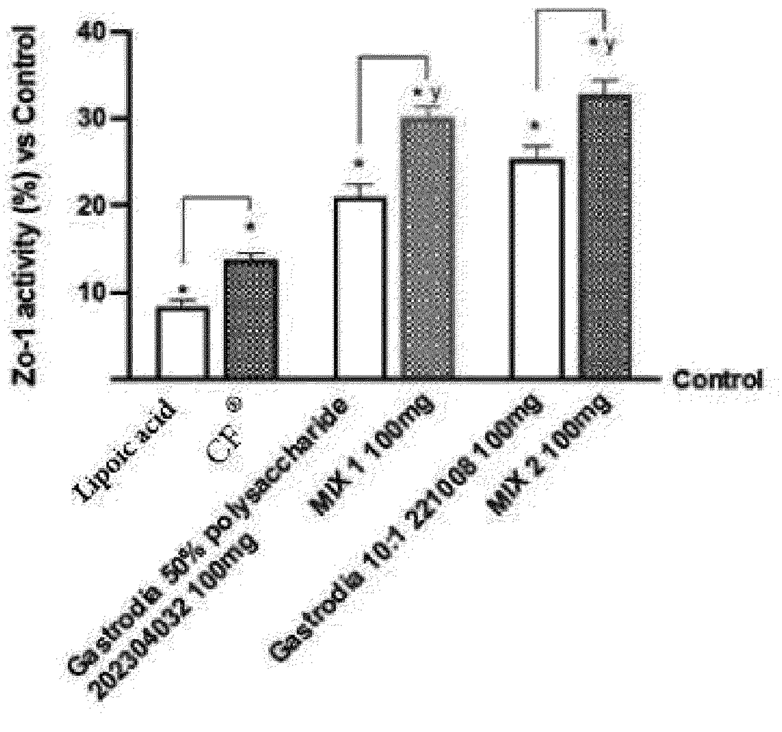
Zo-1 activity (%) vs Control
40
30
20
10
Control
* y
*
Lipoic acid
CF®
Gastrodia 50% polysaccharide 202304032 100mg
MIX 1 100mg
Gastrodia 10:1 221008 100mg
MIX 2 100mg

FIG. 10 A

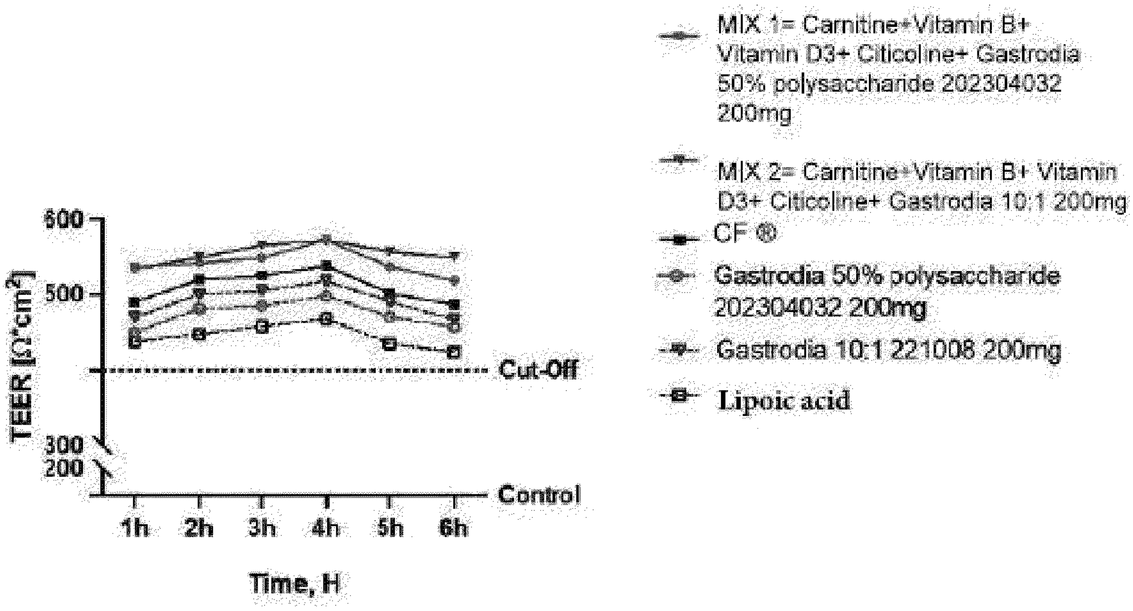


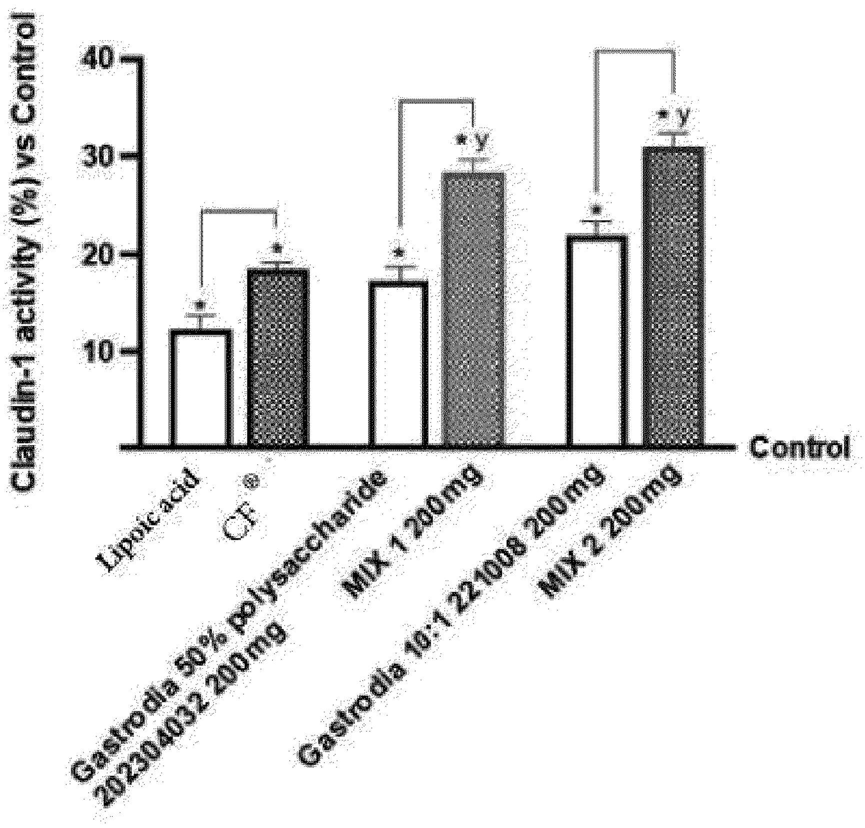


FIG 10 B (continued)

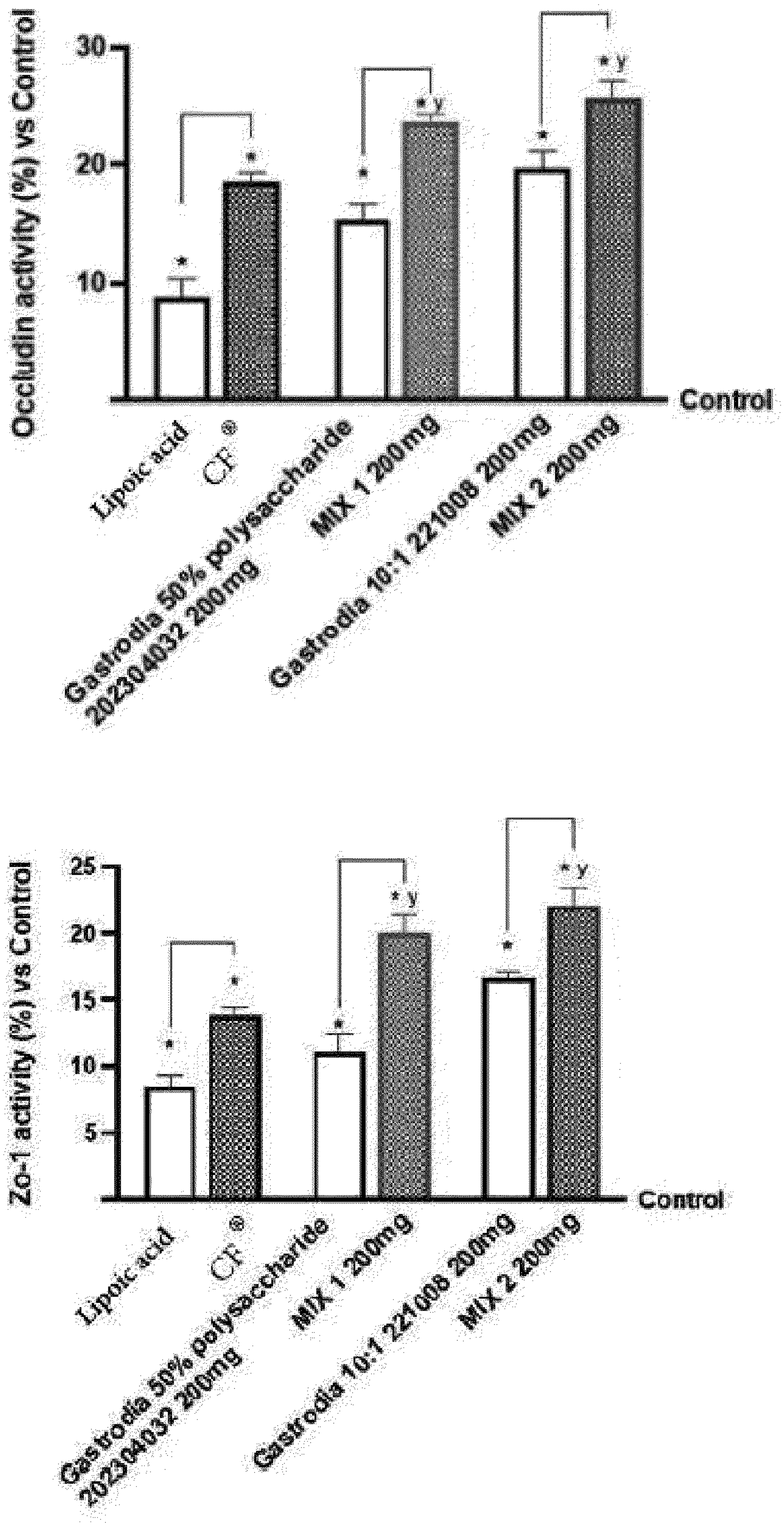
Occludin activity (%) vs Control
30
20
10
Control
*
* y
Lipoic acid
CF®
Gastrodia 50% polysaccharide 20230403 2 200mg
MIX 1 200mg
Gastrodia 10:1 221008 200mg
MIX 2 200mg
Zo-1 activity (%) vs Control
25
20
15
10
5

FIG. 10 B

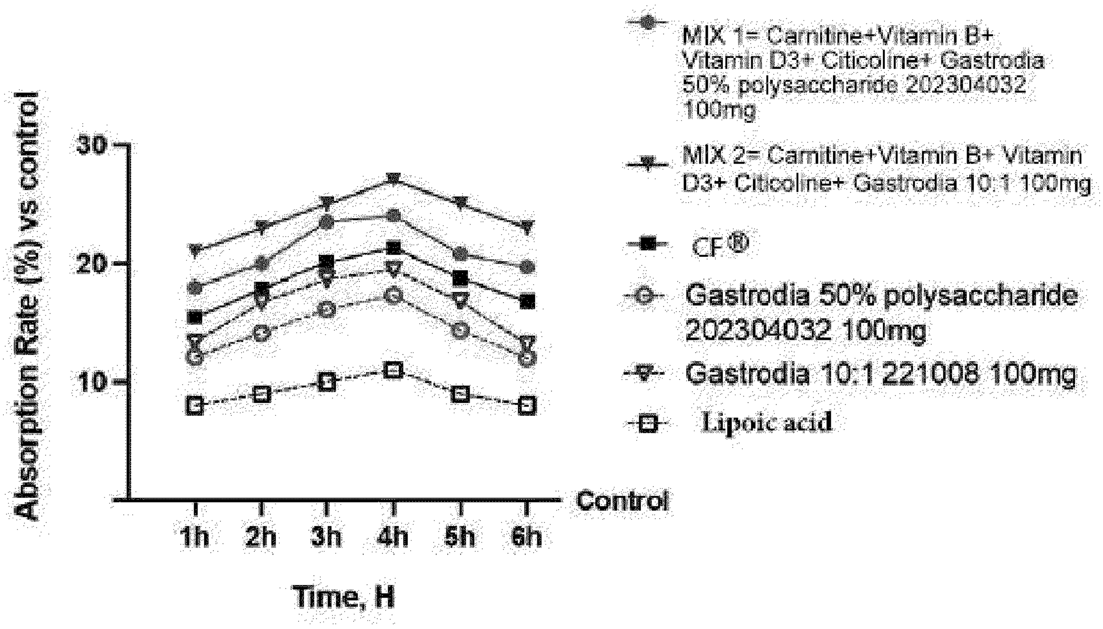

Absorption Rate (%) vs control
30
20
10
Control
1h
2h
3h
4h
5h
6h
Time, H
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 100mg
CF®
Gastrodia 50% polysaccharide 202304032 100mg
Gastrodia 10:1 221008 100mg
Lipoic acid


FIG. 11

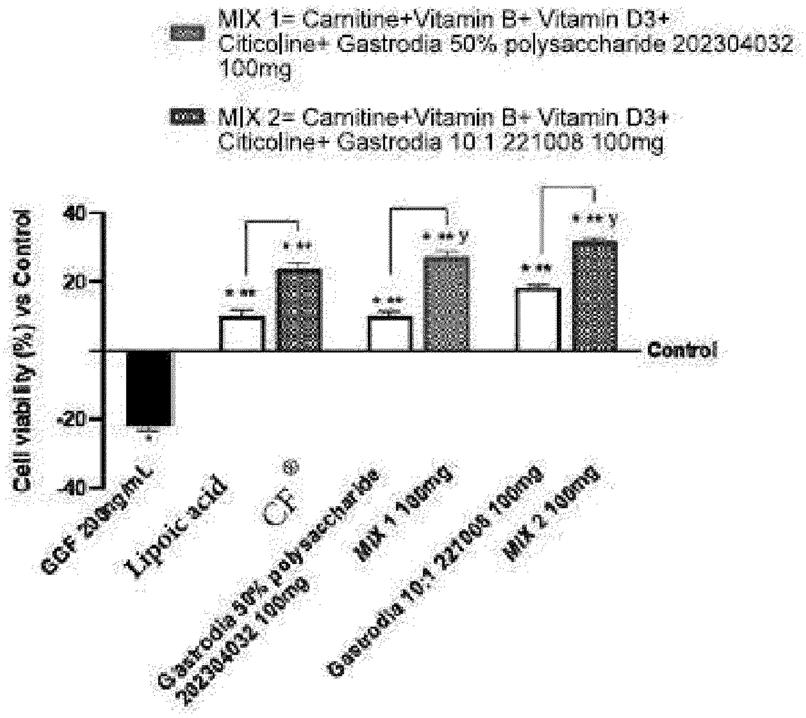


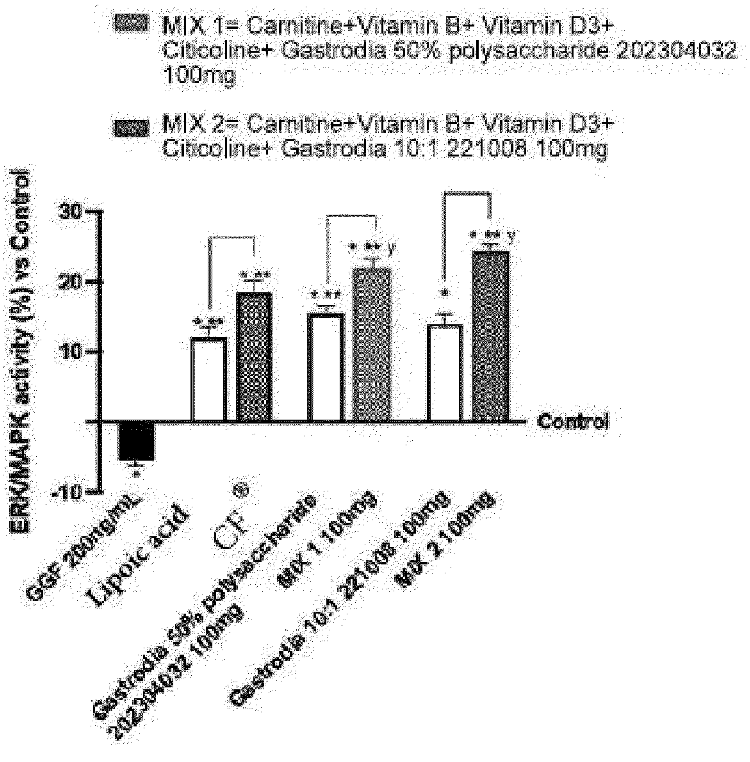


FIG. 12 A (continued)

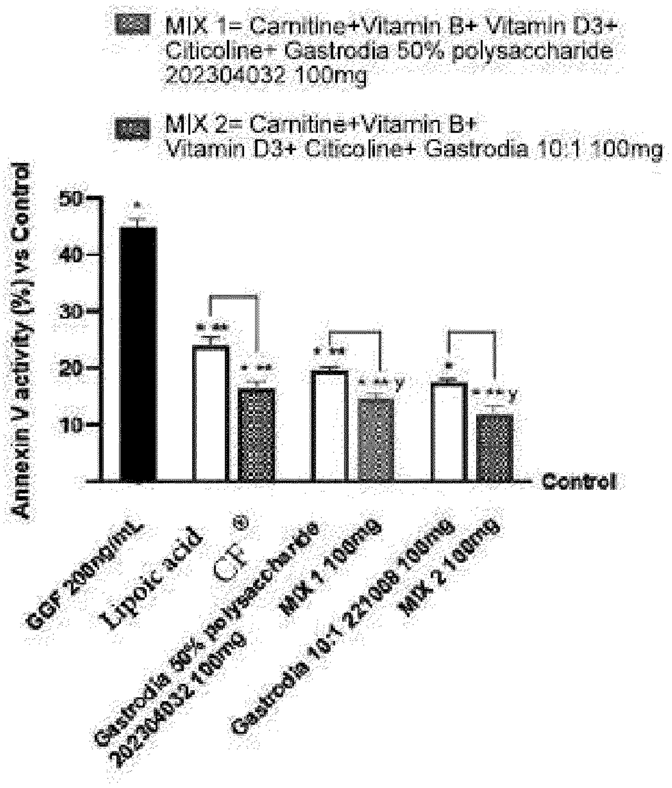
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 100mg
Annexin V activity (%) vs Control
50
40
30
20
10
Control
GGF 200ng/mL
Lipoic acid
CF®
Gastrodia 50% polysaccharide 202304032 100mg
MIX 1 100mg
Gastrodia 10:1 221008 100mg
MIX 2 100mg

FIG. 12 A

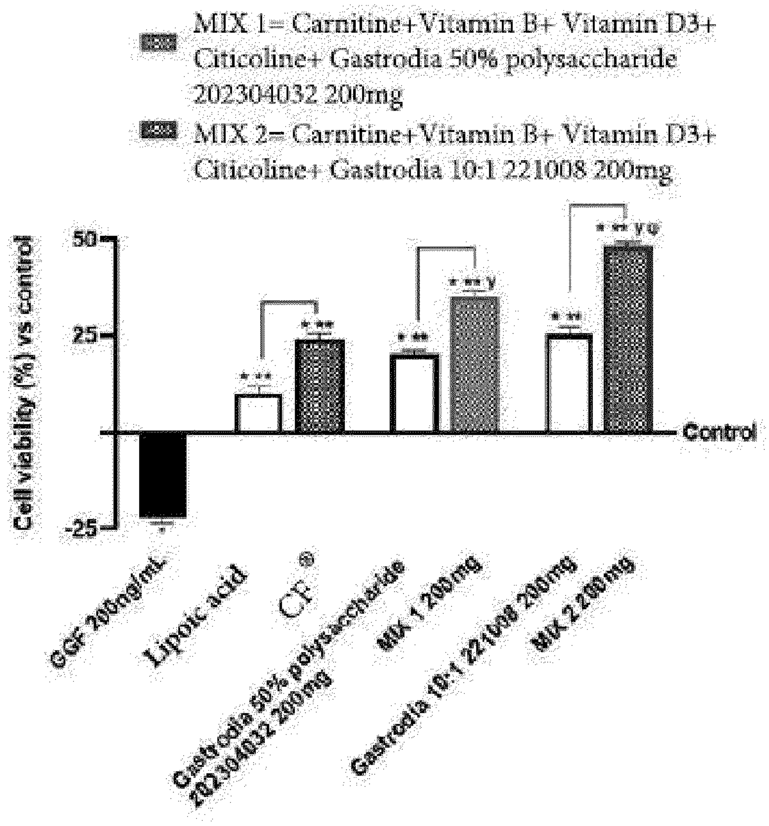


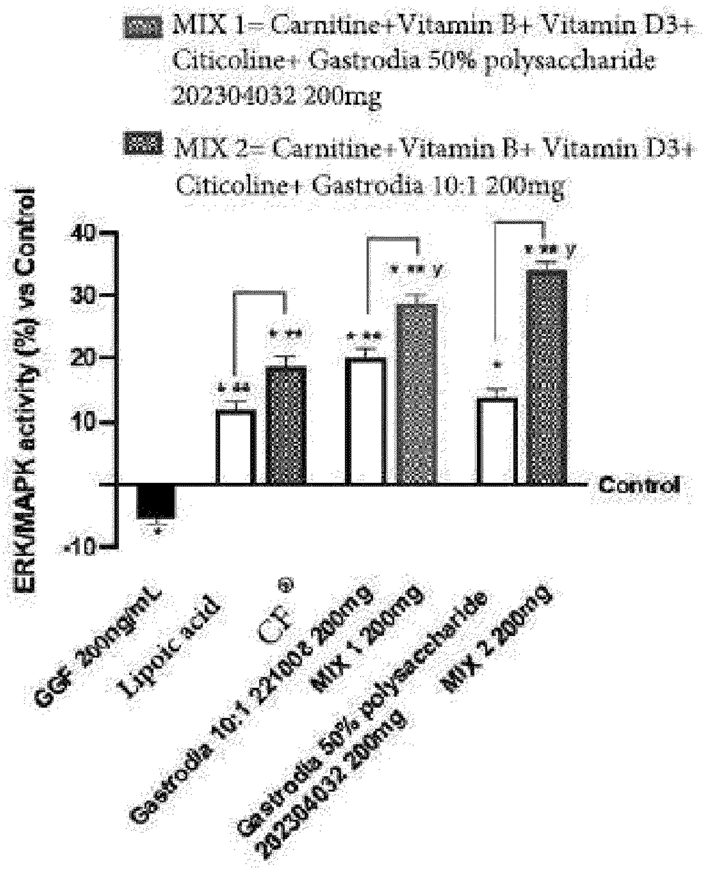


FIG. 12 B (continued)

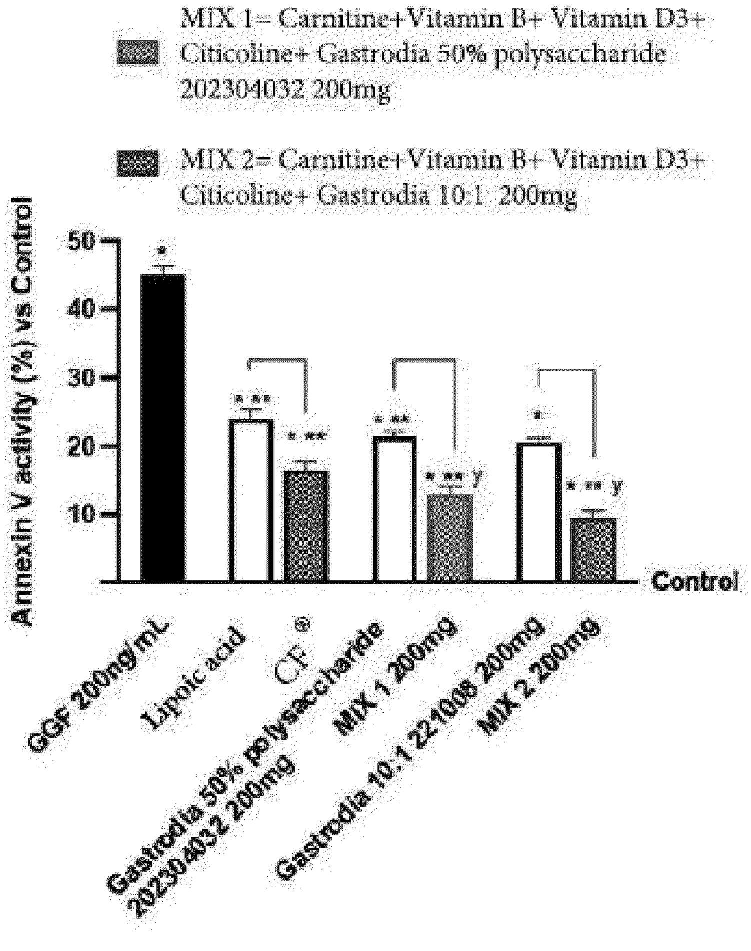
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 200mg
Annexin V activity (%) vs Control
50
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg

FIG. 12 B

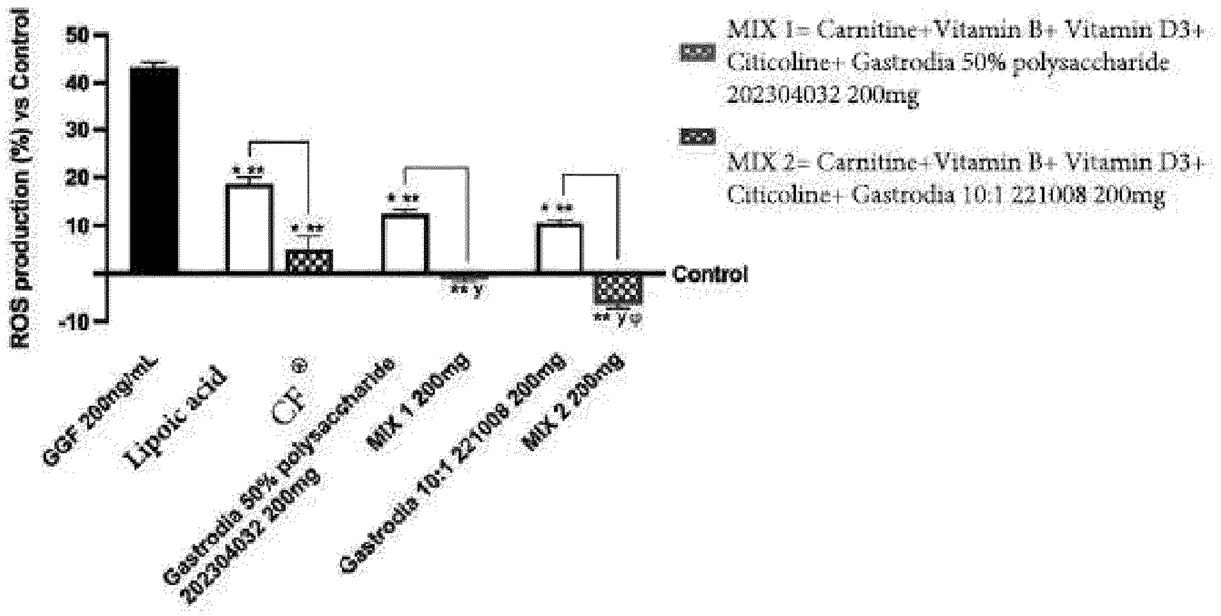
ROS production (%) vs Control
50
40
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

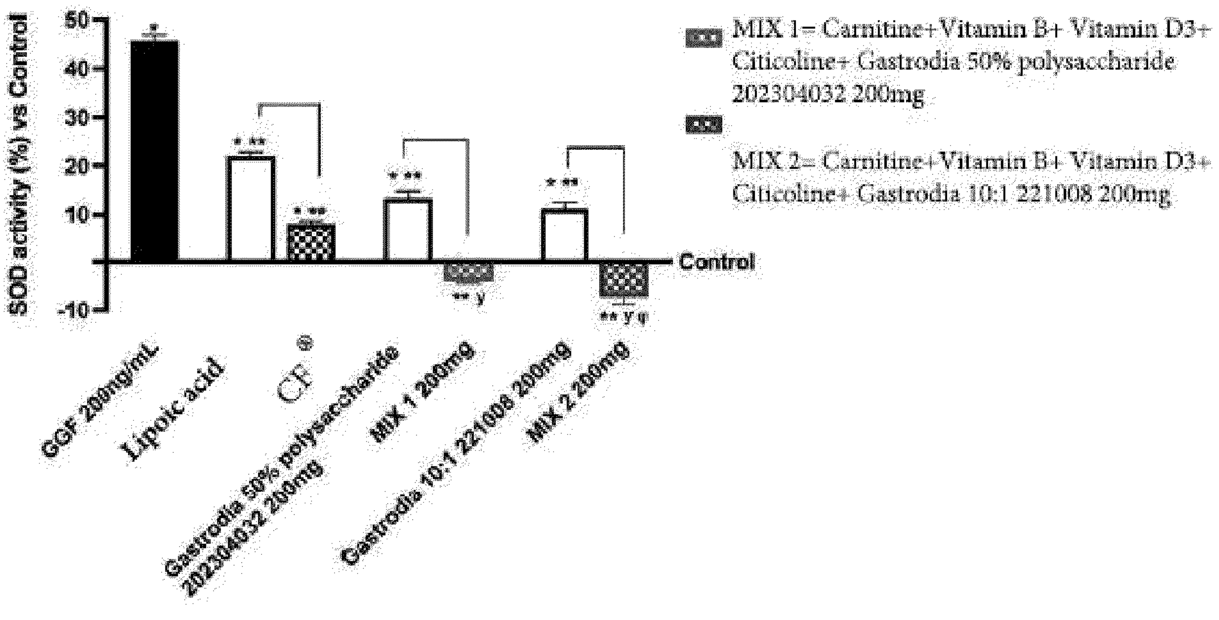
SOD activity (%) vs Control
50
40
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

FIG. 13 A

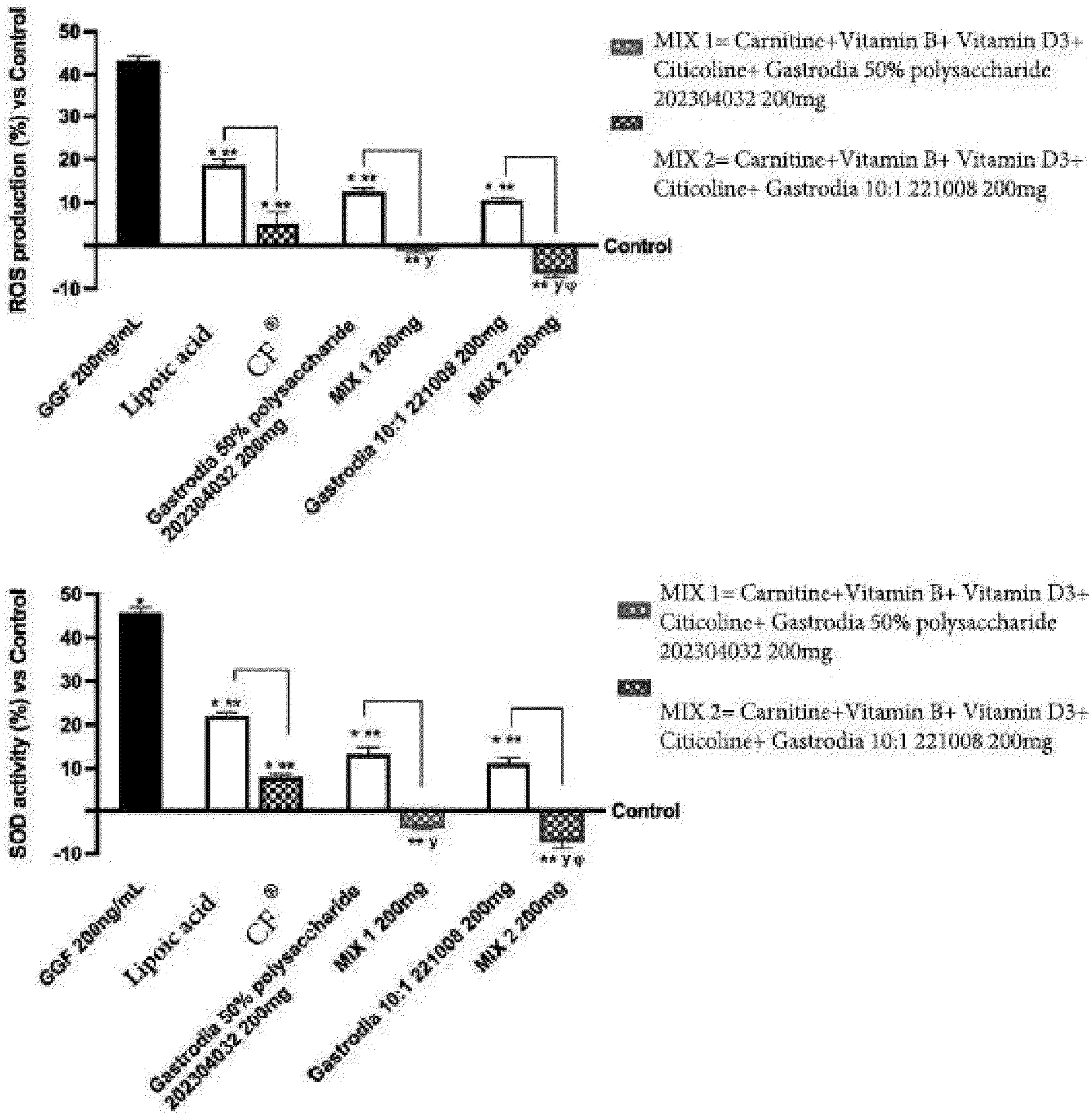
ROS production (%) vs Control
50
40
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
SOD activity (%) vs Control
50
40
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

13B

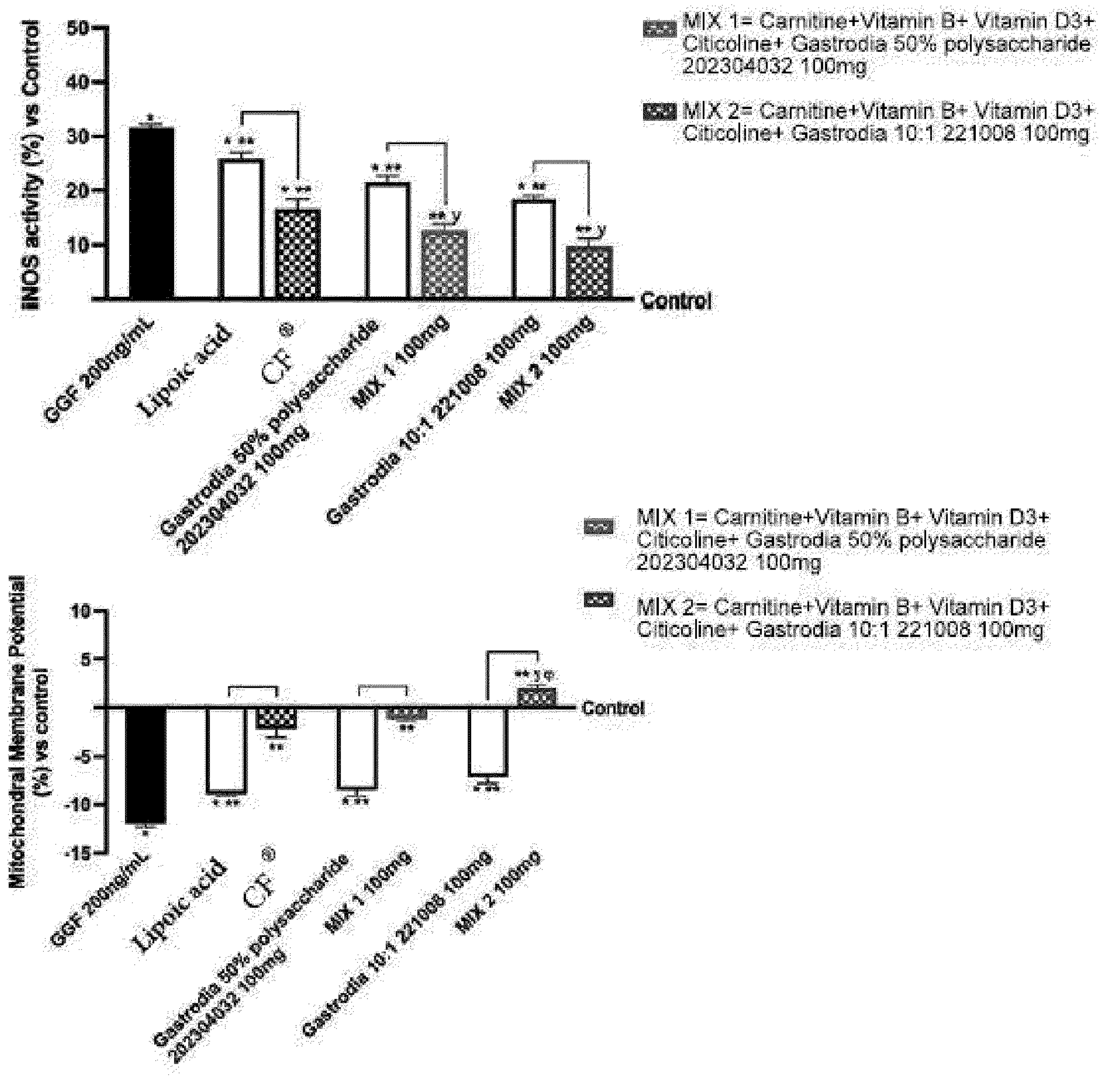
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
iNOS activity (%) vs Control
Control
GGF 200ng/mL
Lipoic acid
CF®
Gastrodia 50% polysaccharide 202304032 100mg
MIX 1 100mg
Gastrodia 10:1 221008 100mg
MIX 2 100mg
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
Mitochondrial Membrane Potential (%) vs control
Control
GGF 200ng/mL
Lipoic acid
CF®
Gastrodia 50% polysaccharide 202304032 100mg
MIX 1 100mg
Gastrodia 10:1 221008 100mg
MIX 2 100mg

FIG. 14 A

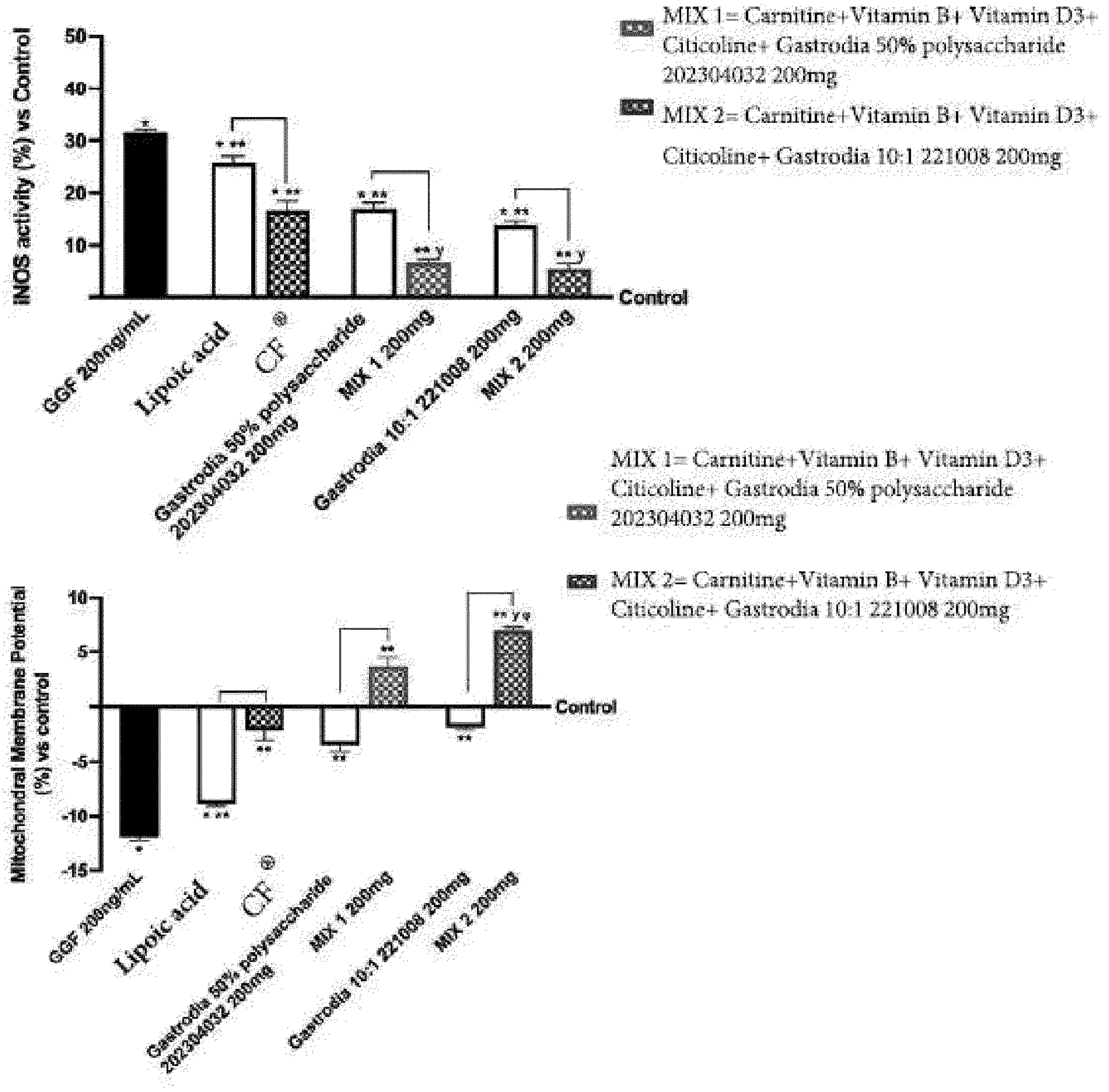
iNOS activity (%) vs Control
50
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
Mitochondral Membrane Potential (%) vs control
10
5
-5
-10
-15
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

FIG. 14 B

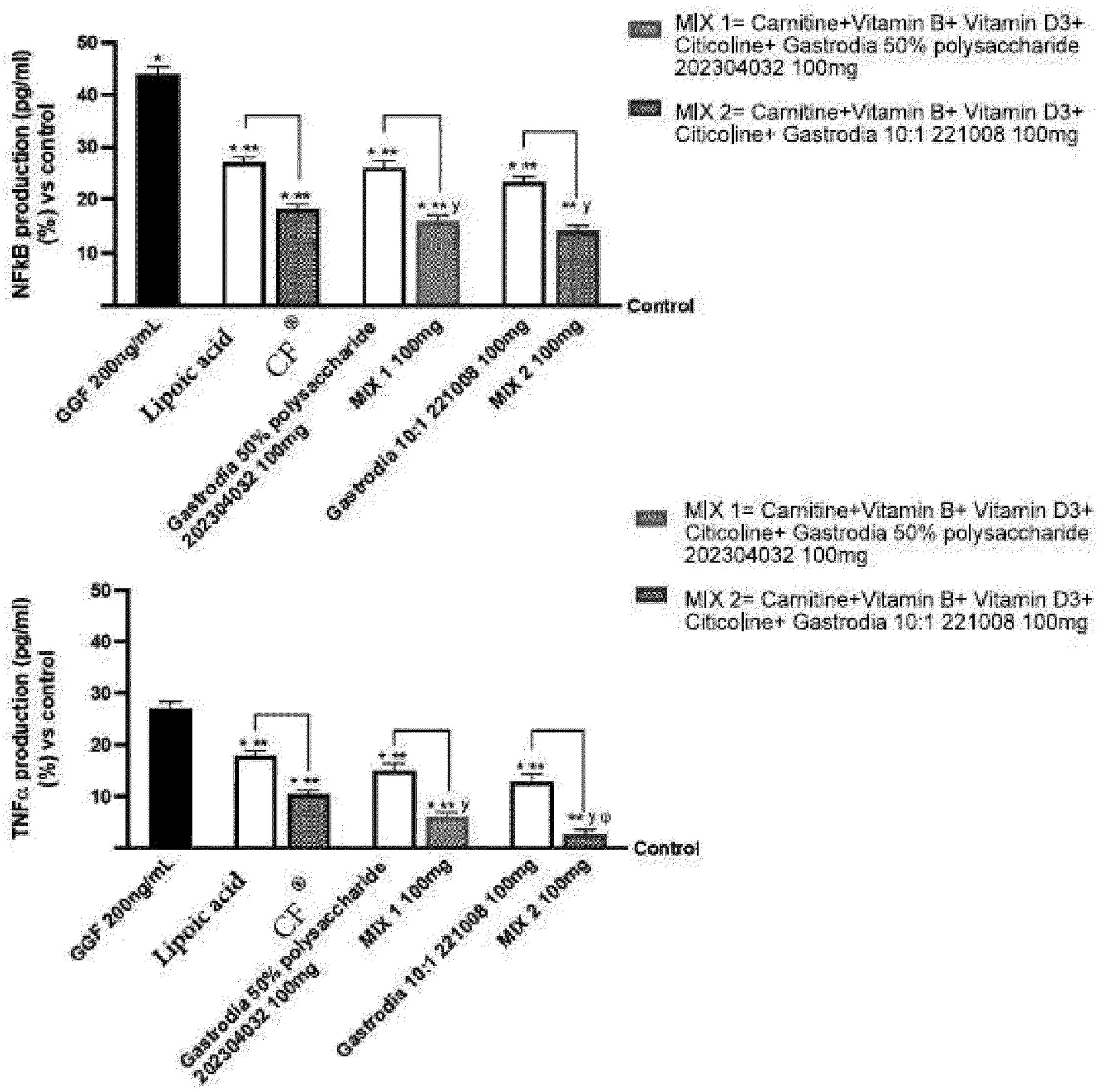
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
NFkB production (pg/ml) (%) vs control
TNFα production (pg/ml) (%) vs control
Control
GGF 200ng/mL
Lipoic acid
CF ®
Gastrodia 50% polysaccharide 202304032 100mg
MIX 1 100mg
Gastrodia 10:1 221008 100mg
MIX 2 100mg

FIG. 15 A

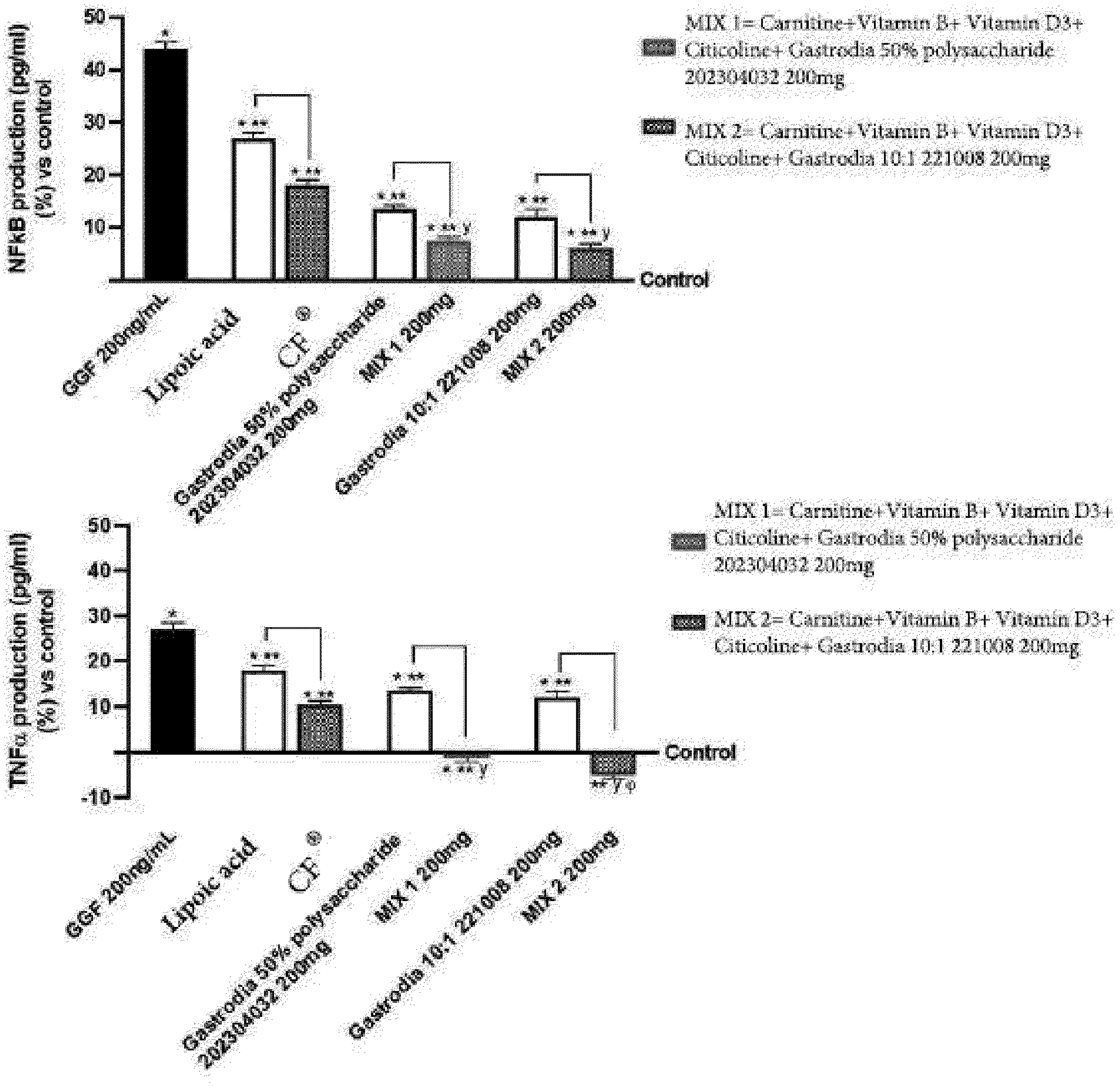

NFkB production (pg/ml) (%) vs control
50
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
TNFα production (pg/ml) (%) vs control
50
40
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg


FIG. 15 B

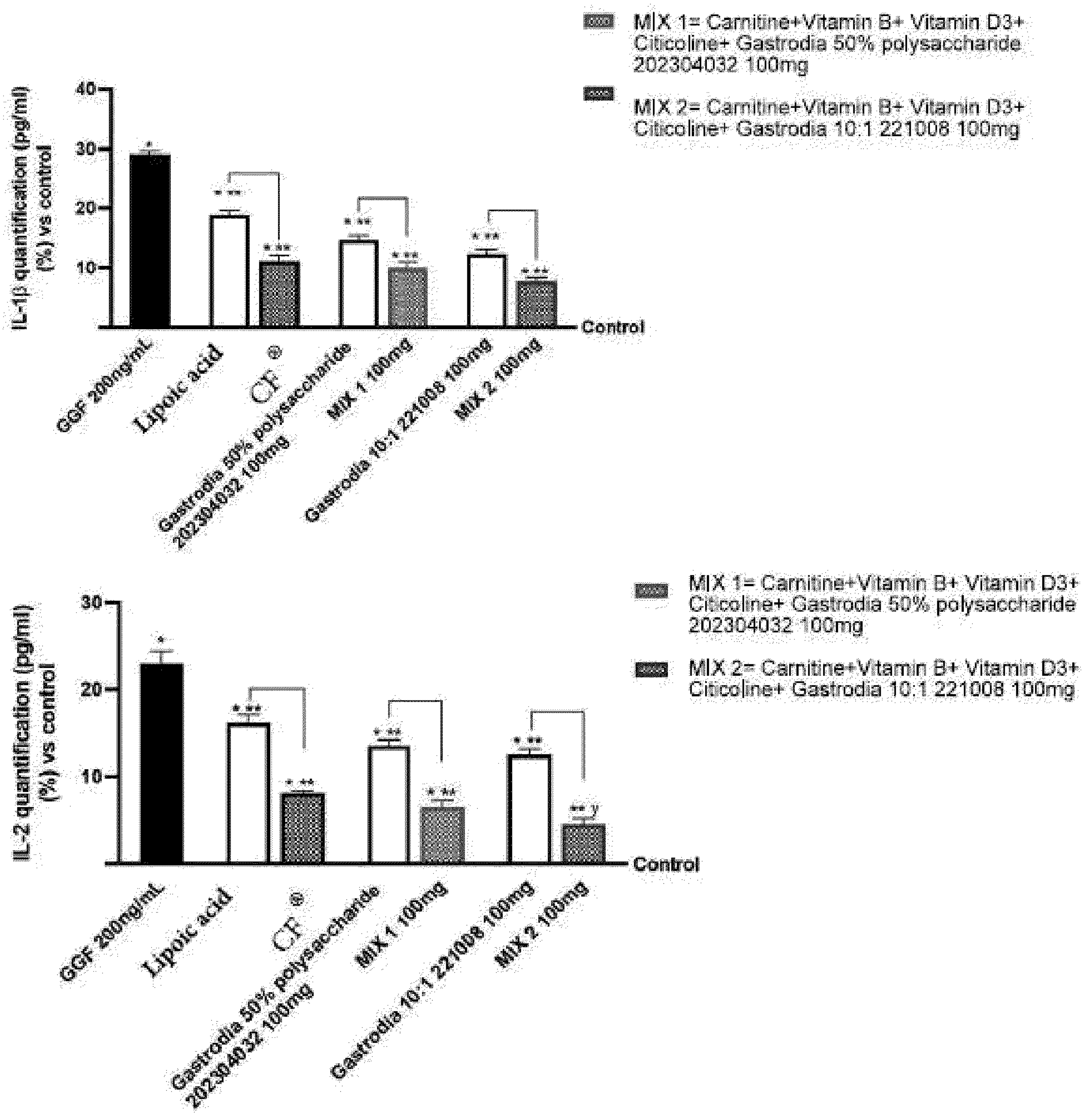


FIG. 16 A (continued)

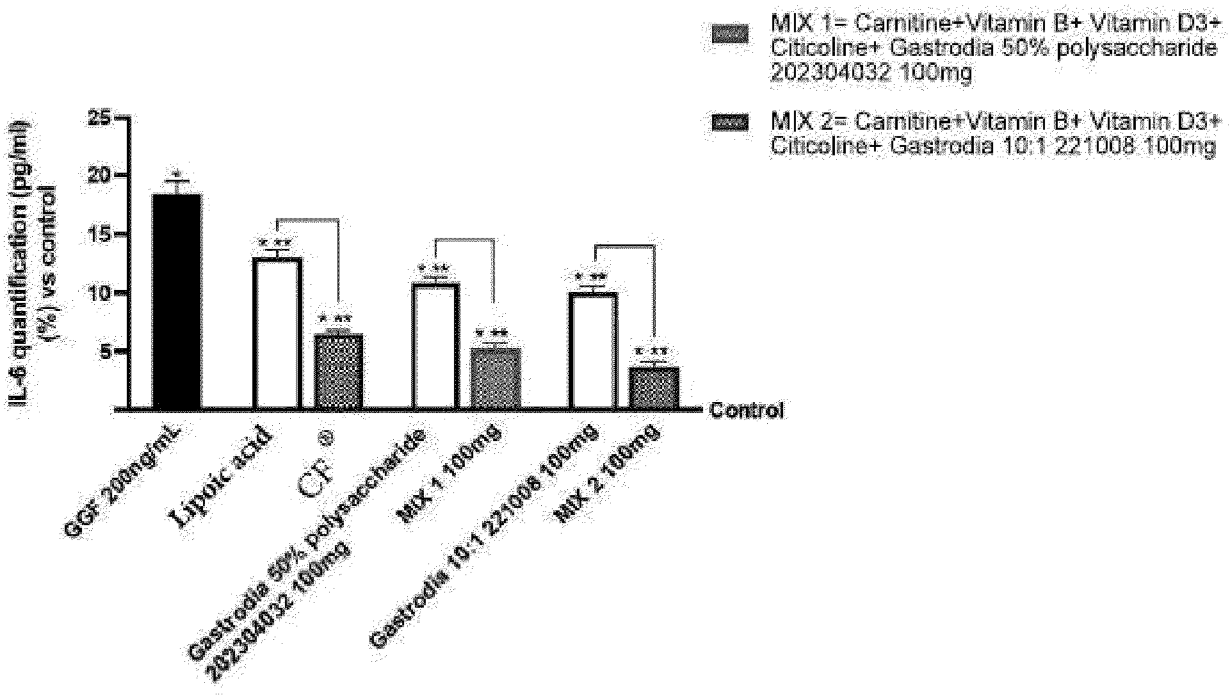
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
IL-6 quantification (pg/ml) (%) vs control
25
20
15
10
5
Control
GGF 200ng/mL
Lipoic acid
CF ®
Gastrodia 50% polysaccharide 202304032 100mg
MIX 1 100mg
Gastrodia 10:1 221008 100mg
MIX 2 100mg

FIG. 16 A

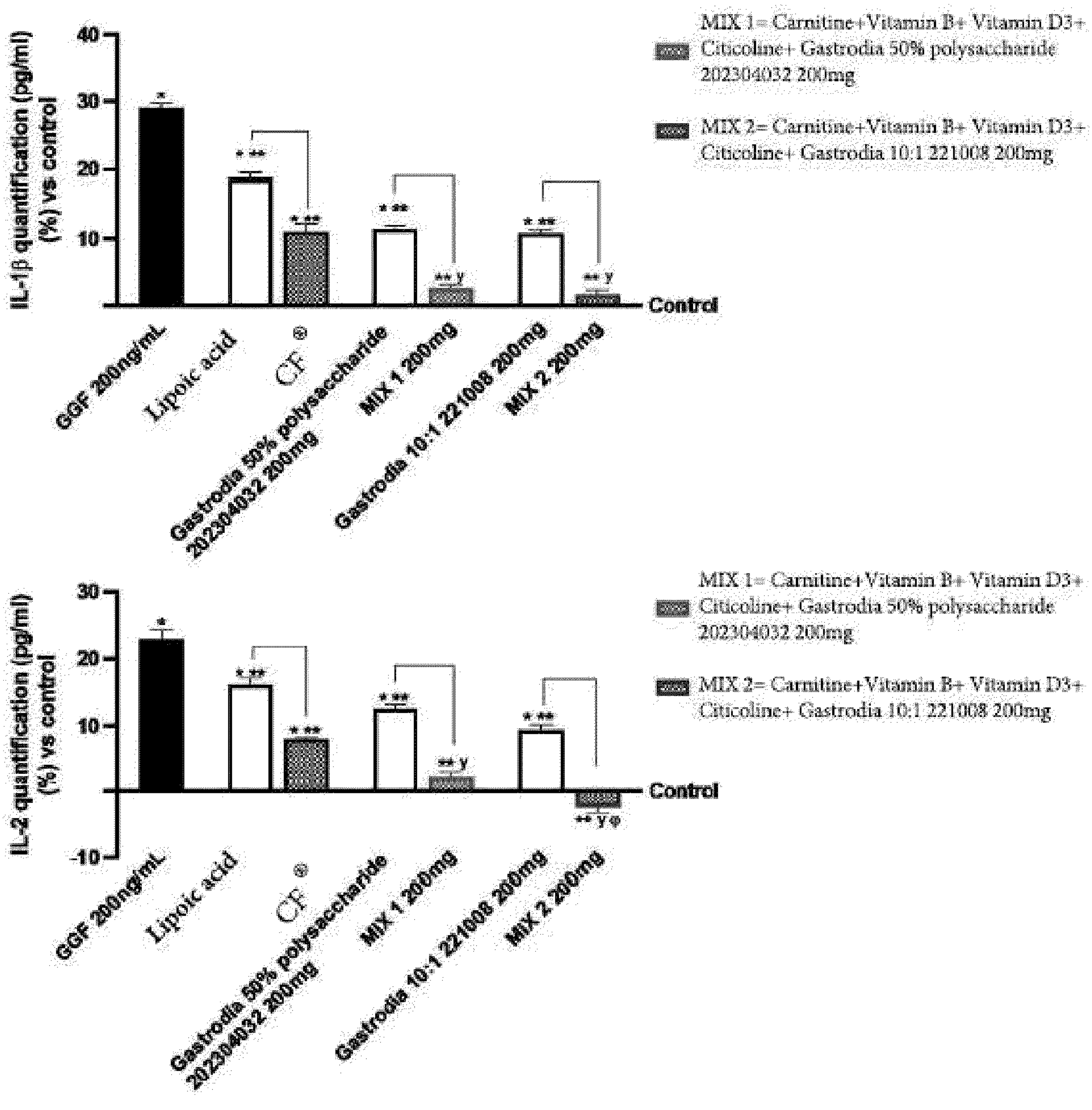


FIG. 16 B (continued)

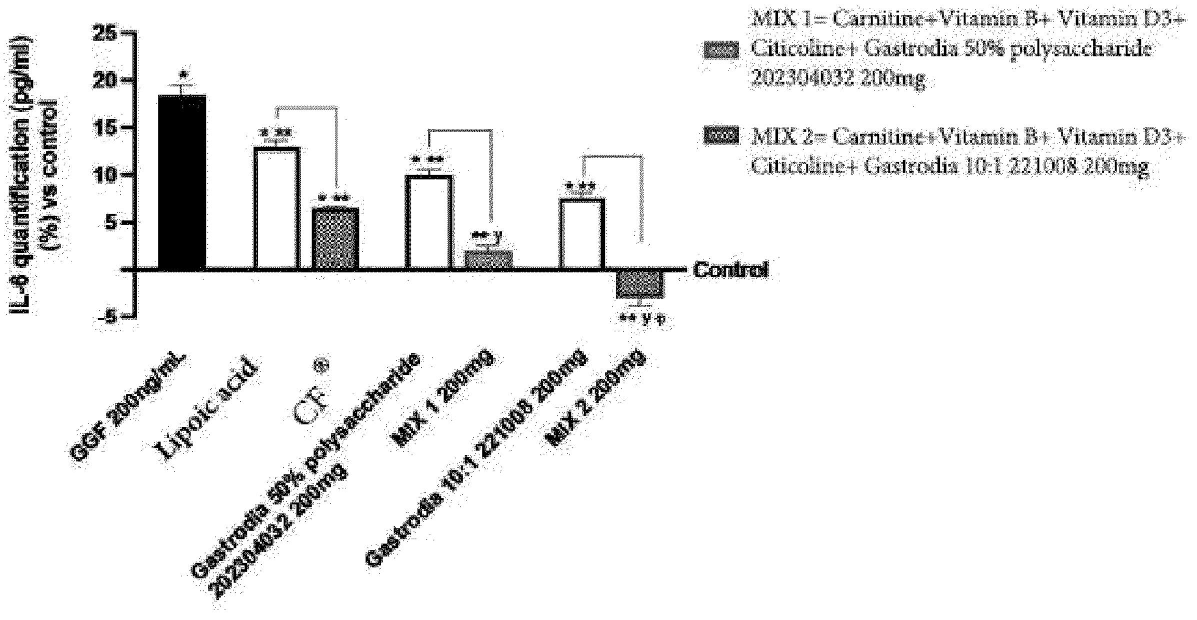
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
IL-6 quantification (pg/ml) (%) vs control
25
20
15
10
5
-5
Control
GGF 200ng/mL
Lipoic acid
CF®
Gastrodia 50% polysaccharide 202304032 200mg
MIX 1 200mg
Gastrodia 10:1 221008 200mg
MIX 2 200mg

FIG. 16 B

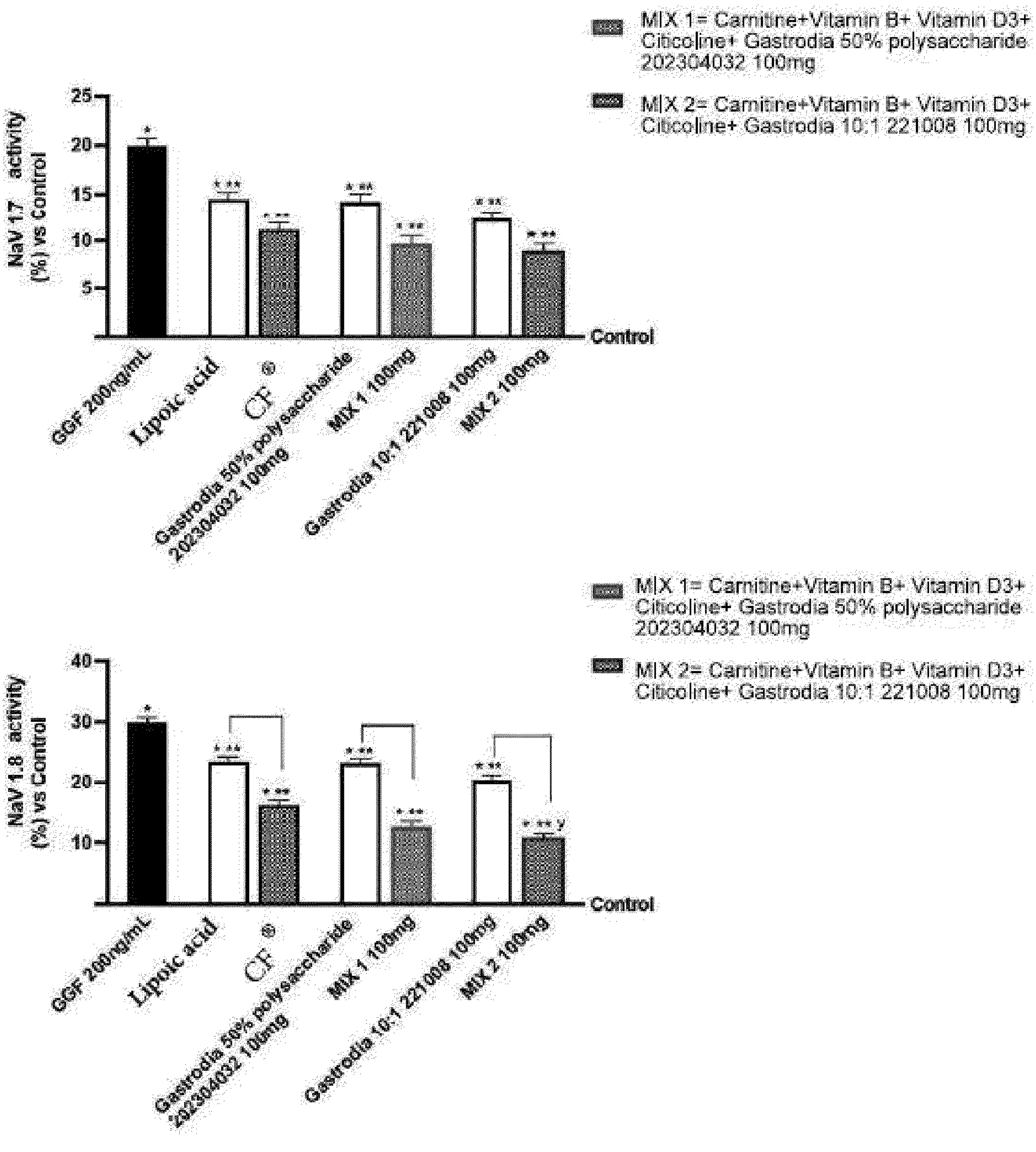
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
NaV 1.7 activity (%) vs Control
25
20
15
10
5
Control
GGF 200ng/mL
Lipoic acid
CF®
Gastrodia 50% polysaccharide 202304032 100mg
MIX 1 100mg
Gastrodia 10:1 221008 100mg
MIX 2 100mg
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
NaV 1.8 activity (%) vs Control
40
30
20
10
Control
GGF 200ng/mL
Lipoic acid
CF®
Gastrodia 50% polysaccharide 202304032 100mg
MIX 1 100mg
Gastrodia 10:1 221008 100mg
MIX 2 100mg

FIG. 17 A

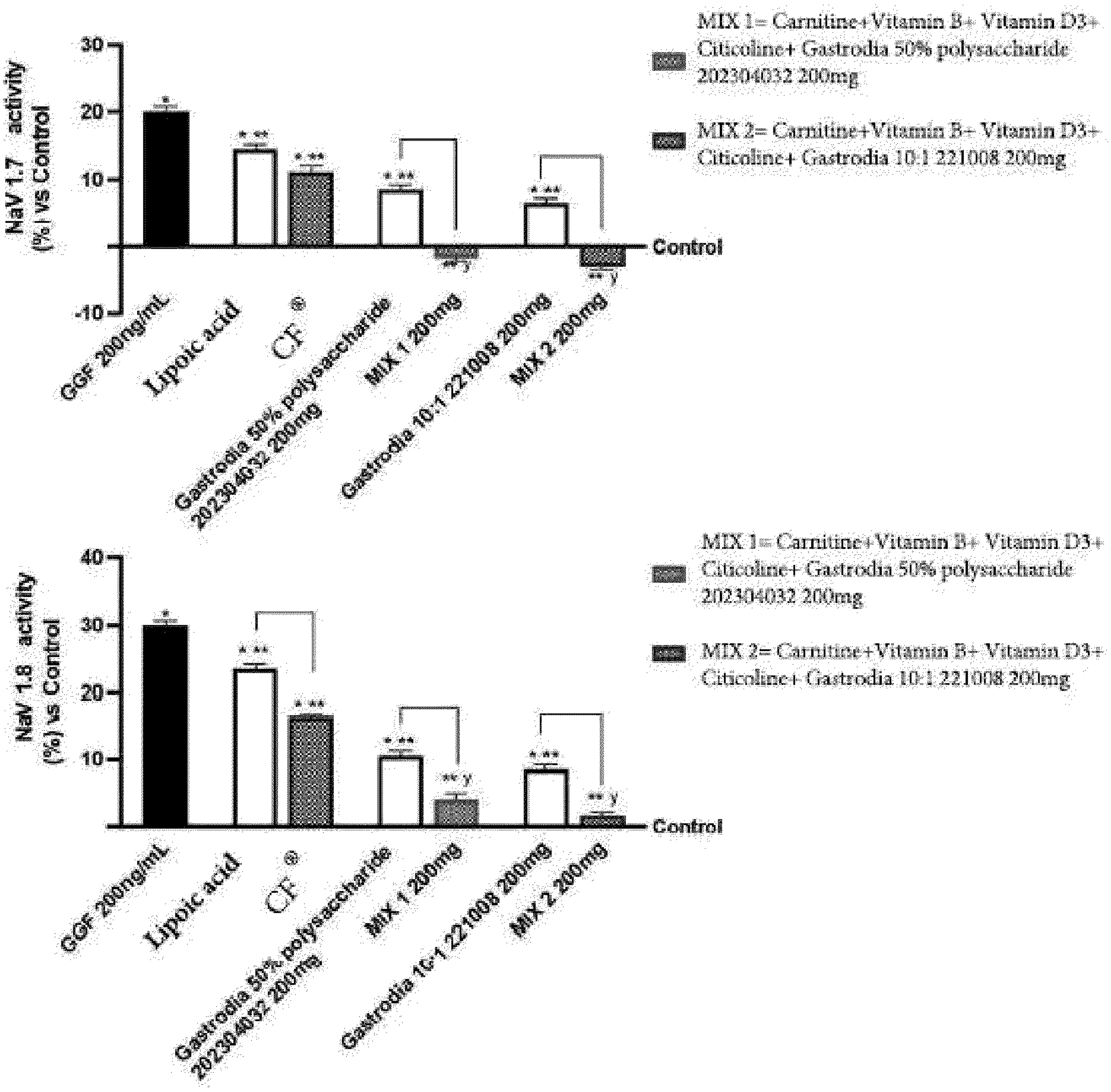
NaV 1.7 activity (%) vs Control
30
20
10
-10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
NaV 1.8 activity (%) vs Control
40
30
20
10
Control

FIG. 17 B

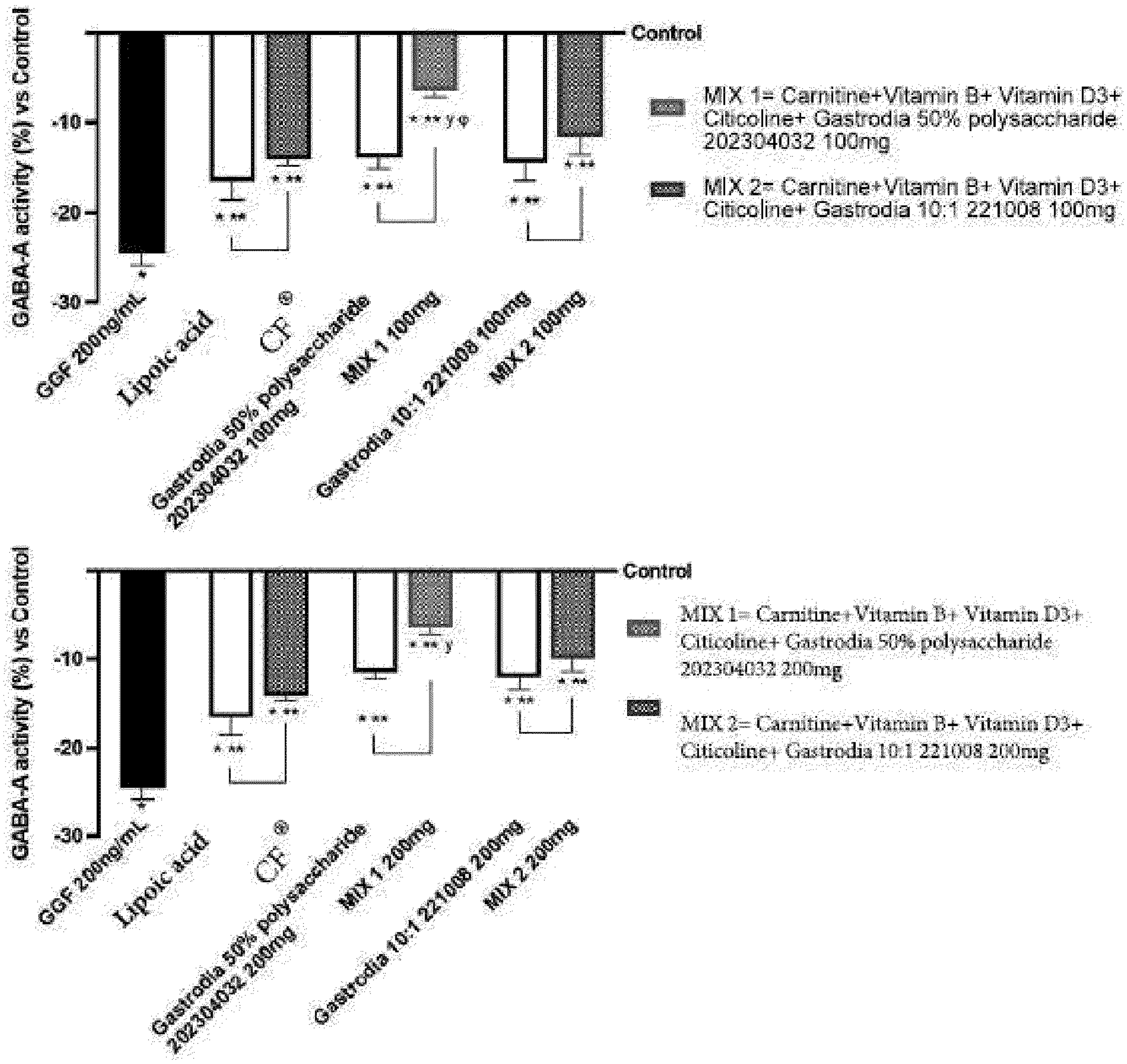

GABA-A activity (%) vs Control
Control
-10
-20
-30
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
GGF 200ng/mL
Lipoic acid
CF®
Gastrodia 50% polysaccharide 202304032 100mg
MIX 1 100mg
Gastrodia 10:1 221008 100mg
MIX 2 100mg
GABA-A activity (%) vs Control
Control
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
GGF 200ng/mL
Lipoic acid
CF®
Gastrodia 50% polysaccharide 202304032 200mg
MIX 1 200mg
Gastrodia 10:1 221008 200mg
MIX 2 200mg


FIG. 18

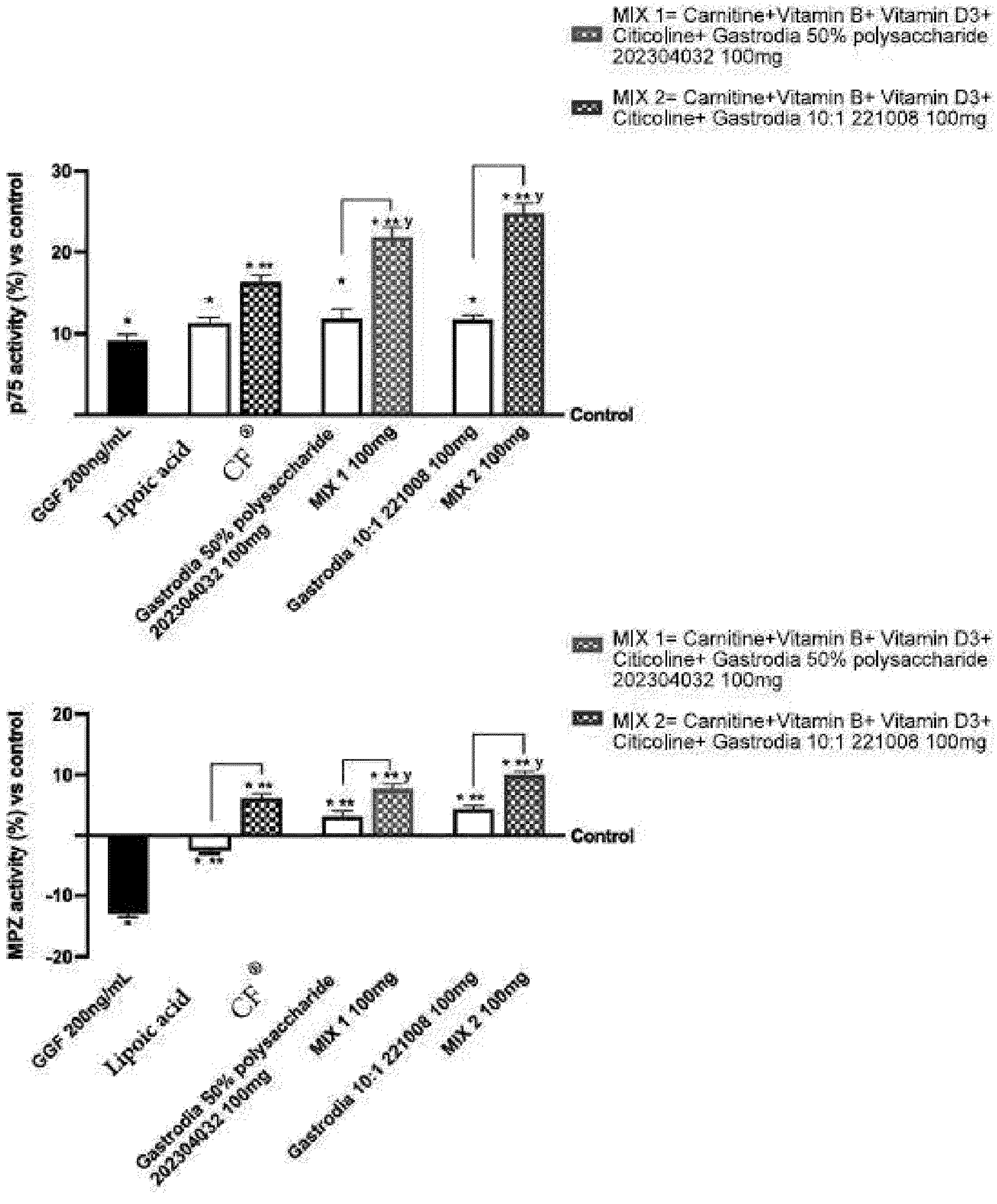
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
p75 activity (%) vs control
30
20
10
Control
GGF 200ng/mL
Lipoic acid
CF®
Gastrodia 50% polysaccharide 202304032 100mg
MIX 1 100mg
Gastrodia 10:1 221008 100mg
MIX 2 100mg
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
MPZ activity (%) vs control
20
10
-10
-20
Control
GGF 200ng/mL
Lipoic acid
CF®
Gastrodia 50% polysaccharide 202304032 100mg
MIX 1 100mg
Gastrodia 10:1 221008 100mg
MIX 2 100mg

FIG. 19 A

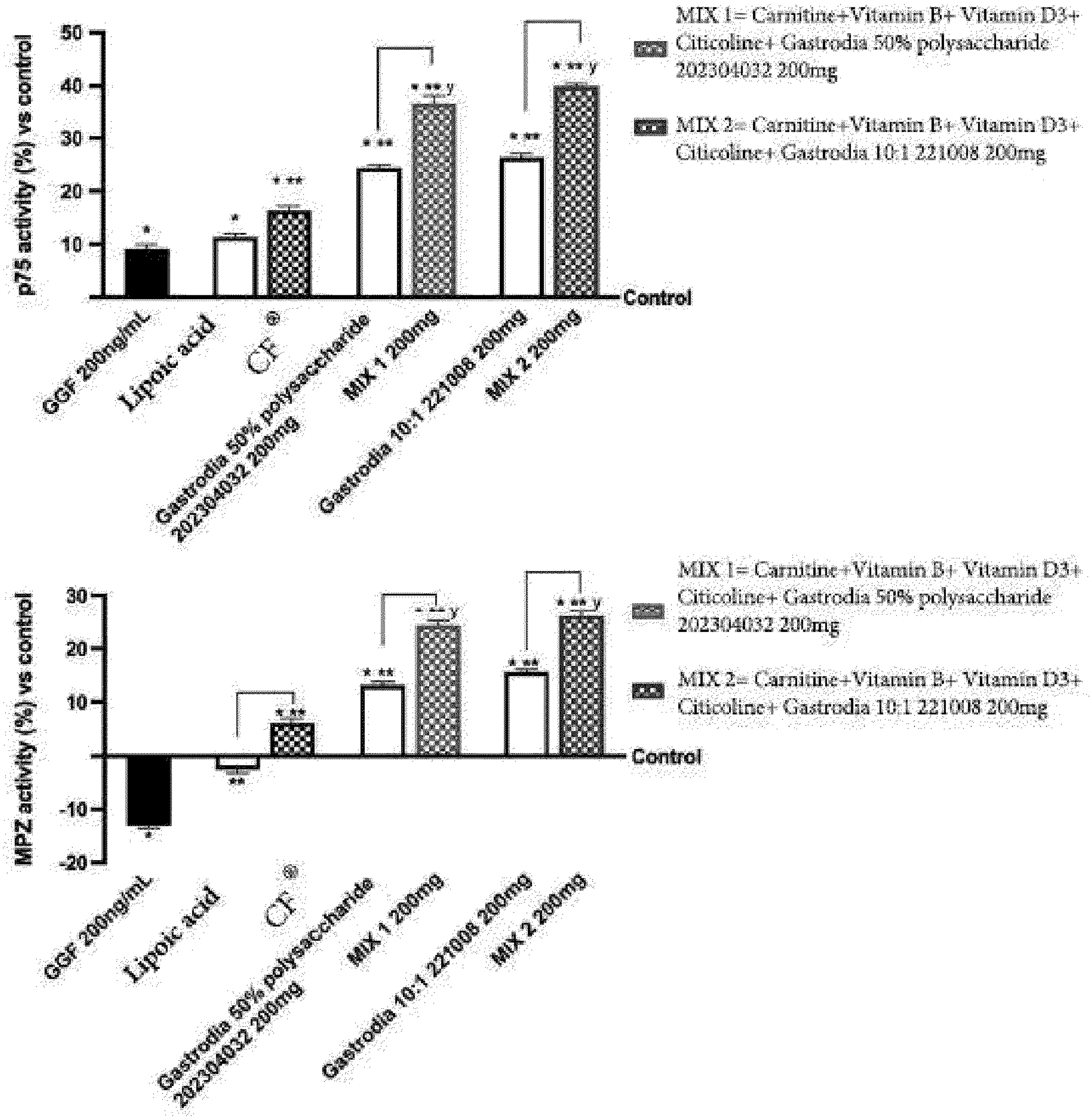
p75 activity (%) vs control
50
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
MPZ activity (%) vs control
30
20
10
-10
-20
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

FIG. 19 B

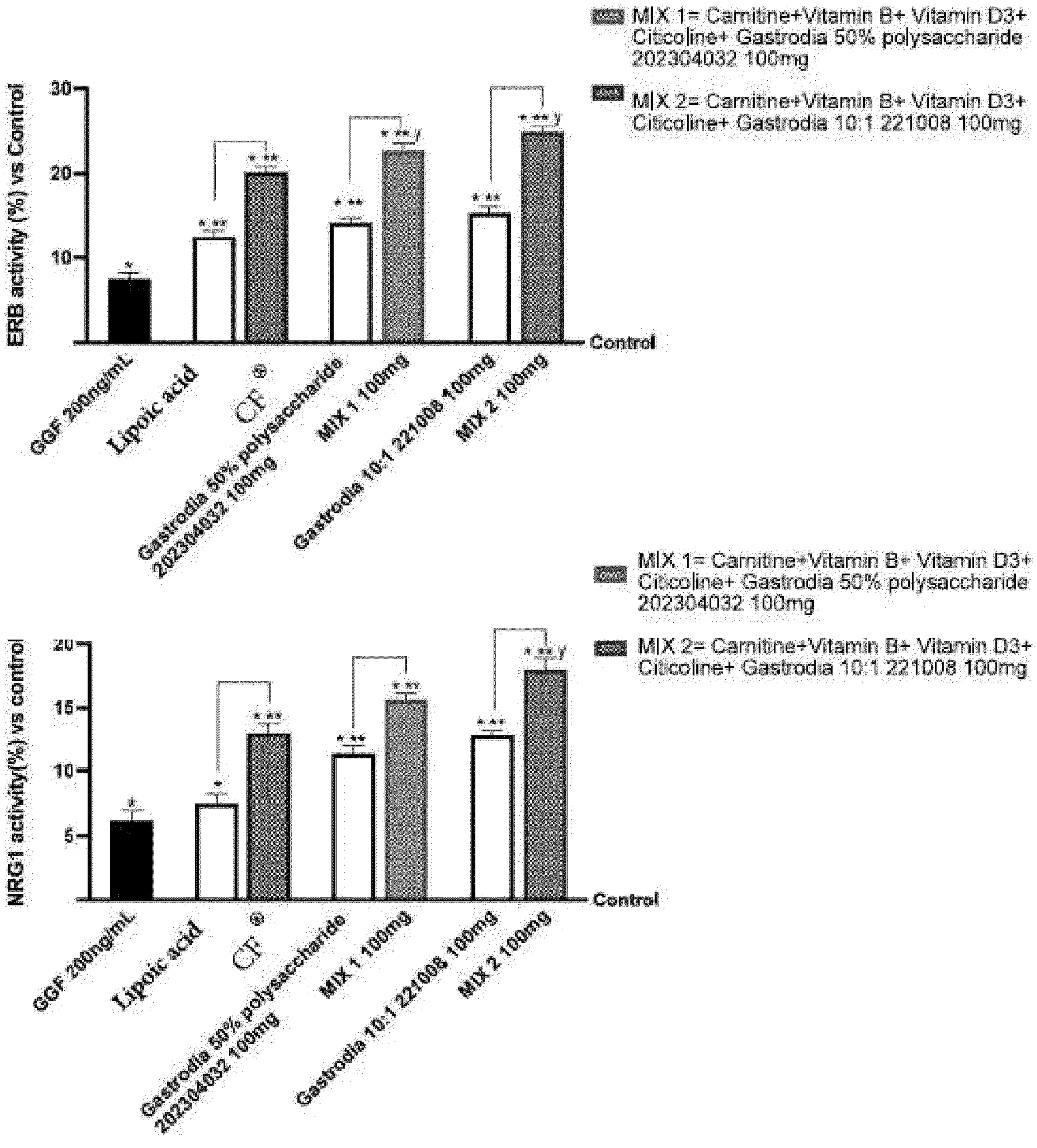
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
ERB activity (%) vs Control
30
20
10
Control
GGF 200ng/mL
Lipoic acid
CF®
Gastrodia 50% polysaccharide 202304032 100mg
MIX 1 100mg
Gastrodia 10:1 221008 100mg
MIX 2 100mg
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
NRG1 activity(%) vs control
20
15
10
5
Control
GGF 200ng/mL
Lipoic acid
CF®
Gastrodia 50% polysaccharide 202304032 100mg
MIX 1 100mg
Gastrodia 10:1 221008 100mg
MIX 2 100mg

FIG. 20 A

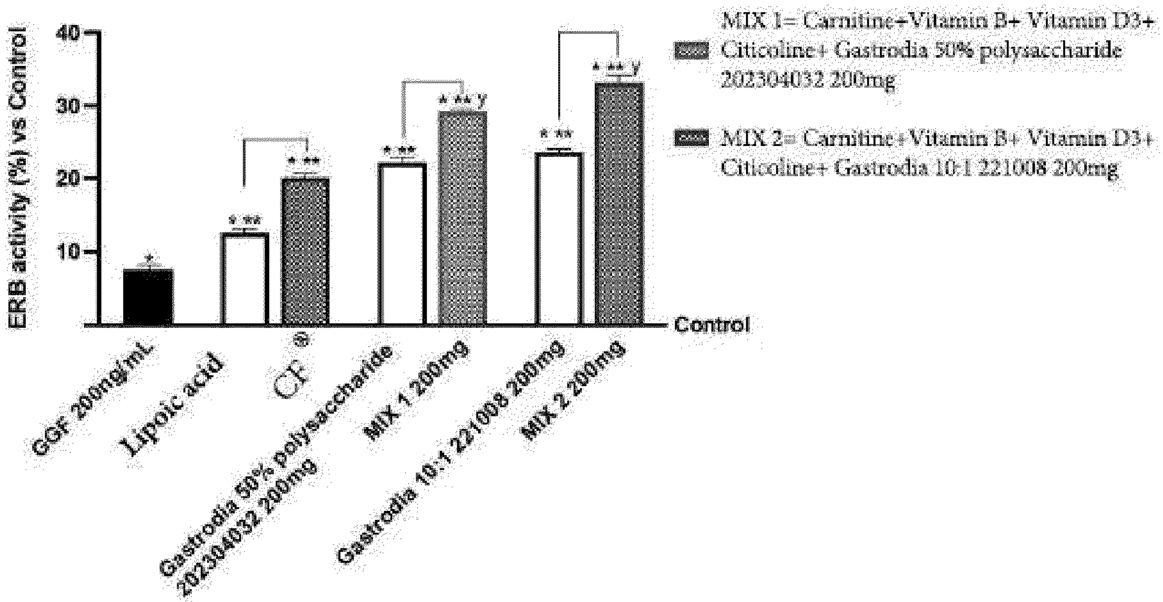
ERB activity (%) vs Control
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

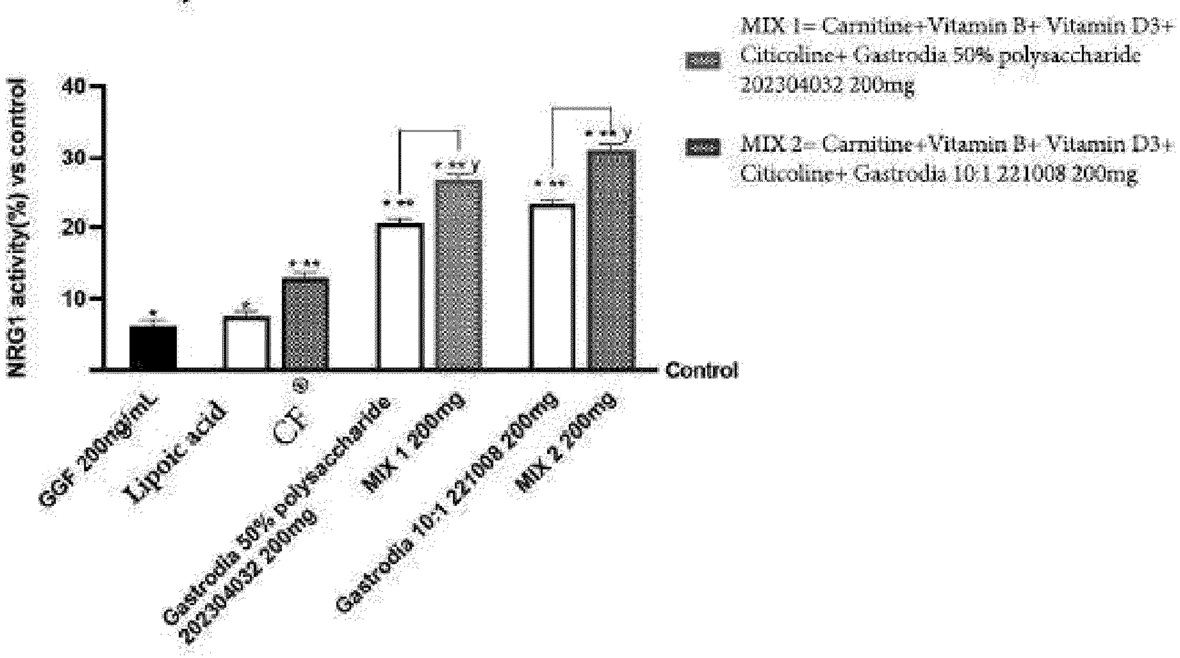
NRG1 activity(%) vs control
40
30
20
10
Control
GGF 200ng/mL
Lipoic acid
CF®
Gastrodia 50% polysaccharide 202304032 200mg
MIX 1 200mg
Gastrodia 10:1 221008 200mg
MIX 2 200mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg

FIG. 20 B

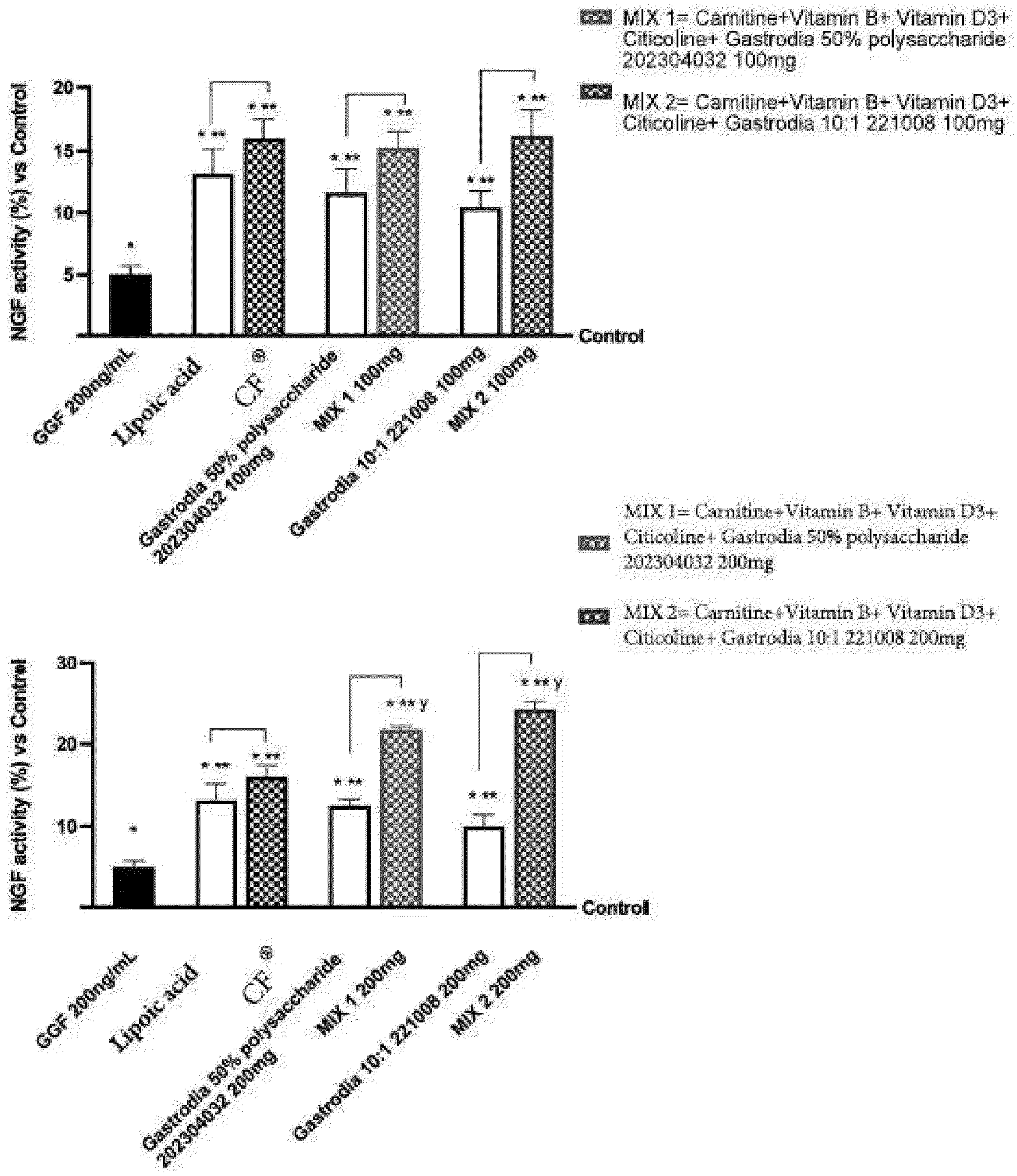
MIX 1= Carnitine+Vitamin B+ Vitamin D3+ Citicoline+ Gastrodia 50% polysaccharide 202304032 100mg
MIX 2= Carnitine+Vitamin B+ Vitamin D3+ Citicoline+ Gastrodia 10:1 221008 100mg
NGF activity (%) vs Control
Control
GGF 200ng/mL
Lipoic acid
CF®
Gastrodia 50% polysaccharide 202304032 100mg
MIX 1 100mg
Gastrodia 10:1 221008 100mg
MIX 2 100mg
MIX 1= Carnitine+Vitamin B+ Vitamin D3+ Citicoline+ Gastrodia 50% polysaccharide 202304032 200mg
MIX 2= Carnitine+Vitamin B+ Vitamin D3+ Citicoline+ Gastrodia 10:1 221008 200mg
NGF activity (%) vs Control
Control
GGF 200ng/mL
Lipoic acid
CF®
Gastrodia 50% polysaccharide 202304032 200mg
MIX 1 200mg
Gastrodia 10:1 221008 200mg
MIX 2 200mg

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *CHEMICAL ABSTRACTS*, 62499-27-8
- *CHEMICAL ABSTRACTS*, 987-78-0
- *CHEMICAL ABSTRACTS*, 3040-38-8
- Remington: The Science and Practice of Pharmacy. Lippincott, Williams & Wilkins